(19) Europäisches Patentamt
European Patent Office
Office européen des brevets

(11) EP 2 789 343 A1

(12) EUROPEAN PATENT APPLICATION

(43) Date of publication:
**15.10.2014 Bulletin 2014/42**

(51) Int Cl.:
***A61K 38/42*** (2006.01) ***A61K 31/739*** (2006.01)
***C07K 14/805*** (2006.01) ***A61P 31/04*** (2006.01)

(21) Application number: **13163414.9**

(22) Date of filing: **11.04.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Clinique La Prairie**
**1815 Clarens-Montreux (CH)**

(72) Inventor: **The designation of the inventor has not yet been filed**

(74) Representative: **Vossius & Partner**
**Siebertstrasse 4**
**81675 München (DE)**

# (54) Novel hemoglobin-derived peptide based pharmaceutical compositions

(57) The present invention relates to a pharmaceutical composition comprising at least one Toll-like receptor (TLR)-activating amphiphilic lipid and at least one hemoglobin-derived peptide and optionally a pharmaceutically acceptable carrier. The present invention also relates to the pharmaceutical composition according to the invention for use in preventing and/or treating tumours, preventing and/or treating infections, preventing and/or treating allergies, preventing and/or treating age-related immune imbalances, stimulating the innate and adaptive immune system and/or alleviating the adverse side effects of irradiation. Moreover, the present invention also relates to a hemoglobin-derived peptide as well as its use in treating a bacterial infection.

EP 2 789 343 A1

## Description

The present invention relates to a pharmaceutical composition comprising at least one Toll-like receptor (TLR)-activating amphiphilic lipid and at least one hemoglobin-derived peptide and optionally a pharmaceutically acceptable carrier. The present invention also relates to the pharmaceutical composition according to the invention for use in preventing and/or treating tumours, preventing and/or treating infections, preventing and/or treating allergies, preventing and/or treating age-related immune imbalances, stimulating the innate and adaptive immune system and/or alleviating the adverse side effects of irradiation. Moreover, the present invention also relates to a hemoglobin-derived peptide as well as its use in treating a bacterial infection.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In the past, the transfer of fetal organs, tissues, or cells has been propagated as a preventive or therapeutic measure against diseases such as cancer or severe infections - and also against the process of aging. Extracts of fetal tissue thus appeared to have a beneficial medical effect and at times were recommended particularly in the treatment or prevention of cancer and certain infections. The medically active principles in these fetal tissue extracts have often been elusive, despite considerable interest in the field. Problems with the registration of such compositions are self-evident.

In addition, since more than a century the treatment of cancer by injections of viable and/or heat-killed bacteria was propagated (Coley, W.B.: Am. J. Med. Sci 105, 487-511 (1893)). About 50 years ago it was shown that the tumor-necrotising effect of such treatment is due to bacterial endotoxin. Endotoxins, also called lipopolysaccharides (LPS), are constituents of the cell wall of Gram-negative bacteria. Endotoxins activate the innate immune system and contribute to the activation of the adaptive immune system. During severe Gram-negative infections endotoxins play a key role in the development of septic shock (Rietschel *et al.* 1994). However, low concentrations of LPS and also LPS partial structures, such as monophosphoryl lipid A (MPLA), exhibit effects beneficial for the host. These effects include enhanced resistance to infection and protection against malignancy due to its striking immunostimulatory and immunomodulatory activity (Fox *et al.* 2010). The harmful as well as the beneficial host responses are not induced directly by LPS, but rather mediated by immune modulator molecules such as tumor necrosis factor (TNF), members of the interleukin family (IL-1, IL-6, IL-8, IL-10, IL-12), interferon, reduced oxygen species and lipids. These mediators are released mainly by monocytes/macrophages, but also by other cell types like vascular, epithelial, polymorphonuclear, and mast cells (Galanos *et al.* (1992), Loppnow (1994); Supajatura *et al.* (2001)). Previously, also T cells have been shown to participate in the response to LPS (Mattern *et al.* (1994)).

Fetal hemoglobin (HbF) of sheep or human origin has been found to synergize with LPS and lipid A in the induction of cytokines from murine macrophages and human peripheral blood monocytes *in vitro.* In addition, LPS-induced modulation of innate and adaptive immune responses by HbF was observed in vivo (Gorczynski *et al.* (2006); Howe *et al.* (2008a); WO 2004/073728 A2). This synergistic activity could be shown to mainly reside in the γ-chain of fetal HbF (Hbγ). One explanation for the cooperative effect between Hbγ and lipid A are Hb-induced conformational changes of lipid A, which result in enhanced biological activity. Biophysical analyses showed that the Hb as well as the Hb chains lead to a change of the endotoxic conformation of lipid A into a more conical shape, with an intercalation of the Hb into the endotoxin aggregates followed by a disaggregation process (Howe *et al.* (2008a); Brandenburg *et al.* (2003); Howe *et al.* (2007); Howe *et al.* (2008b)). Recently it has been shown that Hbα- and Hbβ-subunits of Hb have LPS-binding sites. These binding sites were characterized by distinct synthetic peptides obtained from the Hbα- and Hbβ-subunits sequence, which were able to neutralize but not synergize the endotoxic activity of LPS (Bahl *et al.* (2011)).

Despite these developments, the use of full length fetal hemoglobin or single chains thereof has the drawback that said full length proteins have to be provided, either from tissue by means of purification or via recombinant or (semi)synthetic means, all of which are expensive and time-consuming.

Accordingly, there is still a need to provide means that may advantageously be employed in the fight against diseases such as e.g. tumours or severe infections or against aging, that are easy to prepare and that are not associated with the above discussed disadvantages.

This need is addressed by the provision of the embodiments characterized in the claims.

Accordingly, the present invention relates to a pharmaceutical composition comprising at least one Toll-like receptor (TLR)-activating amphiphilic lipid and at least one hemoglobin-derived peptide and, optionally, a pharmaceutically acceptable carrier, wherein the hemoglobin-derived peptide consists of

(a) an amino acid sequence consisting of the sequence of formula I:

A1 -A2-A3-A4-A5-A6-A7-A8-A9-A10-A11-A12-A13-A14-A15-A16-A17-A18-A19 (formula I),

wherein
A1 is selected from the group consisting of threonine, cysteine and serine;
A2 is selected from the group consisting of valine, alanine, leucine, methionine and isoleucine;
A3 is selected from the group consisting of leucine, alanine, isoleucine, methionine and valine;
A4 is selected from the group consisting of alanine, leucine, isoleucine, methionine and valine;
A5 is selected from the group consisting of isoleucine, histidine, alanine, leucine, methionine and valine;
A6 is selected from the group consisting of histidine, arginine and leucine;
A7 is selected from the group consisting of phenylalanine, tryptophan, leucine, isoleucine and valine;
A8 is selected from the group consisting of glycine and alanine;
A9 is selected from the group consisting of lysine, arginine and histidine;
A10 is selected from the group consisting of glutamic acid and aspartic acid;
A11 is selected from the group consisting of phenylalanine, tryptophan, leucine, isoleucine and valine;
A12 is selected from the group consisting of threonine and serine;
A13 is selected from the group consisting of proline and glycine;
A14 is selected from the group consisting of glutamic acid, proline and aspartic acid;
A15 is selected from the group consisting of valine, alanine, leucine, methionine and isoleucine;
A16 is selected from the group consisting of glutamine and asparagine;
A17 is selected from the group consisting of alanine, leucine, isoleucine, methionine and valine;
A18 is selected from the group consisting of serine, alanine and threonine; and
A19 is selected from the group consisting of tryptophane, tyrosine, phenylalanine, leucine, isoleucine and valine;

and
(b) an amino acid sequence located N-terminally of the amino acid sequence of (a) and/or an amino acid sequence located C-terminally of the amino acid sequence of (a),
wherein

(b-i) the amino acid sequence located N-terminally of the amino acid sequence of (a) is selected from the group consisting of:

N1: V;
N2: LV;
N3: VLV;
N4: NVLV;
N5: GNVLV;
N6: LGNVLV;
N7: LLGNVLV;
N8: KLLGNVLV;
N9: FKLLGNVLV;
N10: NFKLLGNVLV;
N11: ENFKLLGNVLV;
N12: PENFKLLGNVLV;
N13: DPENFKLLGNVLV;
N14: VDPENFKLLGNVLV;
N15: HVDPENFKLLGNVLV; and
N16: LHVDPENFKLLGNVLV;

and wherein
(b-ii) the amino acid sequence located C-terminally of the amino acid sequence of (a) is selected from the group consisting of:

(1) an amino acid sequence consisting of the sequence of formula II:

C1-C2-C3-C4-C5 (formula II),

wherein

C1: is selected from the group consisting of glutamine, asparagine and tyrosine;
C2: is selected from the group consisting of lysine, arginine and histidine;

C3: is selected from the group consisting of methionine, valine, alanine, leucine and isoleucine;
C4: is selected from the group consisting of valine, alanine, leucine, methionine and isoleucine; and
C5: is selected from the group consisting of threonine, alanine, serine and lysine;

(2) an amino acid sequence consisting of the sequence of formula III:

C1-C2-C3-C4-C5-C6-C7-C8-C9-C10-C11-C12 (formula III),

wherein

C1 to C5 is defined as in (b-ii)(1);
C6: is selected from the group consisting of alanine, glycine, leucine, isoleucine, methionine and valine;
C7: is selected from the group consisting of valine, alanine, leucine, methionine and isoleucine;
C8: is selected from the group consisting of alanine, leucine, isoleucine, methionine and valine;
C9: is selected from the group consisting of serine, asparagine and threonine;
C10: is selected from the group consisting of alanine, leucine, isoleucine, methionine and valine;
C11: is selected from the group consisting of leucine, glutamine, alanine, isoleucine, methionine and valine; and
C12: is a peptide consisting of the amino acid sequence SSRYH or is absent; and

(3) an amino acid sequence consisting of the sequence of formula IV:

C1-C2-C3-C4-C5-C6-X (formula IV),

wherein

C1 to C5 is defined as in (b-ii)(1);
C6: is selected from the group consisting of alanine, glycine, leucine, isoleucine, methionine and valine;
X: is a peptide consisting of the five amino acid residues alanine, valine, serine, alanine and leucine in any order

or a peptidomometic thereof.

In accordance with the present invention, the term "pharmaceutical composition" relates to a composition for administration to a patient, preferably a human patient. The pharmaceutical composition of the invention comprises at least one Toll-like receptor (TLR)-activating amphiphilic lipid and at least one hemoglobin-derived peptide. The pharmaceutical composition of the present invention may, optionally and additionally, comprise a pharmaceutically acceptable carrier. By "pharmaceutically acceptable carrier" is meant a non-toxic solid, semisolid or liquid filler, diluent, excipient, encapsulating material or formulation auxiliary of any type. Examples of suitable pharmaceutical carriers are well known in the art and include sodium chloride solutions, phosphate buffered sodium chloride solutions, water, emulsions, such as oil/water emulsions, various types of wetting agents, sterile solutions, organic solvents etc. Preferably the carrier is a parenteral carrier, more preferably a solution that is isotonic with the blood of the recipient. The term "parenteral" as used herein refers to modes of administration, which include intravenous, intramuscular, intraperitoneal and subcutaneous injection and infusion. These modes of administration, together with intramuscular, intradermal, intranasal, oral, buccal, sublingual, mucosal (such as e.g. via nasal sprays, nose or eye drops or cremes as well as rectal or vaginal (gel) formulations) or intrabronchial administration, are preferred modes of administration in accordance with the present invention. Most preferred modes of administration in accordance with the present invention are intramuscular administration or oral application. The carrier optionally contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. The pharmaceutical composition may be for administration once or for a regular administration over a prolonged period of time. Generally, the administration of the pharmaceutical composition in form of a single dose should be in the range of for example 0.1 ng/kg of body weight to 20 $\mu$g/kg of body weight of Toll-like receptor (TLR)-activating amphiphilic lipid and for example 0.1 ng/kg of body weight to 200 $\mu$g/kg of body weight for the hemoglobin-derived peptide of the present invention depending on the route of administration. However, a more preferred dosage of each of the active compounds might be in the range of 0.05 - 10 $\mu$g/kg of body weight, and even more preferably in the range of 0.1 - 1 $\mu$g/kg of body weight for a single dose. More specifically, a preferred single dose for i.v. administration may be in the range of 0.1 - 10 ng/kg of body weight, for s.c., i.m., or i.d. administration in the range of 5 - 500 ng/kg of body weight (), or for oral administration up to 20 $\mu$g/kg body weight for Toll-like receptor (TLR)-activating amphiphilic lipid and for the hemoglobin-derived peptide, respectively. Adjustment of these dosages in case of combination of the agent of the present invention with further pharmaceutically active compounds, such as those described elsewhere herein, is within the skills and judgment of the ordinary clinician or physician. Preferably, the weight relation of the components is in the range of 1:1 to 100:1 of hemoglobin-derived peptide to the Toll-like receptor (TLR)-activating amphiphilic lipid.

The components of the pharmaceutical composition to be used for therapeutic administration must be sterile. Sterility is readily accomplished by filtration through sterile filtration membranes (e.g., 0.2 micron membranes), although the present invention is not limited to this exemplary method of achieving sterility. The pharmaceutical composition of the invention is formulated in accordance with national laws and regulatory requirements according to GMP standards.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared, for example, by reconstituting the lyophilized compound(s) using bacteriostatic water-for-injection. Preservatives and other additives may also be present such as, for example, antimicrobials, anti oxidants, chelating agents, and inert gases and the like. The pharmaceutical composition may comprise further agents depending on the intended use of the pharmaceutical composition.

The term "comprising", as used herein, refers to both "consisting of" as well as "encompassing" (i.e. amongst others). With regard to the active ingredients Toll-like receptor (TLR)-activating amphiphilic lipid(s) and hemoglobin-derived peptide(s), this term refers to the presence of either only these two compounds as the active compounds (i.e. consisting of) or the presence of these two types of active compound and additional agents (i.e. comprising/encompassing). Such additional agents may for example be agents known in the art to be effective in the treatment of the diseases described herein below, such as for example agents for use in preventing and/or treating tumours, preventing and/or treating infections, preventing and/or treating allergies, preventing and/or treating age-related immune imbalances, stimulating the innate and adaptive immune system and/or alleviating the adverse side effects of irradiation. These diseases are described in more detail herein below.

Since the pharmaceutical preparation of the present invention relies on the above mentioned compounds, i.e. (a) Toll-like receptor (TLR)-activating amphiphilic lipid(s) in combination with (a) hemoglobin-derived peptide(s), it is preferred that the further agents are only used as a supplement, i.e. at a reduced dose as compared to the recommended dose when used as the only drug, so as to e.g. reduce side effects conferred by said further agents.

The term "at least", as used herein, refers to the specifically recited amount but also to more than the specifically recited amount or number. For example, the term "at least one TLR-activating amphiphilic lipid" encompasses also at least two, at least three, at least four, at least five TLR-activating amphiphilic lipids and so on. Furthermore, this term also encompasses exactly two, exactly three, exactly four, exactly five TLR-activating amphiphilic lipids and so on.

Toll-like receptor (TLR)-activating amphiphilic lipids are well known in the art. Preferably, the TLR-activating amphiphilic lipid is selected from the group consisting of an endotoxin, or an endotoxically active portion thereof, and a bacterial lipopeptide.

The term "endotoxin" (also referred to herein as lipopolysaccharide, LPS) refers to bioactive compounds produced, in general, by Gram-negative bacteria, and constituting a major component of the bacterial outer membrane from which they may be released biologically or chemically. Endotoxins are amphiphilic molecules consisting of a hydrophilic polysaccharide part and a covalently bound hydrophobic lipid component, termed lipid A. In general, the lipid A component is composed of a phosphorylated $\beta$-1,6-linked D-glucosamine disaccharide that carries up to seven acyl residues. Nevertheless, there are variations in the length, position and number of the fatty acids. Lipid A has been shown to constitute the endotoxic principle of LPS, since the biological effects of LPS are reproduced by polysaccharide-free lipid A (Galanos et al. (1985)). This finding was later confirmed by using completely chemically synthesized lipid A and corresponding lipid A partial structures, such as *E. coli* lipid A (Imoto et al. (1987)). Furthermore such synthetic compounds were the basis for investigations on structure-activity relationship of LPS and lipid A (Rietschel et al. (1987)). The minimal requirement for the lipid A bioactivity, relative to the cytokine-inducing capacity, is a molecule consisting of two D-gluco-configurated hexosamine residues, two phosphoryl groups, and six fatty acids as present in *E. coli* lipid A or *H. influenzae* (Rietschel et al. (1994)). From a large body of data concerning structure-activity relationships, it was concluded that the natural form of *E. coli* lipid A represents the optimal configuration for a strong monocyte/macrophage-activating capacity. All chemically different substructures or derivatives of *E. coli* lipid A, whether differing in the phosphorylation or in the acylation pattern of the hexosamine disaccharide, are less or even not active in inducing monokines.

It is understood in accordance with the invention that endotoxin employed in the pharmaceutical composition of the invention may be derived of any Gram-negative, endotoxin carrying bacterium. Preferably, said endotoxin is derived from *Escherichia coli.*

The term "endotoxically active portion" of endotoxin refers to a portions that displays at least 50%, preferably at least 75%, more preferred at least 90% such as at least 95% and most preferred at least 100% of the endotoxic activity of naturally occurring endotoxin. Endotoxically active portions of endotoxin can, for example, be derived by chemical or enzymatic fragmentation of LPS or by (bio)chemical modification of LPS whereby the pharmaceutically beneficial activities are (essentially) maintained or improved. Such endotoxically active portions include fragments produced by partial enzymatic/hydrolytic deglycosylation, desphosphorylation and deacylation, or derivatives generated by the action of glycosyl, phosphoryl or acyltransferases or by other enzymatic modification steps known to take place in mammalian tissue, in particular in the liver. It is preferred that said endotoxically active portion is the LPS-derived polysaccharide-free native or synthetic lipid A component. Most preferred is that the endotoxically active portion is MPLA.

In biophysical investigations it could be shown that all endotoxically highly active lipid A structures posses a particular 'endotoxic conformation', i.e., a conical shape of the lipid A part corresponding to a higher cross-section of the hydrophobic as the hydrophilic region (Seydel *et al.* (2000)). Connected with the conical shape is an inclination of the lipid A diglucosamine backbone with an inclination angle of approximately 50° with respect to the membrane normal, i.e., the direction of the acyl chains (Seydel *et al.* (2000)).

Monophosphoryl lipid A (MPLA) is a partial structure of lipid A obtained e.g. by controlled acid hydrolysis of LPS of *Salmonella minnesota* or by chemical synthesis (Fox *et al.* (2010)). MPLA exhibits only low toxicity and possesses beneficial immunostimulatory properties including adjuvant activity. Therefore, the vaccine adjuvant MPLA is called a "detoxified form of endotoxin". This structure represents the first Toll-like receptor-activating amphiphilic lipid, which is used as vaccine adjuvant in humans (Fox *et al.* (2010)).

Preferably, the endotoxin is bacterial or synthetic S- or R-form lipopolysaccharide (LPS).

In a further preferred embodiment of the pharmaceutical composition of the invention, the endotoxically active portion of endotoxin is natural or synthetic penta- and/or hexaacyl-lipid A. In another preferred embodiment of the pharmaceutical composition of the invention, the endotoxically active portion of endotoxin is natural or synthetic penta- and/or hexaacyl lipid A monophosphate. The use of these specific endotoxins is advantageous as their acute toxicity is significantly lower (about 100 fold) than the toxicity of hexaacyl bisphosphoryl Lipid A or LPS.

The characterization of the *lps* gene of LPS hyporesponder C3J/HeJ mouse strain identified the Toll-like receptor TLR4 as the LPS signalling receptor (Poltorak *et al.* (1998)). For signaling, TLR4 needs the co-expression of an adaptor protein called MD-2 (Shimazu *et al.* (1999)). This protein consists of large disulfide-linked oligomers of dimeric subunits, is produced and secreted by monocytes and dendritic cells, binds closely to TLR4 and is essential for LPS responses (Shimazu *et al.* (1999); Akashi *et al.* (2000)). Using radiolabelled LPS, physical contact between LPS, TLR4, and MD-2 could be demonstrated only when a further molecule, termed CD14, is present (da Silva *et al.* (2001)). CD14 has been shown to be involved in the binding of LPS to TLR4 and activation of cells by LPS (da Silva *et al.* (2001)). CD14 is located as a glycosylphosphatidylinositol(GPI)-anchored membrane protein (mCD14) on the surface of monocytic cells, polymorphonuclear leukocytes, some B-lymphocytes (Haziot *et al.* (1988)), and epithelial cells (Pugin *et al.* (1993)).

Bacterial lipoproteins are constituents of the membrane of Gram-positive and Gram-negative bacteria. In addition, they are also found in the membrane of *Mycoplasma* and *Mycobacteria.* These lipoproteins are composed of di-O-acylated-S-(2,3-dihydroxypropyl)-cysteinyl residues which are coupled to the N-terminus of distinct polypeptides. The S-(2,3-dihydroxypropyl)-cysteine can be N-acylated with a third fatty acid as it is the case in Gram-negative bacteria like *E.coli.* Also monoacylated lipoproteins have been described (Salunke et al. (2012)). Whereas the fatty acid pattern and the amino acid sequence of the polypeptide may vary, the S-(2,3-dihydroxypropyl)-cysteine backbone is an indispensable constituent of these lipoproteins.

Like LPS and MPLA, lipoproteins are amphiphilic molecules having a hydrophobic part (the fatty acids)) and a hydrophilic part, the protein. Fibroblast-stimulating iipopeptide-1(FSL-1) is a synthetic di-acylated lipopeptid derived from *Mycoplasma salvarium.* $PaM_3C$-$SK_4$ is derived from the lipoprotein of *E.coli.* As mentioned above, MLPA signals through the TLR4/MD2 homomeric receptor, whereas bacterial lipoproteins, like $PaM_3C$-$SK_4$ and FSL-1, signal through TLR2 in a TLR1- or TLR6-dependent manner, respectively. Whereas TLR2 induces only the MyD88 signaling pathway, which results in the induction of proinflammatory cytokines, TLR4/MD2 induces the MyD88 pathway and the TRIF pathway. This later pathway mediates the induction of IFN-alpha and IFN-beta.

In a preferred embodiment of the pharmaceutical composition of the invention, the bacterial lipopeptide is a mono-, di- or triacylated lipopeptide having a S-(2,3-dihydroxypropyl)-cysteine backbone. Examples of such lipopeptides are fibroblast-stimulating lipopeptide-1 (FSL-1), macrophage-activating lipopeptide from *Mycoplasma fermentans* (MALP2), $Pam_2C$-$SK_4$, and $Pam_3C$-$SK_4$ Further synthetic modifications are described by e.g. Buwitt-Beckmann et al (Buwitt-Beckmann et al. (2005), FEBS J.) and in the Biochemical Catalogue 2012/2013 of EMC-microcollection (Tübingen, Germany; see the World Wide Web at microcollections.de/pdf/EMC%20Biochemicals.pdf) as listed in the supplements.

The pharmaceutical composition of the present invention further comprises at least one hemoglobin-derived peptide. The term "peptide", as used herein, refers to linear molecular chains of amino acids containing up to 55 amino acids covalently linked by peptide bonds. Peptides may form oligomers consisting of at least two identical or different molecules. The corresponding higher order structures of such multimers are, correspondingly, termed homo- or heterodimers, homo- or heterotrimers etc.. In accordance with the present invention, the sequences indicated extend from the N- to the C-terminus. The one-letter and three-letter code abbreviations as used to identify amino acids throughout the present invention correspond to those commonly used for amino acids.

The peptides of the present invention are derived from hemoglobin, more particularly from human fetal hemoglobin.

Hemoglobin (Hb) is a tetrameric hemeprotein complex of a molecular weight of approx. 64,500 Da. The main biological function of hemoglobin is the transport of oxygen ($O_2$) in the circulation (Perutz (1979)). Adult human Hb (HbA) consists of two $\alpha$- and two $\beta$-chains with each of these subunits containing one heme group as a prosthetic group. Fetal Hb (HbF) consists of two $\alpha$-chains and two $\gamma$-chains in their heme-complexed forms. In human ontogenetic development HbA is synthesized in the bone marrow in the postnatal lifespan, whereas HbF is primarily produced in the liver and spleen of the fetus (Karlsson and Nienhuis (1985)). The term fetal hemoglobin typically denotes the tetrameric forms of HbF as well as heme-free HbF. The $\alpha$-, $\beta$-, and $\gamma$-chains are the monomeric heme-free globin chains derived from HbA and/or HbF.

In accordance with the present invention, the term "derived from hemoglobin" does not necessitate that the peptides are obtained from a full length hemoglobin protein (e.g. by digestion thereof) but instead refers to the fact that the sequence of the peptide(s) of the present invention is a sequence that corresponds to or is similar to a portion of the sequence of hemoglobin.

Accordingly, the peptide of the present invention can be produced synthetically. Chemical synthesis of peptides is well known in the art. Solid phase synthesis is commonly used and various commercial synthesizers are available, for example automated synthesizers by Applied Biosystems Inc., Foster City, CA; Beckman; MultiSyntech, Bochum, Germany etc. Solution phase synthetic methods may also be used, although they are less convenient. For example, peptide synthesis can be carried out using Na-9-fluorenylmethoxycarbonyl amino acids and a preloaded trityl resin or an aminomethylated polystyrene resin with a p-carboxytritylalcohol linker. Couplings can be performed in dimethylformamide using N-hydroxybenzotriazole and 2-(1H-benzotriazole-1-yl)-1,1,3,3-tetramethyluronium hexafluorophosphate. Commonly used side chain protecting groups are tert-butyl for D, E and Y; trityl for N, Q, S and T; 2,2,4,6,7-pentamethyldihydroxybenzofuran-5-sulfonyl for R; and butyloxycarbonyl for K. After synthesis, the peptides are deprotected and cleaved from the polymer support by treatment with e.g. 92% trifluoracetic acid/ 4% triethylsilane/ 4% $H_2O$. The peptides can be precipitated by the addition of tert-butylether/pentane (8:2) and purified by reversed-phase HPLC. The peptides are commonly analysed by matrix-associated laser desorption time-of-flight mass spectrometry. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins may be introduced into the molecule during chemical synthesis. In addition, small molecules and label moieties may be attached during the synthetic process. Preferably, introduction of the functional groups and conjugation to other molecules does not or does only minimally affect the structure and function of the subject peptide.

The N-and C-terminus may be derivatised using conventional chemical synthetic methods. The peptides of the invention may contain an acyl group, such as an acetyl group. Methods for acylating, and specifically for acetylating the free amino group at the N-terminus are well known in the art. For the C-terminus, the carboxyl group may be modified by esterification with alcohols or amidated to form-$CONH_2$ or CONHR. Methods of esterification and amidation are well known in the art. Furthermore, the peptides of the invention may also be produced semi-synthetically, for example by a combination of recombinant and synthetic production. In the case that fragments of the peptide are produced synthetically, the remaining part of the peptide would have to be produced otherwise, e.g. recombinantly as is well known in the art, and then be linked to the fragment to form the peptide of the invention.

Furthermore, the invention encompasses peptidomimetics of the peptide as defined above. A peptidomimetic is a small protein- or peptide-like chain designed to mimic a peptide. Peptidomimetics typically arise from modifications of an existing peptide in order to alter the properties of the peptide. For example, they may arise from modifications to change the stability of the peptide. These modifications involve changes to the peptide that will not occur naturally (such as altered backbones and the incorporation of non-natural amino acids), including the replacement of amino acids or peptide bonds by functional analogues. Such functional analogues include all known amino acids other than the 20 gene-encoded amino acids, such as for example selenocysteine. The use of peptidomimetics as compared to other mimetics has some particular advantages. For instance, their conformationally restrained structure allows to minimize binding to non-target compounds and to enhance the activity at the desired targets. Through the addition of hydrophobic residues and/or replacement of amide bonds the transport of peptidomimetics through cellular membranes can be improved. Furthermore peptidomimetics such as isosters, retro-inverso (all-d retro or retroenantio) peptides and cyclic peptides are less susceptible to degradation by peptidases and other enzymes. Retro-inverso modification of naturally occurring peptides involves the synthetic assemblage of amino acids with $\alpha$-carbon stereochemistry opposite to that of the corresponding L-amino acids, i.e. D- or D-allo-amino acids, in reverse order with respect to the native peptide sequence. A retro-inverso analogue thus has reversed termini and reversed direction of peptide bonds while approximately maintaining the topology of the side chains as in the native peptide sequence.

The amino acid sequence of the hemoglobin-derived peptide of the present invention is as defined herein above. Accordingly, the peptide comprises a core sequence (i.e. formula I) represented by amino acids A1 to A19 as defined. Furthermore, the peptide comprises additional amino acid sequences at the N- or C-terminus, or at both the N- and C-terminus, of said core sequence. The N-terminal sequence is based on the sequence shown as N16, or fragments thereof as shown as N1 to N15. The C-terminal sequence is based on either formula II, formula III or formula IV. Formula II (C1 to C5) represents a fragment of formulas III and IV.

In a more preferred embodiment of the pharmaceutical composition of the invention, the amino acid residues of formula I are as follows:

A1 is selected from the group consisting of threonine and cysteine;
A2 is valine;
A3 is leucine;
A4 is alanine;
A5 is selected from the group consisting of isoleucine and histidine;
A6 is histidine;
A7 is phenylalanine;
A8 is glycine;
A9 is lysine;
A10 is glutamic acid;
A11 is phenylalanine;
A12 is threonine;
A13 is proline;
A14 is selected from the group consisting of glutamic acid and proline;
A15 is valine;
A16 is glutamine;
A17 is alanine;
A18 is selected from the group consisting of serine and alanine; and
A19 is selected from the group consisting of tryptophane and tyrosine.

In accordance with this preferred embodiment, formula I is: A1-VLA-A5-HFGKEFTP-A14-VQA-A18-A19, wherein residues A1, A5, A14, A18 and A19 are as defined above.

In another more preferred embodiment of the pharmaceutical composition of the invention, the amino acid sequence located N-terminally of the amino acid sequence of (a) is selected from the group consisting of N1, N6 and N16.

In a further more preferred embodiment of the pharmaceutical composition of the invention, the amino acid residues of formula II are as follows:

C1: is glutamine;
C2: is lysine;
C3: is selected from the group consisting of methionine and valine;
C4: is valine; and
C5: is selected from the group consisting of threonine and alanine.

In accordance with this preferred embodiment, formula II is: QK-C3-V-C5, wherein residues C3 and C5 are as defined above.

In yet a further more preferred embodiment of the pharmaceutical composition of the invention, the amino acid residues of formula III are as follows:

| | |
|---|---|
| C1 to C5 | is defined as with regard to formula II in the preceding paragraph; |
| C6: | is selected from the group consisting of alanine and glycine; |
| C7: | is valine; |
| C8: | is alanine; |
| C9: | is selected from the group consisting of serine and asparagine; |
| C10: | is alanine; |
| C11: | is selected from the group consisting of leucine and glutamine; and |
| C12: | is a peptide consisting of the amino acid sequence SSRYH or is absent |

In accordance with this preferred embodiment, formula III is: QK-C3-V-C5-C6-VA-C9-A-C11-C12, wherein residues C3, C5, C6, C9, C11 and C12 are as defined above.

In yet a further more preferred embodiment of the pharmaceutical composition of the invention, the amino acid residues of formula IV are as follows:

| | |
|---|---|
| C1 to C5 | is defined as with regard to formulae II and III in the preceding paragraphs; |
| C6: | is selected from the group consisting of alanine and glycine; |
| X: | is a peptide consisting of the amino acid sequence VASAL |

In accordance with this preferred embodiment, formula IV is: QK-C3-V-C5-C6-VASAL wherein residues C3, C5 and C6 are as defined above.

In accordance with the present invention, in those cases where the formulae recite alternatives, any combination of subject-matter covered thereby is considered to be explicitly disclosed. For example, where for a first residue 1 three alternatives A, B and C, are recited, and for a second residue 2 three alternatives D, E and F are recited, and for a third residue 3 three alternatives G, H and I are recited, then it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

The same considerations apply to the combination of different formulae, i.e. it is intended that each embodiment of the core sequence (formula I) can be combined with each embodiment of an N-terminal sequence (as defined in (b-i)) and/or with each embodiment of a C-terminal sequence (as defined in (b-ii)). For example, a core sequence having as the amino acids A1 to A19 in each case the first listed amino acid, i.e. threonine for A1, valine for A2, leucine for A3, alanine for A4, isoleucine for A5 and so on, may be combined with any N-terminal sequence selected from N1 to N16 and/or with any one of the C-terminal sequences recited in (b-ii) above. Accordingly, any amino acid sequence of (a) may for example be combined with N1; any amino acid sequence of (a) may for example be combined with N1 and the amino acid sequence of (b-ii)(1) having the amino acid sequence QKMVT; any amino acid sequence of (a) may for example be combined with N1 and the amino acid sequence of (b-ii)(1) having the amino acid sequence NRVAA; any amino acid sequence of (a) may for example be combined with N1 and the amino acid sequence of (b-ii)(1) having the amino acid sequence QKMVA and so on; any amino acid sequence of (a) may for example be combined with N2 and the amino acid sequence of (b-ii)(1) having the amino acid sequence QKMVT; any amino acid sequence of (a) may for example be combined with N2 and the amino acid sequence of (b-ii)(1) having the amino acid sequence NRVAA; any amino acid sequence of (a) may for example be combined with N2 and the amino acid sequence of (b-ii)(1) having the amino acid sequence QKMVA and so on.

The purified or partially purified components of the pharmaceutical composition can be mixed together by any method known in the art and in any desired proportion (weight), preferably the proportions detailed herein above.

In accordance with the present invention, it was surprisingly found that certain peptides derived from the native human Hbγ1 chain are capable of synergising with endotoxin or bacterial lipoproteins in stimulating purified human monocytes, thereby modulating innate and adaptive immune responses. In particular, the peptides:

| | |
|---|---|
| Hbg-32: | TVLAIHFGKEFTPEVQASWQKMVTAVASAQ (SEQ ID NO:16) |
| Hbg-33: | LGNVLVTVLAIHFGKEFTPEVQASWQKMVT (SEQ ID NO:17) |
| Hbg-34: | VTVLAIHFGKEFTPEVQASW (SEQ ID NO:14) |
| Hbg-35: | TVLAIHFGKEFTPEVQASWQKMVTAVASAL (SEQ ID NO:13) |
| Hbg-36: | LHVDPENFKLLGNVLVTVLAIHFGKEFTPEVQASW (SEQ ID NO:18) |
| Hbg-39: | VTVLAIHFGKEFTPEVQASWQKMVTAVASALSSRYH (SEQ ID NO:15) |

were shown in the appended examples to provide said synergistic effect. Moreover, as is shown e.g. in Figure 13, these peptides provide an enhanced synergistic activity with endotoxin or bacterial lipoproteins as compared to native Hbγ1.

All these peptides have a dominant alpha-helical structure. Without wishing to be bound by theory, the inventors assume that this structure allows the peptides to cross bilayered lipids such as MPLA, thus disaggregating these compounds. Specifically, the sequence TPEVQASWQKMVTAVA, which is present in a large variety of the synergistically active peptides, is assumed to be important for the alpha-helical structure of the Hbg-peptides within the lipid membrane-spanning region. Another structure present in all the synergistically active peptides tested so far is the sequence VLAIHFGKEFTPEVQASW.

However, as is shown in the appended examples, peptides consisting of only these two amino acid sequences were found to be nearly synergistically inactive (see e.g. Fig. 19). This indicated that adjacent amino acids are important for the synergistic activity, as reflected by the presence of N- and/or C-terminal amino acid sequences as defined above.

In addition, amino acid sequence variants based on conservative amino acid substitutions are included in the amino acid sequences defined herein above, as they are considered to have identical or at least similar synergistic activity as the peptide designated in the appended examples as peptide Hbg-35. This is because conservative substitutions, i.e. the substitution of amino acids whose side chains have similar biochemical properties, are considered to not affect peptide function or to affect its function only minimally. A summary of the conservative amino acid replacements of the residues of Hbg-35 is provided in Table 1, below.

Moreover, as shown in Example 5 below, peptides derived from Hbα- Hbβ- and Hbγ2 chain, having the homologous sequence of peptide Hbg-35, were additionally investigated herein. Surprising, only the Hbβ- and Hbγ2 -derived peptides were found to be synergistically active in a similar range as Hbg-35, whereas the Hbα- derived peptide showed only a minimal synergistic activity. One explanation of this finding might lie in the closer amino acid sequence similarity of the Hbβ- and Hbγ2 chain to the Hbγ1 chain in the structural area that forms the basis for the peptides, as compared to Hbα- chain (see e.g. the boxed sequence comparison in Figure 29).

Accordingly, the structure defined herein above encompasses such Hbβ- and Hbγ2 -derived peptides, but no Hbα- derived peptides.

The relatively short peptides in accordance with the present invention can be produced more cost-effective and less time-consuming, thereby providing an advantage over the use of full-length fetal hemoglobin as previously employed in the art.

In a preferred embodiment of the pharmaceutical composition of the present invention, the amino acid sequence of (a) is selected from the group consisting of TVLAIHFGKEFTPEVQASW (SEQ ID NO:1) and CVLAHHFGKEFTPPVQAAY (SEQ ID NO:2).

The sequence of SEQ ID NO:1 is the core sequence of the specific peptides designated in the appended examples as peptides Hbg-32 to Hbg-36, Hbg-39 and Hbg2-70, while the sequence of SEQ ID NO:2 is the core sequence of the specific peptide Hbb-67. As is shown in the appended examples, all these peptides are capable of synergising with endotoxin or bacterial lipoproteins in stimulating purified human monocytes, thereby modulating innate and adaptive immune responses.

In another preferred embodiment of the pharmaceutical composition of the invention, the amino acid sequence of (b-i) is selected from the group consisting of (i) V (sequence N1), (ii) LGNVLV (SEQ ID NO:4) and (iii) LHVDPENFKLLGNVLV (SEQ ID NO:5) and/or the amino acid sequence of (b-ii) is selected from the group consisting of (i) QKMVT (SEQ ID NO:6), (ii) QKVVA (SEQ ID NO:7), (iii) QKMVTAVASAL (SEQ ID NO:8), (iv) QKMVTAVASAQ (SEQ ID NO:9), (v) QKMVTGVASAL (SEQ ID NO:10), (vi) QKVVAGVANAL (SEQ ID NO:11) and (vii) QKMVTAVASALSSRYH (SEQ ID NO:12).

The sequence of N1 is present at the N-terminus of the peptides Hbg-34 and Hbg-39 shown in the appended examples, while SEQ ID NO:4 is present at the N-terminus of the peptide designated in the appended examples as peptide Hbg-33. The sequence of SEQ ID NO:5 is present at the N-terminus of the peptide designated in the appended examples as peptide Hbg-36.

Moreover, the C-terminal sequences are present in the peptides shown in the appended examples as follows: SEQ ID NO:6 is present in Hbg-33, SEQ ID NO:7 is present in Hbb-67, SEQ ID NO:8 is present in Hbg-35, SEQ ID NO:9 is present in Hbg-32, SEQ ID NO:10 is present in Hbg2-70, SEQ ID NO:11 is present in Hbb-67 and SEQ ID NO:12 is present in Hbg-39.

In an even more preferred embodiment of the pharmaceutical composition of the present invention, the hemoglobin-derived peptide consists of an amino acid sequence selected from the group consisting of:

(i) TVLAIHFGKEFTPEVQASWQKMVTAVASAL (SEQ ID NO:13),
(ii) VTVLAIHFGKEFTPEVQASW (SEQ ID NO:14),
(iii) VTVLAIHFGKEFTPEVQASWQKMVTAVASALSSRYH (SEQ ID NO:15),
(iv) TVLAIHFGKEFTPEVQASWQKMVTAVASAQ (SEQ ID NO:16),
(v) LGNVLVTVLAIHFGKEFTPEVQASWQKMVT (SEQ ID NO:17),

(vi) LHVDPENFKLLGNVLVTVLAIHFGKEFTPEVQASW (SEQ ID NO:18),
(vii) TVLAIHFGKEFTPEVQASWQKMVTGVASAL (SEQ ID NO:19), and
(viii) CVLAHHFGKEFTPPVQAAYQKVVAGVANAL (SEQ ID NO:20).

These peptides, the activity of which is shown in the appended examples, are particularly preferred in accordance with the present invention. SEQ ID NO:13 represents the peptide designated in the appended examples as peptide Hbg-35, SEQ ID NO:14 represents the peptide designated in the appended examples as peptide Hbg-34, SEQ ID NO:15 represents the peptide designated in the appended examples as peptide Hbg-39, SEQ ID NO:16 represents the peptide designated in the appended examples as peptide Hbg-32, SEQ ID NO:17 represents the peptide designated in the appended examples as peptide Hbg-33, SEQ ID NO:18 represents the peptide designated in the appended examples as peptide Hbg-36, SEQ ID NO:19 represents the peptide designated in the appended examples as peptide Hbg2-70 and SEQ ID NO:20 represents the peptide designated in the appended examples as peptide Hbb-67. It is further preferred that the hemoglobin-derived peptide does not consist of or comprise the amino acid sequence TVLAIHFGKEFTPEAQASWQKMVTAVASAL (SEQ ID NO:3).

The present invention further relates to the pharmaceutical composition of the invention for use in preventing and/or treating tumours, preventing and/or treating infections, preventing and/or treating allergies, preventing and/or treating age-related immune imbalances, stimulating the innate and adaptive immune system and/or alleviating the adverse side effects of irradiation.

The term "tumour", in accordance with the present invention refers to a class of diseases or disorders characterized by uncontrolled division of cells and encompasses all types of tumours, such as e.g. cancerous tumours and benign tumours as well as solid tumours and non-solid tumours. Cancerous tumours are further characterized by the ability of these tumours to spread, either by direct growth into adjacent tissue through invasion, or by implantation into distant sites by metastasis (where tumour cells are transported through the bloodstream or lymphatic system).

Preferably, the tumour is a cancerous tumor (also referred to as cancer herein). Cancerous tumors that can be treated or prevented by administration of the composition of the present invention include, but are not limited to prostate cancer such as adenocarcinoma of the prostate, breast cancer, ovarian cancer, bladder cancer, salivary gland cancer, endometrium cancer, thyroid cancer, kidney cancer, lung cancer, cancer concerning the upper gastrointestinal tract, colon cancer, colorectal cancer, squamous cell carcinoma of the head and neck or of the cervix, glioblastomas, malignant ascites, lymphomas and leukemias as well as adenocarcinoma of the pancreas.

An infection in accordance with the present invention is the detrimental colonization of a host organism by a foreign species. In an infection, the infecting organism seeks to utilize the host's resources in order to multiply (usually at the expense of the host). The host's response to infection is inflammation. Infections include e.g. infections by bacteria, viruses and eukaryotic organisms, either of single-cell or of multi-cell structure such as yeast cells, fungi, helminths etc.

Bacterial infections, or conditions arising therefrom, in accordance with the present invention include but are not limited to bacterial meningitis, cholera, diphtheria, listeriosis, pertussis (Whooping Cough), pneumococcal pneumonia, salmonellosis, tetanus, typhus or urinary tract infections.

Viral infections, or conditions arising therefrom,in accordance with the present invention include but are not limited to mononucleosis, AIDS, chickenpox, common cold, cytomegalovirus infection, dengue fever, ebola haemorrhagic fever, hand-foot and mouth disease, herpes, hepatitis, influenza, mumps, poliomyelitis, rabies, smallpox, viral encephalitis, viral gastroenteritis, viral meningitis, viral pneumonia or yellow fever.

Fungal infections, or conditions arising therefrom, in accordance with the present invention include but are not limited to aspergillosis, blastomycosis, candidiasis, coccidioidomycosis, cryptococcosis, histoplasmosis or tinea pedis.

Particularly preferred in accordance with the present invention is that said infections are viral infections, preferably chronic viral infections, more preferred herpes, hepatitis B or hepatitis C infections.

Allergies, in accordance with the present invention, include type-1 allergies, hay fever and allergic asthma.

Age-related immune imbalances, in accordance with the present invention, relate to the usually irreversible generation of immune-imbalances over time. In a preferred embodiment of the pharmaceutical composition of the invention, said age-related immune imbalances comprise abnormal cytokine production. In a more preferred embodiment of the pharmaceutical composition of the present invention, said age-related abnormal cytokine production is an increased TNF$\alpha$, IL-1, IL-4, IL-6, IL-8 and/or IL-10 production and/or a decreased IL-2 production. It is further preferred in accordance with the pharmaceutical composition of the present invention that said preventing and/or treating of age-related immune imbalances is related to an activation of macrophages.

Optionally, the pharmaceutical composition of the present invention may be administered together with further pharmaceutically active compounds developed to halt or retard the physiological or pathophysiological consequences of aging including increased susceptibility to tumours, infection and allergy.

Medical application of LPS including treatment of tumors and prevention of infections was propagated since more than a century ago, but was often hampered by side reactions such as fever and hypotension. Recently, it was shown by the present inventors that a preparation comprising endotoxin and fetal hemoglobin strongly activate macrophages, induce tumor cytotoxicity, inhibit tumor growth, and reverse an aged-associated immune status to that of young individuals (WO 2004/073728). These preparations were also found suitable as a means to block adverse reactions of irradiation, for example, as adjuvants to avoid or reduce adverse side effects in the treatment of tumours by irradiation.

As shown in the appended examples, the peptides of the present invention provide an enhanced synergistic activity with TLR-activating amphiphilic lipids, in particular endotoxin or bacterial lipoproteins, as compared to native Hbγ1 (see e.g. Figure 13) and, consequently, may advantageously be used in the above recited medical approaches.

The present invention further relates to a hemoglobin-derived peptide consisting of:

(a) the amino acid sequence TVLAIHFGKEFTPEVQASW (SEQ ID NO:1); and
(b) an amino acid sequence located N-terminally of the amino acid sequence of (a) and/or an amino acid sequence located C-terminally of the amino acid sequence of (a),
wherein

(b-i) the amino acid sequence located N-terminally of the amino acid sequence of (a) is selected from the group consisting of:

(i) V (SEQ ID NO:3),
(ii) LGNVLV (SEQ ID NO:4) and
(iii) LHVDPENFKLLGNVLV (SEQ ID NO:5) and/or

and
(b-ii) the amino acid sequence located C-terminally of the amino acid sequence of (a) is selected from the group consisting of:

(i) QKMVT (SEQ ID NO:6),
(ii) QKMVTAVASAL (SEQ ID NO:8),
(iii) QKMVTAVASAQ (SEQ ID NO:9),
(iv) QKMVTAVASALSSRYH (SEQ ID NO:12).

This novel hemoglobin-derived peptide of the present invention may advantageously be used in any of the applications described herein above. Accordingly, the definitions as well as the preferred embodiments provided herein above with regard to the pharmaceutical composition apply *mutatis mutandis* also to the embodiments relating to the hemoglobin-derived peptide of the present invention. For example, preferred embodiments of the preparation, administration or use of the pharmaceutical composition have their counterparts in preferred embodiments of the above defined peptide.

In a preferred embodiment of the hemoglobin-derived peptide of the invention, the peptide consists of an amino acid sequence selected from the group consisting of:

(i) TVLAIHFGKEFTPEVQASWQKMVTAVASAL (SEQ ID NO:13),
(ii) VTVLAIHFGKEFTPEVQASW (SEQ ID NO:14),
(iii) VTVLAIHFGKEFTPEVQASWQKMVTAVASALSSRYH (SEQ ID NO:15),
(iv) TVLAIHFGKEFTPEVQASWQKMVTAVASAQ (SEQ ID NO:16),
(v) LGNVLVTVLAIHFGKEFTPEVQASWQKMVT (SEQ ID NO:17) and
(vi) LHVDPENFKLLGNVLVTVLAIHFGKEFTPEVQASW (SEQ ID NO:18).

In a more preferred embodiment of the hemoglobin-derived peptide of the invention, the peptide is for use in treating a bacterial infection.

Lipoproteins are part of the cell membranes of Gram-negative bacteria, Gram-positive bacteria, mycoplasma and mycobacteria. Moreover, also endotoxins are part of the outer membrane of the cell wall of Gram-negative bacteria. Accordingly, the peptide of the present invention may be administered on its own to patients infected with such pathogens, in order to benefit from the synergistic, therapeutic effect of the combination of the inventive peptide with lipoproteins or endotoxins. Additional components may also be administered to the patient, such as e.g. a pharmaceutically acceptable carrier or adjuvant. All of the definitions as well as the preferred embodiments provided herein above with regard to the pharmaceutical composition accordingly also apply to this embodiment relating to the hemoglobin-derived peptide of the present invention for use in treating a bacterial infection.

Non-limiting examples of Gram-negative pathogens causing a bacterial infection amenable to treatment with the inventive peptide include *Escherichia coli, Salmonella, Shigella, Pseudomonas, Neisseria, Haemophilus influenzae, Bordetella pertussis* and *Vibrio cholerae.* In addition, non-limiting examples of Gram-positive pathogens causing a bacterial infection amenable to treatment with the inventive peptide include the genera *Staphylococcus, Streptococcus, Enterococcus" Bacillus, Clostridium* and *Listeria,* while non-limiting examples of Mycoplasma causing a bacterial infection amenable to treatment with the inventive peptide include *Mycoplasma fermentans and Mycoplasma salvarium* and non-limiting examples of Mycobacteria causing a bacterial infection amenable to treatment with the inventive peptide include *Mycobacterium tubercolosis, Mycobacterium bovis, Mycobacterium leprae, and Mycobacterium avium.*

In accordance with the present invention, in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed and also the combination of the subject-matter of claims 3, 2 and 1 is clearly and unambiguously disclosed. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:

**Figure 1:** N-terminal lysine-linked Hbγ1-peptides used for screening. The figure shows the amino acid sequence of the human Hbγ1-protein. This sequence was subdivided in 27 overlapping peptides consisting of 15 or 16 amino acids. Each peptide was N-terminally linked to 5 lysines.

**Figure 2:** Screen test: Effect of $K_5$-Hbγ1 peptides on stimulation of MNC with MPLA. Human MNC were stimulated with MPLA (10 μg/ml) in the presence or absence of the peptides of the $K_5$-Hbγ1-peptide library (10 μM) shown in Figure 1. Control cultures were kept without MPLA. After a culture period of 20 h, the IL-8 concentration in the supernatant was determined by ELISA. Each value represents the mean of three independent experiments. In each of these three individual experiments duplicate cultures have been performed.

**Figure 3:** Hbγ1-peptides used for screening. The figure shows the amino acid sequence of the human Hbγ1-protein. This sequence was subdivided in 27 overlapping peptides consisting of 15 or 16 amino acids.

**Figure 4:** Screen test: Effect of Hbγ1-peptides on stimulation of MNC with MPLA. Human MNC were stimulated with MPLA (10 μg/ml) in the presence or absence of the peptides of the Hbγ1-peptide library (10 μM) shown in Figure 3. Control cultures were kept without MPLA. After a culture period of 20 h, the IL-8 concentration in the supernatant was determined by ELISA. Each value represents the mean ± SD of duplicate cultures.

**Figure 5:** Stimulation of human MNC with MPLA in the presence of various Hbg-peptides. Human MNC were stimulated with MPLA (1 μg/ml) in the presence or absence of Hbg-23-110, Hbg-24-111, and Hbg-34-112 at a concentration of 1, 3, and 10 μM. Control cultures were kept without MPLA. After a culture period of 20 h, the IL-6 concentration in the supernatant was determined by ELISA. Each value represents the mean ± SD of duplicate cultures.

**Figure 6:** The 3 long-chain overlapping peptides derived from a partial sequence of the Hbγ1-protein. Hbg-30-105 ($Hb\gamma_{86-115}$), Hbg-31-106 ($Hb\gamma_{99-28}$), and Hbg-32-107 ($Hb\gamma_{112-141}$).

**Figure 7:** Synergistic effect of Hbγ1-peptides on stimulation of human MNC with MPLA. Human MNC were stimulated with MPLA (3 μg/ml) in the presence or absence of the Hbγ1-derived peptides Hbg-23, Hbg-24, Hbg-30-105, Hbg-31-106, and Hbg-32-107 (3 and 10 μM). Control cultures were kept without MPLA. After a culture period of 20 h, the IL-6 concentration in the supernatant was determined by ELISA. Each value represents the mean ± SD of duplicate cultures.

**Figure 8:** Effect of Hbγ1-peptides on stimulation of human MNC with MPLA in the presence or absence of human serum. Human MNC were stimulated with MPLA (3 μg/ml) in the presence or absence of the Hbγ1-peptides Hbg-23 and Hbg-32-107 (1, 3 and 10 μM). Control cultures were kept without MPLA. The culture medium was supplemented with or without 10 % of human serum. After a culture period of 20 h the IL-6 concentration in the supernatant was determined by ELISA. Each value represents the mean ± SD of duplicate cultures.

**Figure 9:** Synergistic effect of Hbg-23 and Hbg-32 peptide on stimulation of human monocytes with MPLA. Purified human monocytes (> 95 %) were stimulated with MPLA (1 μg/ml) in the presence or absence of the Hbγ1-peptides Hbg-23 and Hbg-32-107 (1, 3 and 10 μM). Control cultures were kept without MPLA. After a culture period of 20 h, the IL-6 concentration in the supernatant was determined by ELISA. Each value represents the mean ± SD of duplicate cultures.

**Figure 10:** Stimulation of human MNC with MPLA in the presence of three different preparations of synthetic Hbg-32 peptides. Human MNC were stimulated with MPLA (3 μg/ml) in the presence or absence of three different preparations of the Hbγ1-peptide Hbg-32, namely Hbg-32-107, Hbg-32-108, and Hbg-32-KB (1, 3, and 10 μM). Control cultures were kept without MPLA. After a culture period of 20 h, the IL-6 concentration in the supernatant was determined by ELISA. Each value represents the mean ± SD of duplicate cultures.

**Figure 11:** Synergistic effect of Hbg-32 peptide with MPLA during induction of various cytokines in human MNC. Human MNC were stimulated with MPLA (3 μg/ml) in the presence or absence of Hbg-32-113 (3, and 10 μM). Control cultures were kept without MPLA. After a culture period of 20 h, the IL-1β, IL-6, IL-8, and TNFα concentration in the supernatant was determined by ELISA. Each value represents the mean ± SD of duplicate cultures.

**Figure 12:** Synergistic effects of Hbg-32 on the stimulation of human MNC with MPLA and LPS. Human MNC were stimulated with MPLA (3 μg/ml) or LPS (1ng/ml) in the presence or absence of Hbg-32-113 (1, 3, and 10 μM). Control cultures were kept without MPLA or LPS. After a culture period of 20 h, the IL-6 concentration in the supernatant was determined by ELISA. Each value represents the mean ± SD of duplicate cultures.

**Figure 13:** Comparison of the synergistic effect of Hbg-32-107 and native Hbg-γ1 chain on stimulation of human MNC with MPLA. Human MNC were stimulated with MPLA (3 μg/ml) in the presence or absence of the native Hbγ1-chain protein and the Hbγ1-peptide Hbg-32-107 (3 and 10 μM). Control cultures were kept without MPLA. After a culture period of 20 h, the IL-8 concentration in the supernatant was determined by ELISA. Each value represents the mean ± SD of duplicate cultures.

**Figure 14:** Comparison of the $Hb\gamma_{112\text{-}141}$ peptide (Hbg-32-113) with the $Hb\gamma1_{112\text{-}141[L141Q]}$ peptide (Hbg-35-114) during stimulation of human MNC with MPLA. Human MNC were stimulated with MPLA (3 μg/ml) in the presence or absence of $Hb\gamma1_{112\text{-}141}$ peptide (Hbg-32-113) or the $Hb\gamma1_{112\text{-}141[L141Q]}$ peptide (Hbg-35-114) (1, 3, and 10 μM). Control cultures were kept without MPLA. The cell culture was performed in (A) absence of human serum or (B) presence of 10 % human serum. After a culture period of 20 h, the IL-6 concentration in the supernatant was determined by ELISA. Each value represents the mean ± SD of duplicate cultures.

**Figure 15:** Synergistic effect of various Hbg-γ1 peptides on stimulation of human MNC with MPLA. Human MNC were stimulated with MPLA (3 μg/ml) in the presence or absence of various Hbg-peptides, namely Hbg-35-114 (Hbγ1112-141), Hbg-33-109 (Hbγ1106-135), Hbg-34-112 (Hbγ1111-130), Hbg-36-115 (Hbγ196-130) at 1, 3 and 10 μM. Control cultures were kept without MPLA. After a culture period of 20 h, the IL-6 concentration in the supernatant was determined by ELISA. Each value represents the mean ± SD of duplicate cultures.

**Figure 16:** Synergistic effect of various Hbg-γ1 peptides on stimulation of human MNC with MPLA. Human MNC were stimulated with MPLA (3 μg/ml) in the presence or absence of various Hbg-peptides, namely Hbg-35-114 (Hbγ1112-141), Hbg-38-117 (Hbγ1106-140), and Hbg-39-118 (Hbγ1111-146) at 1, 3 and 10 μM. Control cultures were kept without MPLA. After a culture period of 20 h, the IL-6 concentration in the supernatant was determined by ELISA. Each value represents the mean ± SD of duplicate cultures.

**Figure 17:** Alanine-Scan of the Hbg-35 peptide. Each amino acid of the Hbg-35 sequence was individually replaced by alanine. The figure shows the sequence of the peptide synthesized.

**Figure 18:** Effect of peptides, derived from the Hbg-35 alanine-scan, on MPLA-stimulated human MNC. Human MNC were stimulated with MPLA (3 μg/ml) in the presence or absence of various Hbg-peptides derived from the Hbg-35 Alanine-scan (3 μM). Control cultures were kept without MPLA. After a culture period of 20 h, the IL-6 concentration in the supernatant was determined by ELISA. Each value represents the mean ± SEM two individual experiments performed with duplicate cultures. * $p < 0.05$ significance in relation to Hbg-35 (Mann-Whitney Rank Sum Test or paired Student's t-test, as appropriate).

**Figure 19:** Synergistic effect of various Hbg-γ1 peptides on stimulation of human MNC with MPLA. Human MNC were stimulated with MPLA (1 μg/ml) in the presence or absence of various Hbg-peptides, namely Hbg-35-114 (Hbγ1112-141), Hbg-68-155 (Hbγ1[113-130]) Hbg-69-156 (Hby1 [123-138]) at 0.1, 1, 3 and 10 μM. Hbg-68-155 represents the common part structure of peptides Hbg-32 to Hbg-39. The sequence of Hbg-69-156 is assumed to be responsible for the alpha-helical structure of Hbg-peptides within the lipid membrane-spanning region. Control cultures were kept without MPLA. After a culture period of 20 h, the IL-6 concentration in the supernatant was determined by ELISA. Each value represents the mean ± SD of duplicate cultures.

**Figure 20:** Adjuvant effect of MPLA during stimulation of memory T-cells with recall antigens. Human MNC were stimulated with purified protein derivative of *M. tuberculosis* (PPD) (1 μg/ml) or tetanus toxoid (TT) (0.1 limits of flocculation[LF]/ml) in the presence or absence of MPLA (10, 100, and 1000 ng/ml). After 6 days of culture, cells were labeled with $^3$H-Thymidine (3HTdR; 2 Ci/mmol, 0.2 μCi/culture) and after an additional day of culture the cells were harvested on glass-filter mats for measurement of incorporated radioactivity into the DNA. The results are expressed as mean ± SD of duplicate cultures.

**Figure 21:** Synergistic effect of Hbg-35 on the adjuvant activity of MPLA during stimulation of human memory T-cell with PPD and TT. Human MNC were stimulated with PPD (1 μg/ml) or TT (0.1 LF/ml) in the presence or absence of MPLA (10 ng/ml) and Hbg-35 (1 or 3 μM). After 6 days of culture, cells were labeled with 3HTdR (2 Ci/mmol, 0.2 μCi/culture) and after an additional day of culture the cells were harvested on glass-filter mats for measurement of incorporated radioactivity into the DNA. The results are expressed as mean ± SD of duplicate cultures.

**Figure 22**: Small angle X-ray scattering pattern of MPLA alone (A) or in the presence of Hbg-32 (B). The logarithm of the scattering intensity is plotted versus the scattering vector s (=1/d, d = spacings). In the absence of the peptide, the sharp reflection at 4.11 nm indicates the existence of the multi-lamellar periodicity, in the presence of the peptide the occurrence of the various additional reflections is characteristic for the existence of a cubic aggregate structure.

**Figure 23**: Förster resonance energy transfer spectroscopy (FRET) of MPLA aggregates, to which Hbg-32 was added. The MPLA aggregates were labeled with the dyes NBD-PE (donor) and RhoPE (acceptor), and the intensity ratio $I_D/I_A$ is a sensitive measure of the incorporation of an external compound such as the Hbg-32 peptide observed here.

**Figure 24**: Laser light scattering at 90° of a MPLA dispersion alone or in the presence of Hbg-32. Broken line: MPLA alone (0.1 mM). Solid line: MPLA (0.1 mM) + Hbg-32 (0.1 mM). A clear disaggregating effect of MPLA by the peptide is exhibited by a change of sizes around 1000 nm (right peak) for MPLA alone and a decrease in the presence of Hbg-32 at equimolar concentration (left two peaks).

**Figure 25**: Freeze fracture electron microscopy of MPLA (1 mM) alone (A) and in the presence of Hbg-32 (B) at an equimolar concentration. In the absence of the peptide, the MPLA aggregates are densely packed forming large multi-bilayered arrangements. In the presence of the peptide, these aggregates are dispersed and become more spherical.

**Figure 26**: Atomic force microscopy of MPLA in the presence or absence of Hbg-32. (A) Atomic force microscopic picture of MPLA (25 μM) on a mica surface Presented is the depth profile of a x-y = 5.0 x 5.0 μm2 section of the lipid aggregate. The different relief shading represent the different heights of the MPLA assembly showing compact layers between z = 50 and 300 nm. (B) Atomic force microscopic picture of MPLA (25 μM) in the presence of an equimolar content of Hbγ32 on mica. Presented is the depth profile of a 5.0 x 5.0 μm2 section of the lipid aggregate. The different relief shading represent the different heights of the MPLA assembly with maximum heights up to 10 nm. More than 90 % of the plane shows bilayered structures of approximately 5 nm thickness.

**Figure 27**: Isothermal calorimetric titration of monophosphoryl lipid A (MPLA) with Hbγ35. Isothermal calorimetric titration of MPLA with Hbγ35 in dependence on time (top) and the resulting enthalpy change ΔH was determined as a function of the molar ratio of peptide to MPLA. The peptide was titrated in 3 μl portions every 5 min into the MPLA-containing cell under constant stirring, and the heat of interaction after each injection measured by the ITC instrument was plotted versus time.

**Figure 28**: Infrared spectrum for Hbγ35 alone and in the presence of MPLA. The infrared spectrum was measured in the range of the amide I vibrational band (predominantly C=O stretching vibration) between 1700 and 1590 $cm^{-1}$ for Hbγ35 alone (top) and in the presence of an equimolar content of MPLA (bottom). The absorbance maxima around 1659 and 1628 $cm^{-1}$ are indicative for the existence of α-helical and β-sheet secondary structures, respectively.

**Figure 29**: Comparison of the synergistic effect of Hbg-35 with homologous peptides of the Hbα-, Hbβ-, and Hbγ2 chain during stimulation of human MNC with MPLA. Human MNC were stimulated with MPLA (3000 ng/ml) in the presence or absence of various Hb-peptides, namely Hbg-35-158 (Hby1[112-141]), Hba-66-153 (Hbα[107-136]), Hbb-67-154 (Hbβ[112-141]), and Hbg2-70-157 (Hbγ2[112-141] ) at 1, 3, and 10 μM. Control cultures were kept without MPLA. After a culture period of 20 h, the IL-6 concentration in the supernatant was determined by ELISA. Each value represents the mean ± SD of duplicate cultures.

**Figure 30**: Synergistic effect of the Hbg-35 peptide on stimulation of human MNC with synthetic lipopeptides. Human MNC were stimulated with Pam3C-SK4 (10 nM), FSL-1 (1 nM), or MPLA (3 μg/ml) in the presence or absence of Hbg-35 at 1, 3 and 10 μM. Control cultures were kept without lipopeptide or MPLA. After a culture period of 20 h, the IL-6 concentration in the supernatant was determined by ELISA. Each value represents the mean ± SD of duplicate cultures.

The examples illustrate the invention:

**Example 1: Materials and Methods**

*Hbγ, peptides, and MPLA*

All synthetic peptides were prepared and obtained from EMC microcollections GmbH (Tübingen, Germany). The short length peptides (15-16 amino acids) were purified by precipitation and their structures were verified by MS analysis. The long length peptides were purified by HPLC and declared to have a purity of more than 95%. The peptides of the peptide libraries were dissolved in DMSO at a concentration of 10 mM. All other peptides were dissolved in 0.9 % NaCl at a concentration of 10 mM. The peptides derived from the Hbγ chain were named as Hbg-##-***. The ##-numbers refer to the peptide number, and the ***-numbers refer to the lot number. Correspondingly, peptides derived from the Hbα chain or the Hbβ chain were named Hba and Hbb, respectively.

Chemical purified native Hbγ chains were prepared by Prof. J.P Mach (Lausanne). They were dissolved in 0.9 % NaCl at a concentration of 100 μM.

MPLA was obtained from Avanti Polar Lipids, Inc. (Alabaster, Alabama USA). MPLA was dissolved in *aqua ad injectabilia* (B. Braun Melsungen AG, Melsungen, Germany) by five cycles of vortexing (30 min) and incubation in a sonic bath (15 min) at a concentration of 250 μg/ml.

*Cell cultures*

Human mononuclear cells (MNC) were isolated from peripheral blood of healthy donors by density-gradient centrifugation on Ficoll gradient media (PAA Laboratories GmbH, Pasching, Austria). The cells were suspended and cultured in RPMI-1640 medium, containing 1 μg/ml of penicillin and 100 U/ml of streptomycin. 50 μl of a peptide solution were incubated with 50 μl of a MPLA solution in a U-form 96 well micro cell culture plate #3799 (Corning Costar GmbH, Bodenheim, Germany) for 15 min at 37°C. After this incubation period 20 μl of heat-inactivated (56°C / 30 min) human serum or RPMI-1640 medium were added. Finally, 80 μl of a MNC cell suspension was added to give a final concentration of 1 to 2 x $10^6$ cells for culture in the absence of human serum or 1 x $10^6$ cells for culture in the presence of serum. After a culture time of 20 h the cells were spun down and the culture supernatants were harvested. The cytokine concentrations in the supernatants were determined by matched antibody pairs for ELISA obtained from Invitrogen GmbH, Darmstadt (Germany). Mostly, each value represents the mean ± SD of duplicate culture. In Figure 3 the mean of three independent experiments is given, each of these three independent experiments having duplicate cultures.

For stimulation of T-lymphocytes, human MNC were cultured in RPMI-1640 medium, containing 1 μg/ml of penicillin, 100 U/ml of streptomycin, and 10% of inactivated human serum at a concentration of 0.5 to 1.0 x $10^6$ cells per ml in flat-bottom 96-well tissue culture plates. To investigate the adjuvant effect of MPLA and Hbg-peptides, the recall antigens tetanus toxoid (TT) and purified protein derivatives of *M. tuberculosis* (PPD) (both from Statens Serum Institute, Copenhagen, Danmark) were used. After 6 days of culture, cells were labeled with $^3$H-Thymidine (3HTdR (2 Ci/mmol, 0.2 pCi/culture)) and after an additional day of culture the cells were harvested on glass-filter mats for measurement of incorporated radioactivity into the DNA. The results are expressed as mean ± SD of duplicate cultures.

For transfection and culture of HEK293 cells the cells were plated at a density of 1.5x$10^5$/ml in 96 well plates in DMEM supplemented with 10 % FCS, 0.5 units/ ml penicillin and 0.5 μg/ ml streptomycin. The following day, cells were transiently transfected using Polyfect (Quiagen, Hilden, Germany) according to the manufacturer's protocol. Expression plasmids containing huTLR4, huMD2, and huCD14 were used at 200 ng per transfection. After 6 h of transfection, cells were washed and stimulated for further 20 h with ligands as indicated in the figures. The IL-8 content in the culture supernatants were quantified using matched antibody pairs for ELISA obtained from Invitrogen GmbH, Darmstadt (Germany). Each value represents the mean ± SD of duplicate cultures.

Statistic analyses were performed using paired Student's *t*-test or Mann-Whitney Rank Sum Test as appropriate with the aid of Sigma Plot software. Differences were considered to be significant at $p < 0.05$.

*Laser light scattering*

The sizes and size distributions of MPLA suspensions (0.1 mM) in the absence and presence of the Hbg-32 peptide at different concentrations were performed on a Malvern particle sizer (Malvern, Herrsching, Germany). The light scattering intensity was monitored at a scattering angle of 90°, and the intensity was recorded and evaluated by assuming spherical particle sizes.

*Freeze-fracture transmission electron microscopy (FFTEM)*

A small amount of the samples was sandwiched between two copper profiles and frozen by plunging the sandwiches immediately into liquefied ethane-propane-mixture cooled in liquid nitrogen. Fracturing and replication were performed at -150 °C in a BAF 400T freeze-fracture device (BAL-TEC, Balzers, Liechtenstein) equipped with electron guns and a film sheet thickness monitor. For replication Pt(C) was evaporated under an angle of 35° and (C) under 90°. The replicas were placed on copper grids and cleaned with a chloroform-methanol mixture.

*Small angle X-ray scattering (SAXS)*

SAXS measurements were performed at the European Molecular Biology Laboratory (EMBL) outstation at the Hamburg synchrotron radiation facility HASYLAB using the camera X33. Diffraction patterns in the range of the scattering vector $0.1 < s < 1.0$ $nm^{-1}$ ($s = 2 \sin \theta/\lambda$, $2\theta$ scattering angle and A the wavelength = 0.15 nm) were recorded at 40 °C with exposure times of 1 min using an image plate detector with online readout (MAR345, MarResearch, Norderstedt/Germany). The lipid concentration was in all cases 20 mM. The s-axis was calibrated with Ag-behenate, which has a periodicity of 58.4 nm. The diffraction patterns were evaluated as described previously (Howe *et al.* (2008a)), assigning the spacing ratios of the main scattering maxima to defined three-dimensional structures. The lamellar and cubic structures are the most relevant here. The following features characterize the supramolecular structure:

(1) lamellar: The reflections are grouped in equidistant ratios, i.e., 1, 1/2, 1/3, 1/4, etc. of the lamellar repeat distance $d_\lambda$;
(2) cubic: The different space groups of these non-lamellar three-dimensional structures differ in the ratio of their spacings. The relation between reciprocal spacing $s_{hkl}$ = 1/dhkl and lattice constant a is

$$s_{hkl} = [(h^2 + k^2 + l^2)/a]^{1/2}$$

(hkl = Miller indices of the corresponding set of plane).

*Förster (fluorescence) resonance energy transfer spectroscopy (FRET)*

Intercalation of Hbg-32 into MPLA aggregates was determined by FRET spectroscopy applied as a probe dilution assay. MPLA was labelled with the donor dye NBD-phosphatidylethanolamine (NBD-PE) and the acceptor dye Rhodamine-PE. The donor dye was excited at 470 nm, and its emission intensity measured at 531 nm. In the FRET methodology, the donor emission fluorescence intensity is transferred in a radiation less process to the acceptor, which has as an excitation wavelength corresponding to the donor emission wavelength (531 nm). The Hbγ peptide was added to MPLA (0.01 mM) at a final concentration of 0.01 or 1 mM. Intercalation was monitored as the increase of the ratio of the donor emission intensity $I_d$ at 531 nm to that of the acceptor emission intensity $I_A$ [at 593 nm (FRET signal) in a time-dependent manner.

*Atomic force microscopic analyses*

Atomic force microscopy (AFM) allows a direct look onto the morphology of lipid aggregates on a molecular scale under almost physiological conditions. This allows obtaining depth profiles of MPLA dispersions down to a resolution limit of lower than 1 nm. For the analysis, MPLA dispersions were prepared at a concentration of 25 μM in physiological saline in the absence and presence of an equimolar content of Hbg-32.

*Isothermal Titration Calorimetry (ITC)*

Microcalorimetric measurements of peptide binding to MPLA were performed on a MCS isothermal titration calorimeter (Microcal Inc.) at 37 °C as recently described (Kaconis et al. (2011)). MPLA (0.05 mM) - prepared as described above - was dispensed into the microcalorimetric cell (volume 1.3 ml) and the peptide solutions (1 mM) were filled into the syringe compartment (volume 100 $\mu$l). After temperature equilibration, the peptides were titrated in 3 $\mu$l portions every 5 min into the lipid-containing cell under constant stirring, and the heat of interaction after each injection measured by the ITC instrument was plotted versus time. In this assay, endothermic reactions between the two ligands lead to peaks upward and exothermic reactions to one downward.

*Fourier-transform infrared spectroscopy*

The infrared spectroscopic measurements were performed on an IFS-55 spectrometer (Bruker). Peptide and MPLA:peptide mixtures were dispersed in 20 mM Hepes buffer, pH 7.0, and spread on an ZnSe attenuated total reflectance (ATR) unit. After evaporating of free buffer solution, 100 interferograms of the samples were accumulated, apodized, Fourier-transformed, and converted to absorbance spectra. The infrared spectra were evaluated in the range of the amide I vibration (predominantly C=O stretching vibration), the peak positions of which are characteristic for the secondary structure of the Hb$\gamma$35 peptide. Usually, $\alpha$-helical structures are found at a peak position of 1655 to 1662 $cm^{-1}$, while $\beta$-sheet structures show a main absorbance maximum around 1630 $cm^{-1}$.

**Example 2: Synergism between synthetic Hb$\gamma$-derived peptides and MPLA**

A peptide library of 27 overlapping human (hu) Hb-derived peptides from the known huHb-$\gamma$1 amino acid sequence (see the world wide web at uniprot.org/uniprot/P69891) having 15 or 16 amino acids each was generated. The peptides were N-terminally linked to 5 lysine residues to enhance solubility of the peptides in aqueous solutions (Fig.1). It was found, that several of these lysine-coupled peptides expressed enhancing activity during stimulation of MNC with MPLA (Fig. 2). However, some of these peptides also have a considerable stimulatory activity in the absence of MPLA (Fig. 2) and, in addition, lysine-coupled peptides are known to activate polymorphic nuclear leucocytes in a nonspecific way. Therefore, a corresponding library of 27 native (i.e. non-modified) overlapping peptides (Fig. 3) was generated instead. Several native peptides of these native Hb$\gamma$-derived peptides were found to express synergistic activity during stimulation of MNC with MPLA (Fig. 4). Especially peptides No. 23 (Hb$\gamma_{11\text{-}125}$) and No. 24 (Hb$\gamma_{116\text{-}130}$) exhibited profound synergistic activities in enhancing the MPLA-induced cytokine release in human MNCs. None of these native peptides expressed any cytokine-induction capacity in the absence of MPLA.

The peptides Hbg-23 and Hbg-24 showed the best hits. Therefore, a peptide having the merged amino acids of both, namely VTVLAIHFGKEFTPEVQASW, was synthesized which was called Hbg-34 (Hb$\gamma1_{111\text{-}130}$). This peptide expressed a similar degree of synergistic activity with MPLA as Hbg-23 and Hbg24 (Fig. 5).

Because most of the C-terminal peptides (peptide No. 20 to No. 27) were bioactive, three longer overlapping peptides, designated as Hbg-30-105 (Hby86-115), Hbg-31-106 (Hby99-128), and Hbg-32-107 (Hb$\gamma$112-141 [L141Q]) were synthesized (Fig. 6). As compared to Hbg-23, the prolonged peptide Hbg-32-107 was found to display increased synergistic effects concerning MPLA-induced immunostimulation (Fig. 7).

Hbg-23 and Hbg-32-107 both showed synergistic effects in the absence as well as in the presence of human serum during MPLA-stimulation of MNC (Fig. 8). In addition to the synergistic effects in stimulation of MNC, Hbg-23 and Hbg-32-107 were also shown to synergize with MPLA in stimulating purified human monocytes (Fig. 9).

To confirm these synergistic effects of the extended C-terminal Hbg peptide, two additional preparations of Hbg-32-107, namely, Hbg-32-108, and Hbg-32-KB were produced and analysed. It was found that Hbg-32-108 and Hbg-32-KB expressed synergistic effects comparable to Hbg-32-107 (Fig. 10). A second batch of Hbg-23, however, showed no synergistic effect with MPLA during induction of IL-release in human MNC (data not shown).

These data indicate that Hbg peptides and in particular Hbg-32 synergize with MPLA during induction of IL-6 and IL-8 release in MNC. As shown in Fig. 11, this synergistic effect of Hb-32 (batch 113) was not only limited to IL-6 and IL-8 release, but was also found with respect to IL-1$\beta$ and TNF$\alpha$ release.

As MPLA is a partial structure of LPS, it was investigated whether Hbg-32 also displays synergism in MNC stimulation by complete LPS as compared to the MPLA-related effects of this peptide. As shown in Fig. 12, the immunostimulatory activities of both endotoxic compounds were found by to be synergically enhanced by Hbg-32.

Finally, the synergistic effects of Hbg-32 were compared with the synergistic effects of the complete Hb$\gamma$ chain, namely Hb-$\gamma$-133. In comparison to native Hb$\gamma$, Hbg-32-107 was found to express higher synergistic activity during stimulation of MNC (Fig. 13). These results show that the synergistic activity of Hbg-32 with MPLA exceeds the activity of the total native Hb$\gamma$ chain.

At this stage of the investigations it was recognized that due to an error in data transfer the C-terminal amino acid residue had been incorrectly selected in the synthesis of the Hgb-32-peptide. Instead of leucine ($L_{141}$), as present in the native Hbγ1-sequence, a glutamine (Q) residue had been introduced in Hbg-32. Consequently a Hbg-peptide with the correct native amino acid of the Hbγ-sequence was synthesized, namely Hbg-35 having the sequence TVLAIHFGKEFTPEVQASWQKMVTAVASAL. The synergistic effect of this peptide resembled the synergism mediated by Hbg-32 in the presence or absence of human serum during culture of the cells (Fig. 14A and 14B). It can be concluded that the C-terminal polar/neutral glutamine, as present in Hbg-32, can be replaced by the nonpolar/neutral leucine without impairing the synergistic effect with MPLA. In the following experiments, therefore, the Hbg-35 peptide instead of Hbg-32 peptide was used in order to use the native sequence.

The following experiments were done to characterize the active region of Hbg-35, which is responsible for the synergistic effect. Hbg-derived peptides, therefore, were synthesized in which the sequence of the Hbg-35 peptide was elongated or truncated to the N-terminal and C-terminal end of the Hbg-protein and/or shifted toward the N-terminal end of the Hbγ1-protein. The data, presented in Fig. 14 and 15 show that all synthesized Hbg-peptides are biologically active with respect to synergizing the inflammatory activity of MPLA *in vitro.* However, there is a trend, which indicates that a C-terminal truncated and/or N-terminal shifted peptide expresses a lower degree of synergistic activity in comparison to Hbg35 (Fig. 15 and 16), whereas an C-terminal elongation of Hbg-35 sequence up to the end of the native Hbγ1-protein somewhat enhances the synergistic effect (Fig. 16).

Next, an alanine-scan was carried out to determine the contribution of specific amino acids to the stability or function of Hbg-35. In this technique every individual amino-acid of a peptide is replaced by an alanine, as demonstrated for Hbg-35 in Fig. 17. From the 25 Hbg-peptides synthesized, three alanine replacements, as present in Hbg-51, Hbg-54, and Hbg-55 were found to affect the synergistic activity of Hbg-35 (Fig. 18). Whereas a Phe122/Ala-replacement, as present in Hbg-51, enhanced the synergistic activity of Hbg-35, a replacement of Glu125/Ala and Val126/Ala abolished the synergistic effects. All three replacements are located within the sequences of Hbg-23 and Hbg-24, which were found to have strong synergistic activity (Fig. 5). However, Hbg-25, in which also these amino acids are included, was inactive. This indicates that together with F122, E125, and V126, additional amino acids participate in the stability and/or function of Hbg-35.

All synergistically active Hbg-peptides have a dominant alpha-helical structure, namely TPEVQASWQKMVTAVA, which can be assumed to cross bilayered lipids such as MPLA and, thus, disaggregate these compounds. The secondary structures were predicted by Internet programs such as LOMETS and 3D-JIGSAW. In addition, a common part structure of these Hbg-peptides is VLAIHFGKEFTPEVQASW. These peptides were therefore synthesized and their synergistic activity with MPLA was determined. It was found that these peptides express, however, only a minute synergistic activity or no activity at all (Fig. 19). This indicates that these sequences are not sufficient for optimal synergistic effects, but additional N- and/or C-terminal amino acids are necessary.

### Example 3: Contribution of MPLA and Hbg-35 in the activation of T-lymphocytes with recall antigens

There is a close interaction between the innate immune system, as presented by e.g. monocytes and dendritic cells, and the adaptive immune system, as presented by T-lymphocytes and B-lymphocytes. Monocytes and dendritic cells present antigenic fragments to the cells of the adaptive immune system to initiate a specific immune reaction. In addition, they support the strength and polarization of the immune response by means of the release of cytokines. For the present investigations, the model of activation of T-memory lymphocytes in a cell preparation of human MNC with *Purified Protein Derivative of M. tuberculosis* (PPD, which is used for the skin tuberculin sensitivity test) and *Tetanus Toxoid* (TT, which is used as tetanus vaccine) was used. In this cell system only memory T-lymphocytes respond in a specific way to these antigens. This requires that the donor of the blood has had a primary contact with these antigens during an episode of bacterial infection or during vaccination.

After stimulation of human MNC with PPD and TT it was found that MPLA has a very effective adjuvant activity on the stimulation of T-memory cells with the recall antigens. As less as 10 ng/ml of MPLA are sufficient to exert a nearly optimal adjuvant effect (Fig. 20). In some experiments (e.g. Fig. 20A) a proliferation of T-lymphocytes with MPLA alone was observed. However, an adjuvant effect of MPLA was also observed in experiments where no T-lymphocyte response to MPLA alone was detected (e.g. Fig. 20B). The adjuvant effect of MPLA on PPD and TT induced T-lymphocyte proliferation can be enhanced by the addition of Hbg-35 (Fig. 21A and 21 B), indicating that a composition comprising MPLA and Hbg-35 is also synergistically active in the adaptive immune system.

### Example 4: Mechanism of synergism between synthetic Hbγ-derived peptides and MPLA

To investigate the molecular mechanism of the synergistic activity of the Hbg-32 peptide with MPLA, physical methods were applied. The aggregate structure of endotoxins is a determinant of its ability to induce cytokines in immune cells. This aggregate structure was characterized by performing synchrotron radiation X-ray small-angle scattering in the absence and presence of peptides. The structure of MPLA alone was found to be largely multi-lamellar (Fig. 22A), corresponding to an aggregation form with no or only low bioactivity. In the presence of the peptide, this aggregate was found to be converted into one with cubic symmetry (Fig. 22B), previously shown to represent the bioactive form of endotoxins (10).

The ability of Hb to incorporate into endotoxin aggregates was shown to represent an important property for synergistic actions. Förster (fluorescence) resonance energy transfer spectroscopy was applied to monitor the intercalation behaviour of the Hbg-32 peptide into MPLA aggregates. A considerable intercalation of the peptide into MPLA already at a [MPLA]:[Hbg-32] molar ratio of 10:1 was found, being much more pronounced at an equimolar concentration (Fig. 23).

The disaggregation of endotoxins due to the action of Hb is a further prerequisite for enhancement of bioactivity (Howe *et al.* (2008a); Brandenburg *et al.* (2003); Howe *et al.* (2007); Howe *et al.* (2008b)). Thus, the size distribution of the MPLA aggregates with and without peptide was determined by monitoring the particles by Laser-light scattering in a cell. The results show rather large MPLA particle sizes in the range of 500 to 2000 nm for MPLA without peptide (Fig. 24). Upon addition of the peptide, a drastic decrease of these sizes, having a wide distribution in the range from 80 to 300 nm was observed.

Since the size distribution data from Laser-light scattering are only valid by assuming spherical particles, the morphology of MPLA was studied by an independent method, the freeze-fracture electron microscopy. This allows maintaining the structures of the lipids by shock-freezing the samples. The results for MPLA alone are indicative of very densely and lamellar packed aggregates with large sizes in the micrometer range, in accordance with the above results (Fig. 25A). In the presence of the peptide, there is a strong reduction of the particles sizes, accompanied by a change in morphology, leading to the dissolution of the packing arrangement into spherical-like aggregates in the size range from 20 to some hundred nm (Fig. 25). These results are in accordance with the data obtained by light scattering.

Atomic force microscopy (AFM) allows a direct look onto the morphology of lipid aggregates on a molecular scale under near physiological conditions. This allows obtaining depth profiles of MPLA dispersions down to a resolution limit of lower than 1 nm. The data show for pure MPLA, spread on a mica plate, the existence of densely packed multilamellar aggregates with some hundred nm extension in z-direction. The peptide, spread cautiously onto the lipid dispersion, interacts by only passive diffusion with the lipid aggregates. This administration technique has the advantage that mixing processes between the two compounds, which normally occur on a time-scale of minutes, are slowed down to some hours, thus allowing a detailed analysis of the interaction process.

As can be seen for the MPLA assembly in the presence of Hbg-32 (Fig. 26A and 26B; recorded after one hour) the tightly packed layers disaggregate dramatically by breaking down the multi-lamellae into bilayered structures. The mode of action of the peptide is its transmembraneous incorporation into the MPLA bilayers, thus reducing the sizes of the lamellar units considerably.

These data are in accordance with the electron microscopic data with freeze-fracture (Fig. 25A and 25B) and the SAXS experiments (Fig. 22), giving now a clear picture of the observation that the peptides cause an increase in biological activity: The considerably smaller MPLA particles can much better bind to serum- and membrane proteins such as CD14 and LBP, which are responsible for the inflammation reaction induced by MPLA.

With isothermal titration calorimetry (ITC) the thermodynamics of the interaction of two different molecular species can be deduced. In Fig. 27, the power of the interaction and the enthalpy change measured versus the [Hbγ35]:[MPLA] molar ratio shows that (i) only endothermic reactions (peaks are directed upwards) occur and (ii) that there is only a slight decrease of the enthalpy change versus time and molar ratio. This observation was also found to hold true for even higher molar ratios, i.e., the interaction of the peptide with the MPLA aggregates shows a non-saturable behaviour. This is indicative of a catalytic rather than a typical binding reaction (which would form a complex) and is in contrast to the action of antimicrobial peptides with endotoxins, for which exothermic reactions take place leading to saturation of binding.

Finally, to obtain information on the secondary structures of the peptide, Fourier-transform infrared spectroscopy (FTIR) was applied. In Fig. 28, the spectra of the pure Hbγ35 (top) and that of a Hbγ35:MPLA mixture (bottom, 1:1 molar) are plotted. As can be seen, both figures show a main absorbance maximum around 1659 $cm^{-1}$ and a second peak around 1628 $cm^{-1}$, which are indicative of a main α-helical structure and a minor β-sheet component. Importantly, the addition of the lipid does not influence the peptide's secondary structure. This is again in accordance with the data presented above, since a direct binding of the peptide rather than a catalytic reaction would lead to a change of its secondary structure.

**Example 5: Peptides derived from Hbα- Hbβ and Hbγ2 chain**

Peptides derived from Hbα- Hbβ- and Hbγ2 chain, having the homologous sequence of Hbg-35, were additionally investigated. These three peptides have the following sequence:

Hba-66-153: $Hb\alpha_{[107-136]}$ VTLAAHLPAEFTPAVHASLDKFLASVSTVL (SEQ ID NO:21)

Hbb-67-154: $Hb\beta_{[112-141]}$ CVLAHHFGKEFTPPVQAAYQKVVAGVANAL (SEQ ID NO:20)
Hbg2-70-157: $Hb\gamma 2_{[112-141]}$ TVLAIHFGKEFTPEVQASWQKMVTGVASAL (SEQ ID NO:19).

Also these peptides have a dominant alpha-helical structure, which can be assumed to cross bilayered lipids such as MPLA und thus disaggregate these compounds. As is shown in Figure 29, both Hbb-67-154 and Hbg2-70-157 were found to be synergistically active in a similar range as Hbg-35-146m whereas Hba-66-153 showed only a minimal synergistic activity.

**Example 6: Synergistic activity of Hb peptides with bacterial lipoproteins**

It was additionally investigated whether bacterial lipoproteins, such as Pam3C-SK4 or FSL-1, synergize with Hbg-35 in the induction of cytokines in human MNC, similar to LPS and MPLA. The results shown in Figure 30 indicate that there is indeed a synergistic activity of synthetic lipopeptides with Hbg-35.

**Reference List**

Akashi, S., Shimazu, R., Ogata, H., Nagai, Y., Takeda, K., Kimoto, M., Miyake, K. (2000) Cutting edge: cell surface expression and lipopolysaccharide signaling via the toll-like receptor 4-MD-2 complex on mouse peritoneal macrophages. J.Immunol. 164, 3471-3475

Akira, S. (2003) Mammalian Toll-like receptors. Curr.Opin.Immunol. 15, 5-11

Bahl, N., Du, R., Winarsih, I., Ho, B., Tucker-Kellogg, L., Tidor, B., Ding, J. L. (2011) Delineation of Lipopolysaccharide (LPS)-binding Sites on Hemoglobin: From in silico predictions to biophysical characterization. J.Biol.Chem. 286, 37793-37803

Brandenburg, K., Garidel, P., Andra, J., Jurgens, G., Muller, M., Blume, A., Koch, M. H., Levin, J. (2003) Cross-linked hemoglobin converts endotoxically inactive pentaacyl endotoxins into a physiologically active conformation. J.Biol.Chem. 278, 47660-47669

Buwitt-Beckmann, U., Heine, H., Wiesmüller, K.-H., Jung, G., Brock, R., and Ulmer, A.J. (2005): Lipopeptide structure determines TLR2 dependent cell activation level. FEBS J. 272, 6354-6364

da Silva, C. J., Soldau, K., Christen, U., Tobias, P. S., Ulevitch, R. J. (2001) Lipopolysaccharide is in close proximity to each of the proteins in its membrane receptor complex. transfer from CD14 to TLR4 and MD-2. J.Biol.Chem. 276, 21129-21135

Fox, C. B., Friede, M., Reed, S. G., Ireton, G. C. (2010) Synthetic and natural TLR4 agonists as safe and effective vaccine adjuvants. Subcell.Biochem. 53, 303-321

Frier, J. A., Perutz, M. F. (1977) Structure of human foetal deoxyhaemoglobin. J.Mol.Biol. 112, 97-112

Galanos, C., Luderitz, O., Rietschel, E. T., Westphal, O., Brade, H., Brade, L., Freudenberg, M., Schade, U., Imoto, M., Yoshimura, H., . (1985) Synthetic and natural Escherichia coli free lipid A express identical endotoxic activities. Eur.J.Biochem. 148, 1-5

Galanos, C., Freudenberg, M. A., Katschinski T., Salomao, R., Mossmann, H., Kumazawa, Y. (1992) Tumor necrosis factor and host response to endotoxin. In Bacterial endotoxic lipopolysaccharides. II. Immunopharmacology and pathophysiology (Ryan, J. L. and Morrison, D. C., eds) pp. 75-104, CRC Press, Boca Raton.

Gorczynski, R. M., Alexander, C., Bessler, W., Brandenburg, K., Fournier, K., Hoffmann, P., Mach, J. P., Mueller, S., Rietschel, E. T., Terzioglu, E., Ulmer, A. J., Waelli, T., Zahringer, U., Khatri, I. (2006) Role of MIF and glutathione, in association with fetal ovine globin chain (Hbgamma) and LPS, in induction of TNFalpha from cells of young and aged mice, and PBL from healthy human populations. Immunol.Lett. 105, 140-149

Haziot, A., Chen, S., Ferrero, E., Low, M. G., Silber, R., Goyert, S. M. (1988) The monocyte differentiation antigen, CD14, is anchored to the cell membrane by a phosphatidylinositol linkage. J.Immunol. 141, 547-552

Howe, J., Hammer, M., Alexander, C., Rossle, M., Fournier, K., Mach, J. P., Waelli, T., Gorczynski, R. M., Ulmer, A. J., Zahringer, U., Rietschel, E. T., Brandenburg, K. (2007) Biophysical characterization of the interaction of endotoxins with hemoglobins. Med.Chem. 3, 13-20

Howe, J., Richter, W., Hawkins, L., Rossle, M., Alexander, C., Fournier, K., Mach, J. P., Waelli, T., Gorczynski, R. M., Ulmer, A. J., Brade, H., Zamyatina, A., Kosma, P., Rietschel, E. T., Brandenburg, K. (2008a) Hemoglobin enhances the biological activity of synthetic and natural bacterial (endotoxic) virulence factors: a general principle. Med.Chem. 4, 520-525

Howe, J., Garidel, P., Roessle, M., Richter, W., Alexander, C., Fournier, K., Mach, J. P., Waelli, T., Gorczynski, R. M., Ulmer, A. J., Zahringer, U., Hartmann, A., Rietschel, E. T., Brandenburg, K. (2008b) Structural investigations into the interaction of hemoglobin and part structures with bacterial endotoxins. Innate.Immun. 14, 39-49

Imoto, M., Yoshimura, H., Shimamoto, S., Sakaguchi, N., Kusumoto, S., Shiba, T. (1987) Total synthesis of Escherichia coli lipid A, the endotoxically active principle of cellsurface lipopolysaccharide. Bull. Chem. Soc.Jpn. 60, 2205-2214

Kaconis, Y., Kowalski, I., Howe, J., Brauser, A., Richter, W., Razquin-Olazaran, I., Inigo-Pestana, M., Garidel, P., Rossle, M., Martinez de, T. G., Gutsmann, T., Brandenburg, K. (2011) Biophysical mechanisms of endotoxin neutralization by cationic amphiphilic peptides. Biophys.J. 100, 2652-2661

Karlsson, S., Nienhuis, A. W. (1985) Developmental regulation of human globin genes. Annu.Rev.Biochem. 54, 1071-1108

Loppnow, H. (1994) LPS, reclL1 and smooth muscle cell-IL1 activate vascular cells by specific mechanisms. Prog.Clin.Biol.Res. 388, 309-321

Mak, P., Siwek, M., Pohl, J., Dubin, A. (2007) Menstrual hemocidin HbB115-146 is an acidophilic antibacterial peptide potentiating the activity of human defensins, cathelicidin and lysozyme. Am.J.Reprod.Immunol. 57, 81-91

Mattern, T., Thanhauser, A., Reiling, N., Toellner, K. M., Duchrow, M., Kusumoto, S., Rietschel, E. T., Ernst, M., Brade, H., Flad, H. D., . (1994) Endotoxin and lipid A stimulate proliferation of human T cells in the presence of autologous monocytes. J.Immunol. 153, 2996-3004

Parish, C. A., Jiang, H., Tokiwa, Y., Berova, N., Nakanishi, K., McCabe, D., Zuckerman, W., Xia, M. M., Gabay, J. E. (2001) Broad-spectrum antimicrobial activity of hemoglobin. Bioorg.Med.Chem. 9, 377-382

Perutz, M. F. (1979) Regulation of oxygen affinity of hemoglobin: influence of structure of the globin on the heme iron. Annu.Rev.Biochem. 48, 327-386

Poltorak, A., He, X., Smirnova, I., Liu, M. Y., Van, H. C., Du, X., Birdwell, D., Alejos, E., Silva, M., Galanos, C., Freudenberg, M., Ricciardi-Castagnoli, P., Layton, B., Beutler, B. (1998) Defective LPS signaling in C3H/HeJ and C57BL/10ScCr mice: mutations in Tlr4 gene. Science 282, 2085-2088

Pugin, J., Schürer-Maly, C.-C., Leturcq, D., Moriarty, A., Ulevitch, R. J., Tobias, P. S. (1993) Lipopolysaccharide activation of human endothelial and epithelial cells is mediated by lipopolysaccharide-binding protein and soluble CD14. Proc.Natl.Acad.Sci. USA 90, 2744-2748

Rietschel, E. T., Brade, L., Brandenburg, K., Flad, H. D., de Jong-Leuveninck, J., Kawahara, K., Lindner, B., Loppnow, H., Luderitz, T., Schade, U., . (1987) Chemical structure and biologic activity of bacterial and synthetic lipid A. Rev.Infect.Dis. 9 Suppl 5, S527-S536

Rietschel, E. T., Kirikae, T., Schade, F. U., Mamat, U., Schmidt, G., Loppnow, H., Ulmer, A. J., Zahringer, U., Seydel, U., Di, P. F., . (1994) Bacterial endotoxin: molecular relationships of structure to activity and function. FASEB J. 8, 217-225

Salunke, DB., Shukla, N.M., Yoo, E., Crall, B.M., Balakrishna, R., Malladi, S.S., David, S.A. (2012). Structure-activity relationships in human Toll-like receptor 2-specific monoacyl lipopeptides. J Med Chem. 55, 3353-3363

Seydel, U., Oikawa, M., Fukase, K., Kusumoto, S., Brandenburg, K. (2000) Intrinsic conformation of lipid A is responsible for agonistic and antagonistic activity. Eur.J.Biochem. 267, 3032-3039

Shimazu, R., Akashi, S., Ogata, H., Nagai, Y., Fukudome, K., Miyake, K., Kimoto, M. (1999) MD-2, a molecule that confers lipopolysaccharide responsiveness on Toll-like receptor. J.Exp.Med. 189, 1777-1782

Supajatura, V., Ushio, H., Nakao, A., Okumura, K., Ra, C., Ogawa, H. (2001) Protective roles of mast cells against enterobacterial infection are mediated by Toll-like receptor. J.Immunol. 167, 2250-2256

**Table 1:** Conservative variants of Hbg-35. Conservative substitutions of the amino acids of Hbg-35 are listed that result in variant sequences that are expected to have identical or at least similar synergistic activity as Hbg-35.

| **Nr. in *'Hbg-35'*** | **AA. 'Hbg-35'** | ***Secondary Structure of h-Hb-γ1 in-h-HbF*** | ***SidechainType*** | ***Homologous AA. Residues*** |
|---|---|---|---|---|
| **1** | **Thr (T)** | **α-Helix (I)** | **Polar** | **Ser (S)** |
| **2** | **Val (V)** | **α-Helix (I)** | **Hydrophobic** | **Ala (A), Leu (L), Met (M), Ile (I)** |
| **3** | **Leu (L)** | **α-Helix (I)** | **Hydrophobic** | **Ala (A), Ile (I), Met (M), Val (V)** |
| **4** | **Ala (A)** | **α-Helix (I)** | **Hydrophobic** | **Leu (L), Ile (I), Met (M), Val (V)** |
| **5** | **Ile (I)** | **α-Helix (I)** | **Hydrophobic** | **Ala (A), Leu (L), Met (M), Val (V)** |

(continued)

| Nr. in *'Hbg-35'* | AA. 'Hbg-35' | *Secondary Structure of h-Hb-γ1 in-h-HbF* | *SidechainType* | *Homologous AA. Residues* |
|---|---|---|---|---|
| **6** | **His (H)** | **α-Helix (I)** | **Basic** | **Arg (R), Lys (L)** |
| **7** | **Phe (F)** | **α-Helix (I)** | **Hydrophobic** | **Trp (W) / Leu (L), Ile (I), Val (V)** |
| **8** | **Gly (G)** | ***Loop / Turn*** | ***'Hydrophobic'*** | **Ala (A)** |
| **9** | **Lys (K)** | ***Loop/Turn*** | **Basic** | **Arg (R), His (H)** |
| **10** | **Glu (E)** | ***Loop/Turn*** | **Acidic** | **Asp (D)** |
| **11** | **Phe (F)** | ***Loop/Turn*** | **Hydrophobic** | **Trp (W) / Leu (L), Ile (I), Val (V)** |
| **12** | **Thr (T)** | ***Loop/Turn*** | **Polar** | **Ser (S)** |
| **13** | **Pro (P)** | ***Loop/Turn*** | ***Cyclic*** | **-** |
| **14** | **Glu (E)** | **α-Helix (II)** | **Acidic** | **Asp (D)** |
| **15** | **Val (V)** | **α-Helix (II)** | **Hydrophobic** | **Ala (A), Leu (L), Met (M), Ile (I)** |
| **16** | **Gln (Q)** | **α-Helix (II)** | **Polar** | **Asn (N)** |
| **17** | **Ala (A)** | **α-Helix (II)** | **Hydrophobic** | **Leu (L), Ile (I), Met (M), Val (V)** |
| **18** | **Ser (S)** | **α-Helix (II)** | **Polar** | **Thr (T)** |
| **19** | **Trp (W)** | **α-Helix (II)** | **Hydrophobic** | **Phe (F) / Leu (L), Ile (I), Val (V)** |
| **20** | **Gln (Q)** | **α-Helix (II)** | **Polar** | **Asn (N)** |
| **21** | **Lys (K)** | **α-Helix (II)** | **Basic** | **Arg (R), His (H)** |
| **22** | **Met (M)** | **α-Helix (II)** | **Hydrophobic** | **Ala (A), Leu (L), Ile (I), Val (V)** |
| **23** | **Val (V)** | **α-Helix (II)** | **Hydrophobic** | **Ala (A), Leu (L), Met (M), Ile (I)** |
| **24** | **Thr (T)** | **α-Helix (II)** | **Polar** | **Ser (S)** |
| **25** | **Ala (A)** | **α-Helix (II)** | **Hydrophobic** | **Leu (L), Ile (I), Met (M), Val (V)** |
| **26** | **Val (V)** | **α-Helix (II)** | **Hydrophobic** | **Ala (A), Leu (L), Met (M), Ile (I)** |
| **27** | **Ala (A)** | **α-Helix (II)** | **Hydrophobic** | **Leu (L), Ile (I), Met (M), Val (V)** |
| **28** | **Ser (S)** | **α-Helix (II)** | **Polar** | **Thr (T)** |
| **29** | **Ala (A)** | **α-Helix (II)** | **Hydrophobic** | **Leu (L), Ile (I), Met (M), Val (V)** |
| **30** | **Leu (L)** | **α-Helix (II)** | **Hydrophobic** | **Ala (A), Ile (I), Met (M), Val (V)** |

## Claims

1. A pharmaceutical composition comprising at least one Toll-like receptor (TLR)-activating amphiphilic lipid and at least one hemoglobin-derived peptide and, optionally, a pharmaceutically acceptable carrier, wherein the hemoglobin-derived peptide consists of

   (a) an amino acid sequence consisting of the sequence of formula I:

   A1 -A2-A3-A4-A5-A6-A7-A8-A9-A10-A11 -A12-A13-A14-A15-A16-A17-A18-A19 (formula I), wherein

   A1 is selected from the group consisting of threonine, cysteine and serine;
   A2 is selected from the group consisting of valine, alanine, leucine, methionine and isoleucine;
   A3 is selected from the group consisting of leucine, alanine, isoleucine, methionine and valine;
   A4 is selected from the group consisting of alanine, leucine, isoleucine, methionine and valine;
   A5 is selected from the group consisting of isoleucine, histidine, alanine, leucine, methionine and valine;
   A6 is selected from the group consisting of histidine, arginine and leucine;
   A7 is selected from the group consisting of phenylalanine, tryptophan, leucine, isoleucine and valine;
   A8 is selected from the group consisting of glycine and alanine;
   A9 is selected from the group consisting of lysine, arginine and histidine;
   A10 is selected from the group consisting of glutamic acid and aspartic acid;
   A11 is selected from the group consisting of phenylalanine, tryptophan, leucine, isoleucine and valine;
   A12 is selected from the group consisting of threonine and serine;
   A13 is selected from the group consisting of proline and glycine;
   A14 is selected from the group consisting of glutamic acid, proline and aspartic acid;
   A15 is selected from the group consisting of valine, alanine, leucine, methionine and isoleucine;
   A16 is selected from the group consisting of glutamine and asparagine;
   A17 is selected from the group consisting of alanine, leucine, isoleucine, methionine and valine;
   A18 is selected from the group consisting of serine, alanine and threonine; and
   A19 is selected from the group consisting of tryptophane, tyrosine, phenylalanine, leucine, isoleucine and valine;
   and

   (b) an amino acid sequence located N-terminally of the amino acid sequence of (a) and/or an amino acid sequence located C-terminally of the amino acid sequence of (a), wherein

   (b-i) the amino acid sequence located N-terminally of the amino acid sequence of (a) is selected from the group consisting of:

   N1: V;
   N2: LV;
   N3: VLV;
   N4: NVLV;
   N5: GNVLV;
   N6: LGNVLV;
   N7: LLGNVLV;

N8: KLLGNVLV;
N9: FKLLGNVLV;
N10: NFKLLGNVLV;
N11: ENFKLLGNVLV;
N12: PENFKLLGNVLV;
N13: DPENFKLLGNVLV;
N14: VDPENFKLLGNVLV;
N15: HVDPENFKLLGNVLV; and
N16: LHVDPENFKLLGNVLV;

and wherein
(b-ii) the amino acid sequence located C-terminally of the amino acid sequence of (a) is selected from the group consisting of:

(1) an amino acid sequence consisting of the sequence of formula II:

C1-C2-C3-C4-C5 (formula II),

wherein

C1: is selected from the group consisting of glutamine, asparagine and tyrosine;
C2: is selected from the group consisting of lysine, arginine and histidine;
C3: is selected from the group consisting of methionine, valine, alanine, leucine and isoleucine;
C4: is selected from the group consisting of valine, alanine, leucine, methionine and isoleucine; and
C5: is selected from the group consisting of threonine, alanine, serine and lysine;

(2) an amino acid sequence consisting of the sequence of formula III:

C1-C2-C3-C4-C5-C6-C7-C8-C9-C10-C11-C12 (formula III),

wherein

C1 to C5 is defined as in (b-ii)(1);
C6: is selected from the group consisting of alanine, glycine, leucine, isoleucine, methionine and valine;
C7: is selected from the group consisting of valine, alanine, leucine, methionine and isoleucine;
C8: is selected from the group consisting of alanine, leucine, isoleucine, methionine and valine;
C9: is selected from the group consisting of serine, asparagine and threonine;
C10: is selected from the group consisting of alanine, leucine, isoleucine, methionine and valine;
C11: is selected from the group consisting of leucine, glutamine, alanine, isoleucine, methionine and valine; and
C12: is a peptide consisting of the amino acid sequence SSRYH or is absent; and

(3) an amino acid sequence consisting of the sequence of formula IV:

C1-C2-C3-C4-C5-C6-X (formula IV),

wherein

C1 to C5 is defined as in (b-ii)(1);
C6: is selected from the group consisting of alanine, glycine, leucine, isoleucine, methionine and valine;
X: is a peptide consisting of the five amino acid residues alanine, valine, serine, alanine and leucine in any order

or a peptidomometic thereof.

**2.** The pharmaceutical composition according to claim 1, wherein the amino acid sequence of

(a) is selected from the group consisting of TVLAIHFGKEFTPEVQASW (SEQ ID NO:1) and CVLAHHFGKEFTPPVQAAY (SEQ ID NO:2).

**3.** The pharmaceutical composition according to claim 1 or 2, wherein the amino acid sequence of (b-i) is selected from the group consisting of

(i) V (SEQ ID NO:3),
(ii) LGNVLV (SEQ ID NO:4) and
(iii) LHVDPENFKLLGNVLV (SEQ ID NO:5) and/or

the amino acid sequence of (b-ii) is selected from the group consisting of

(i) QKMVT (SEQ ID NO:6),
(ii) QKVVA (SEQ ID NO:7),
(iii) QKMVTAVASAL (SEQ ID NO:8),
(iv) QKMVTAVASAQ (SEQ ID NO:9),
(v) QKMVTGVASAL (SEQ ID NO:10),
(vi) QKVVAGVANAL (SEQ ID NO: 11) and
(vii) QKMVTAVASALSSRYH (SEQ ID NO:12).

**4.** The pharmaceutical composition according to any one of claims 1 to 3, wherein the hemoglobin-derived peptide consists of an amino acid sequence selected from the group consisting of:

(i) TVLAIHFGKEFTPEVQASWQKMVTAVASAL (SEQ ID NO:13),
(ii) VTVLAIHFGKEFTPEVQASW (SEQ ID NO:14),
(iii) VTVLAIHFGKEFTPEVQASWQKMVTAVASALSSRYH (SEQ ID NO:15),
(iv) TVLAIHFGKEFTPEVQASWQKMVTAVASAQ (SEQ ID NO:16),
(v) LGNVLVTVLAIHFGKEFTPEVQASWQKMVT (SEQ ID NO:17),
(vi) LHVDPENFKLLGNVLVTVLAIHFGKEFTPEVQASW (SEQ ID NO:18),
(vii) TVLAIHFGKEFTPEVQASWQKMVTGVASAL (SEQ ID NO:19), and
(viii) CVLAHHFGKEFTPPVQAAYQKVVAGVANAL (SEQ ID NO:20).

**5.** The pharmaceutical composition according to any one of claims 1 to 4, wherein the TLR-activating amphiphilic lipid is selected from the group consisting of an endotoxin, or an endotoxically active portion thereof, and a bacterial lipopeptide.

**6.** The pharmaceutical composition according to claim 5, wherein the endotoxin is bacterial or synthetic S- or R-form lipopolysaccharide (LPS).

**7.** The pharmaceutical composition according to claim 5 or 6, wherein the endotoxically active portion of endotoxin is the LPS-derived polysaccharide-free native or synthetic lipid A component.

**8.** The pharmaceutical composition according to claim 5 or 6, wherein the endotoxin is natural or synthetic penta- and/or hexaacyl-lipid A.

**9.** The pharmaceutical composition according to claim 5 or 6, wherein the endotoxin is natural or synthetic penta- and/or hexaacyl lipid A monophosphate.

**10.** The pharmaceutical composition according to claim 5 or 6, wherein the bacterial lipopeptide is a mono-, di- or triacylated lipopeptide having a S-(2,3-dihydroxypropyl)-cysteine backbone.

**11.** The pharmaceutical composition according to any one of claims 1 to 10, for use in preventing and/or treating tumours, preventing and/or treating infections, preventing and/or treating allergies, preventing and/or treating age-related immune imbalances, stimulating the innate and adaptive immune system and/or alleviating the adverse side effects of irradiation.

**12.** A hemoglobin-derived peptide consisting of:

(a) the amino acid sequence TVLAIHFGKEFTPEVQASW (SEQ ID NO:1); and
(b) an amino acid sequence located N-terminally of the amino acid sequence of (a) and/or an amino acid sequence located C-terminally of the amino acid sequence of (a), wherein

(b-i) the amino acid sequence located N-terminally of the amino acid sequence of (a) is selected from the group consisting of:

(i) V (SEQ ID NO:3),
(ii) LGNVLV (SEQ ID NO:4) and
(iii) LHVDPENFKLLGNVLV (SEQ ID NO:5) and/or

and
(b-ii) the amino acid sequence located C-terminally of the amino acid sequence of (a) is selected from the group consisting of:

(i) QKMVT (SEQ ID NO:6),
(ii) QKMVTAVASAL (SEQ ID NO:8),
(iii) QKMVTAVASAQ (SEQ ID NO:9),
(iv) QKMVTAVASALSSRYH (SEQ ID NO:12).

**13.** The hemoglobin-derived peptide according to claim 12, wherein the hemoglobin-derived peptide consists of an amino acid sequence selected from the group consisting of:

(i) TVLAIHFGKEFTPEVQASWQKMVTAVASAL (SEQ ID NO:13),
(ii) VTVLAIHFGKEFTPEVQASW (SEQ ID NO:14),
(iii) VTVLAIHFGKEFTPEVQASWQKMVTAVASALSSRYH (SEQ ID NO:15),
(iv) TVLAIHFGKEFTPEVQASWQKMVTAVASAQ (SEQ ID NO:16),
(v) LGNVLVTVLAIHFGKEFTPEVQASWQKMVT (SEQ ID NO:17) and
(vi) LHVDPENFKLLGNVLVTVLAIHFGKEFTPEVQASW (SEQ ID NO:18).

**14.** The hemoglobin-derived peptide according to claim 12 or 13, for use in treating a bacterial infection.

# Fig. 1

## Sequence of hemoglobin subunit gamma-1

| | 1 | 11 | 21 | 31 | |
|---|---|---|---|---|---|
| -1 | MGHFTEEDKAT | ITSLWGKVNV | EDAGGETLGR | LLVVYPWTQR | 50 |
| 41 | FFDSFGNLSS | ASAIMGNPKV | KAHGKKVLTS | LGDAIKHLDD | 60 |
| 61 | LKGTFAQLSE | LHCDKLHVDP | ENFKLLGNVL | VTVLAIHFGK | 120 |
| 121 | EFTPEVQASW | QKMVTAVASA | LSSRYH | | |

## Sequence of Hbg-K5-peptides derived from hemoglobin subunit gamma-1

| Peptide No | Amino acid Sequence | Amino acids No. |
|---|---|---|
| Hbg-1-K5 | KKKKK-MGHFTEEDKATITSLW | Hbγ1 (-1-15) |
| Hbg-2-K5 | KKKKK-EDKATITSLWGKVNV | Hbγ1 (06-20) |
| Hbg-3-K5 | KKKKK-ITSLWGKVNVEDAGG | Hbγ1 (11-25) |
| Hbg-4-K5 | KKKKK-GKVNVEDAGGETLGR | Hbγ1 (16-30) |
| Hbg-5-K5 | KKKKK-EDAGGETLGRLLVVY | Hbγ1 (21-35) |
| Hbg-6-K5 | KKKKK-ETLGRLLVVYPWTQR | Hbγ1 (26-40) |
| Hbg-7-K5 | KKKKK-LLVVYPWTQRFFDSF | Hbγ1 (31-45) |
| Hbg-8-K5 | KKKKK-PWTQRFFDSFGNLSS | Hbγ1 (36-50) |
| Hbg-9-K5 | KKKKK-FFDSFGNLSSASAIM | Hbγ1 (41-55) |
| Hbg-10-K5 | KKKKK-GNLSSASAIMGNPKV | Hbγ1 (46-60) |
| Hbg-11-K5 | KKKKK-ASAIMGNPKVKAHGK | Hbγ1 (51-65) |
| Hbg-12-K5 | KKKKK-GNPKVKAHGKKVLTS | Hbγ1 (56-70) |
| Hbg-13-K5 | KKKKK-KAHGKKVLTSLGDAI | Hbγ1 (61-75) |
| Hbg-14-K5 | KKKKK-KVLTSLGDAIKHLDD | Hbγ1 (66-80) |
| Hbg-15-K5 | KKKKK-LGDAIKHLDDLKGTF | Hbγ1 (71-85) |
| Hbg-16-K5 | KKKKK-KHLDDLKGTFAQLSE | Hbγ1 (76-90) |
| Hbg-17-K5 | KKKKK-LKGTFAQLSELHCDK | Hbγ1 (81-95) |
| Hbg-18-K5 | KKKKK-AQLSELHCDKLHVDP | Hbγ1 (86-100) |
| Hbg-19-K5 | KKKKK-LHCDKLHVDPENFKL | Hbγ1 (91-105) |
| Hbg-20-K5 | KKKKK-LHVDPENFKLLGNVL | Hbγ1 (96-110) |
| Hbg-21-K5 | KKKKK-ENFKLLGNVLVTVLA | Hbγ1 (101-115) |
| Hbg-22-K5 | KKKKK-LGNVLVTVLAIHFGK | Hbγ1 (106-120) |
| Hbg-23-K5 | KKKKK-VTVLAIHFGKEFTPE | Hbγ1 (111-125) |
| Hbg-24-K5 | KKKKK-IHFGKEFTPEVQASW | Hbγ1 (116-130) |
| Hbg-25-K5 | KKKKK-EFTPEVQASWQKMVT | Hbγ1 (121-135) |
| Hbg-26-K5 | KKKKK-VQASWQKMVTAVASA | Hbγ1 (126-140) |
| Hbg-27-K5 | KKKKK-QKMVTAVASALSSRYH | Hbγ1 (131-146) |

(underlined are the amino acids overlapping with the precedent peptide)

Fig. 2

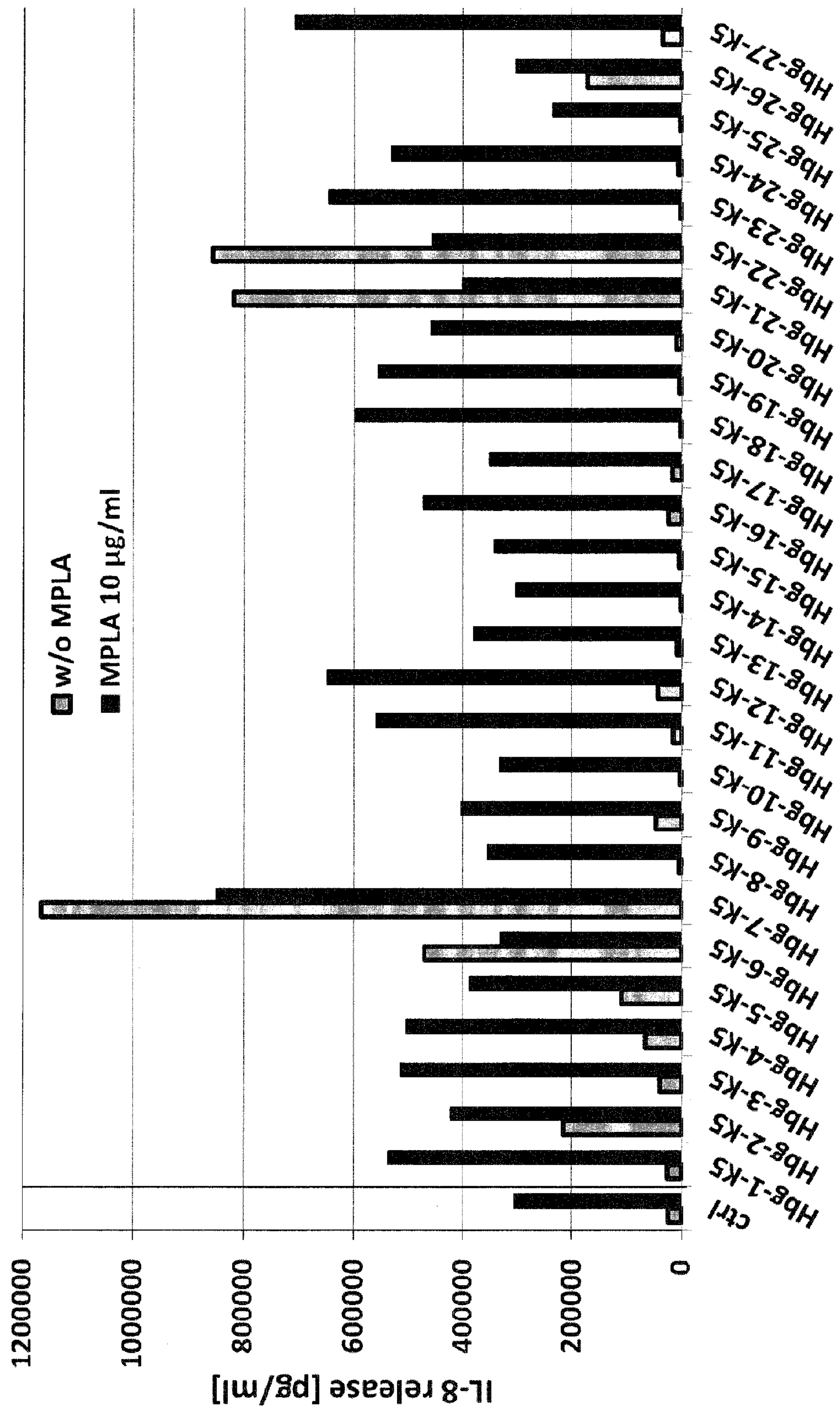
IL-8 release [pg/ml]
1200000
1000000
800000
600000
400000
200000
0
w/o MPLA
MPLA 10 µg/ml
ctrl
Hbg-1-KS
Hbg-2-KS
Hbg-3-KS
Hbg-4-KS
Hbg-5-KS
Hbg-6-KS
Hbg-7-KS
Hbg-8-KS
Hbg-9-KS
Hbg-10-KS
Hbg-11-KS
Hbg-12-KS
Hbg-13-KS
Hbg-14-KS
Hbg-15-KS
Hbg-16-KS
Hbg-17-KS
Hbg-18-KS
Hbg-19-KS
Hbg-20-KS
Hbg-21-KS
Hbg-22-KS
Hbg-23-KS
Hbg-24-KS
Hbg-25-KS
Hbg-26-KS
Hbg-27-KS

## Fig. 3
## Sequence of hemoglobin subunit gamma-1

```
      1          11         21         31
 -1 MGHFTEEDKAT ITSLWGKVNV EDAGGETLGR LLVVYPWTQR  40
 41  FFDSFGNLSS ASAIMGNPKV KAHGKKVLTS LGDAIKHLDD  60
 61  LKGTFAQLSE LHCDKLHVDP ENFKLLGNVL VTVLAIHFGK  120
121  EFTPEVQASW QKMVTAVASA LSSRYH
```

### Sequence of peptides derived from hemoglobin subunit gamma-1

| Peptide No | Amino acid Sequence | Amino acids No. |
|---|---|---|
| Hbg-1 | MGHFTEEDKATITSLW | Hbγ1 (-1-15) |
| Hbg-2 | EDKATITSLWGKVNV | Hbγ1 (06-20) |
| Hbg-3 | ITSLWGKVNVEDAGG | Hbγ1 (11-25) |
| Hbg-4 | GKVNVEDAGGETLGR | Hbγ1 (16-30) |
| Hbg-5 | EDAGGETLGRLLVVY | Hbγ1 (21-35) |
| Hbg-6 | ETLGRLLVVYPWTQR | Hbγ1 (26-40) |
| Hbg-7 | LLVVYPWTQRFFDSF | Hbγ1 (31-45) |
| Hbg-8 | PWTQRFFDSFGNLSS | Hbγ1 (36-50) |
| Hbg-9 | FFDSFGNLSSASAIM | Hbγ1 (41-55) |
| Hbg-10 | GNLSSASAIMGNPKV | Hbγ1 (46-60) |
| Hbg-11 | ASAIMGNPKVKAHGK | Hbγ1 (51-65) |
| Hbg-12 | GNPKVKAHGKKVLTS | Hbγ1 (56-70) |
| Hbg-13 | KAHGKKVLTSLGDAI | Hbγ1 (61-75) |
| Hbg-14 | KVLTSLGDAIKHLDD | Hbγ1 (66-80) |
| Hbg-15 | LGDAIKHLDDLKGTF | Hbγ1 (71-85) |
| Hbg-16 | KHLDDLKGTFAQLSE | Hbγ1 (76-90) |
| Hbg-17 | LKGTFAQLSELHCDK | Hbγ1 (81-95) |
| Hbg-18 | AQLSELHCDKLHVDP | Hbγ1 (86-100) |
| Hbg-19 | LHCDKLHVDPENFKL | Hbγ1 (91-105) |
| Hbg-20 | LHVDPENFKLLGNVL | Hbγ1 (96-110) |
| Hbg-21 | ENFKLLGNVLVTVLA | Hbγ1 (101-115) |
| Hbg-22 | LGNVLVTVLAIHFGK | Hbγ1 (106-120) |
| Hbg-23 | VTVLAIHFGKEFTPE | Hbγ1 (111-125) |
| Hbg-24 | IHFGKEFTPEVQASW | Hbγ1 (116-130) |
| Hbg-25 | EFTPEVQASWQKMVT | Hbγ1 (121-135) |
| Hbg-26 | VQASWQKMVTAVASA | Hbγ1 (126-140) |
| Hbg-27 | QKMVTAVASALSSRYH | Hbγ1 (131-146) |

(underlined are the amino acids overlapping with the precedent peptide)

Fig. 4

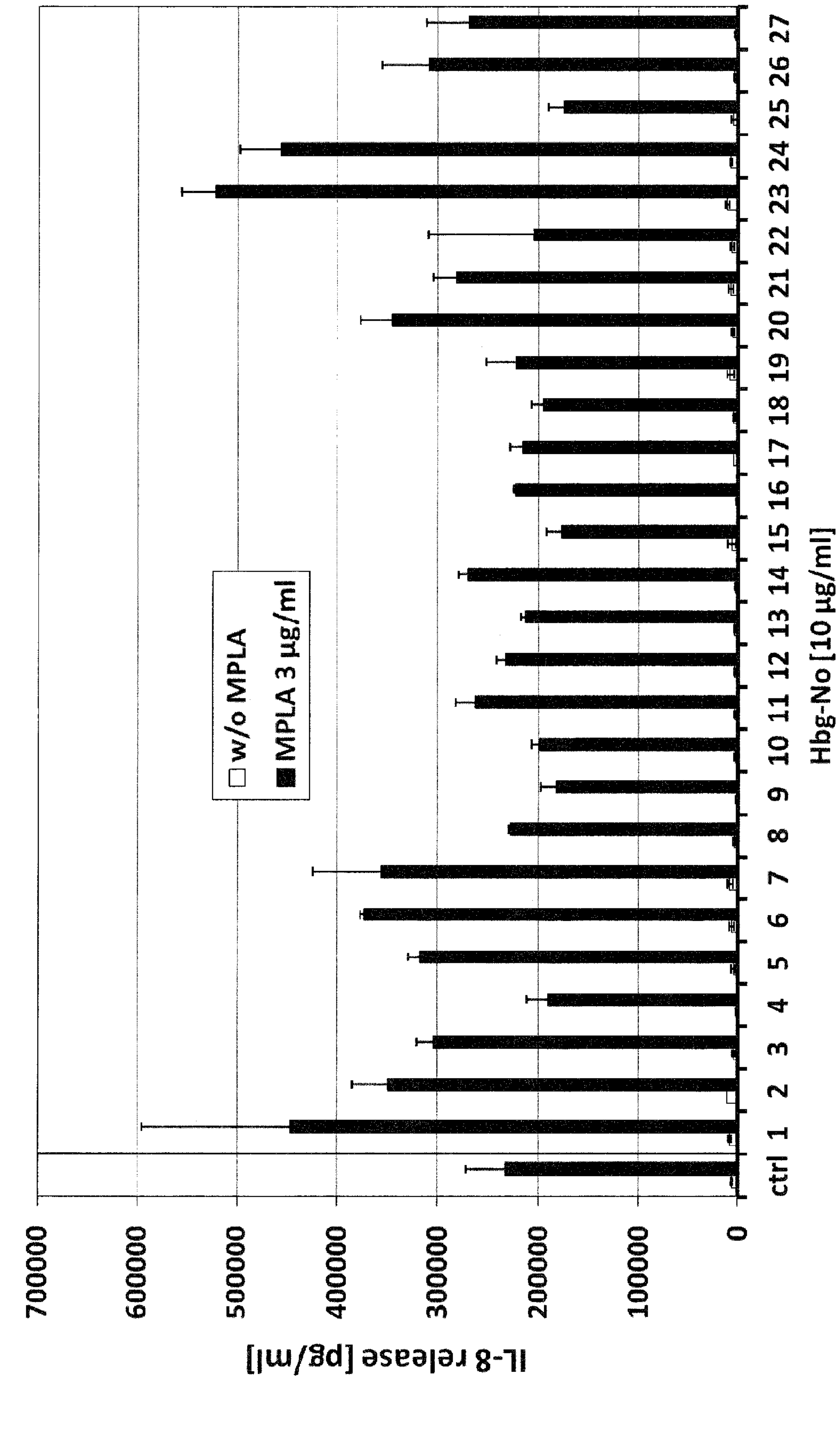
IL-8 release [pg/ml]
700000
600000
500000
400000
300000
200000
100000
0
w/o MPLA
MPLA 3 µg/ml
ctrl 1 2 3 4 5 6 7 8 9 10 11 12 13 14 15 16 17 18 19 20 21 22 23 24 25 26 27
Hbg-No [10 µg/ml]

Fig. 5

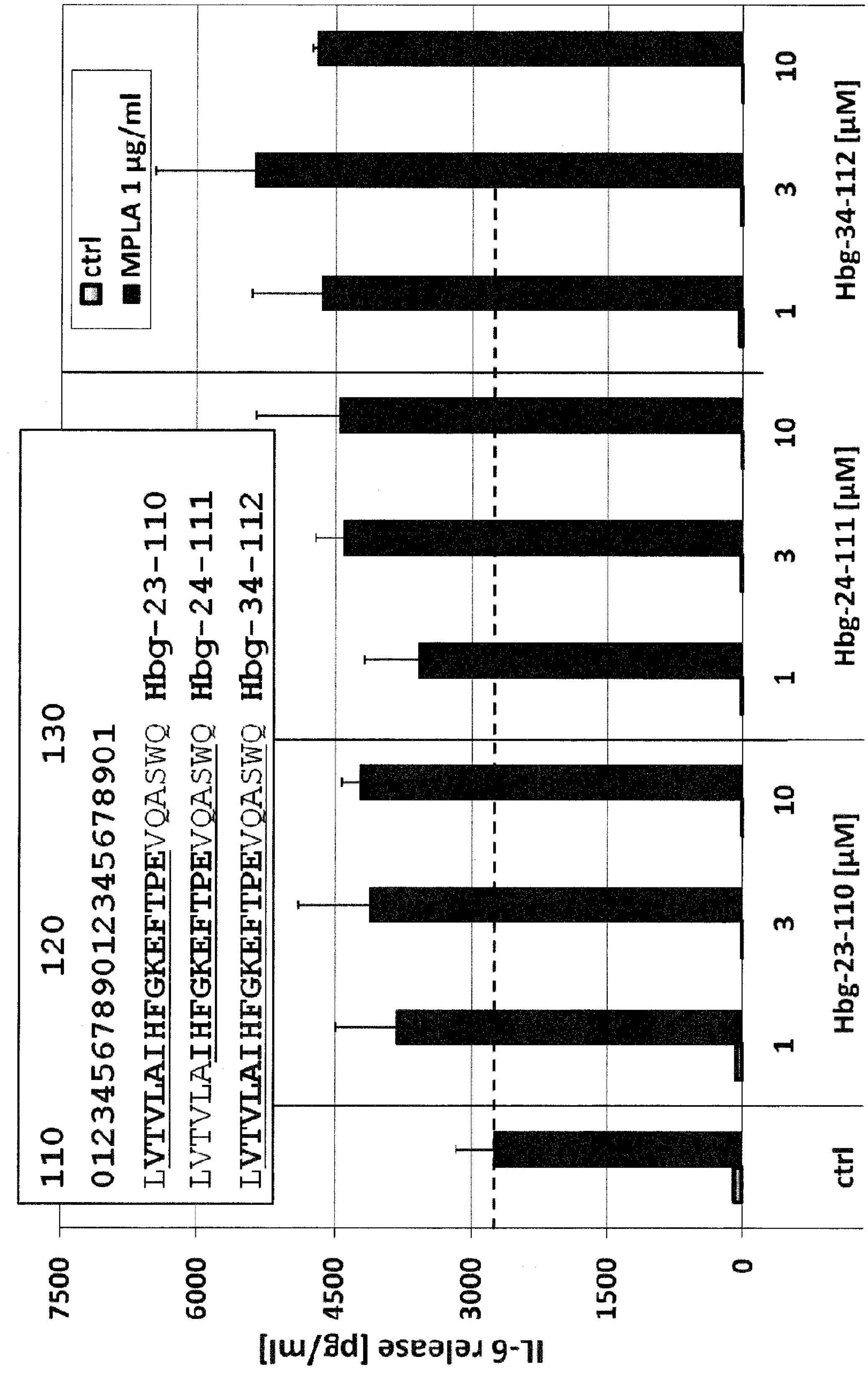
110 120 130
0123456789012345678901
LVTVLAIHFGKEFTPEVQASWQ Hbg-23-110
LVTVLAIHFGKEFTPEVQASWQ Hbg-24-111
LVTVLAIHFGKEFTPEVQASWQ Hbg-34-112
ctrl
MPLA 1 µg/ml
IL-6 release [pg/ml]
7500
6000
4500
3000
1500
0
ctrl
1
3
10
Hbg-23-110 [µM]
1
3
10
Hbg-24-111 [µM]
1
3
10
Hbg-34-112 [µM]

**Fig. 6**

**Sequence of hemoglobin subunit gamma-1**

```
       1          11         21         31         41         51
 -1  MGHFTEEDKAT ITSLWGKVNV EDAGGETLGR LLVVYPWTQR FFDSFGNLSS ASAIMGNPKV
 61   KAHGKKVLTS LGDAIKHLDD LKGTFAQLSE LHCDKLHVDP ENFKLLGNVL VTVLAIHFGK
121   EFTPEVQASW QKMVTAVASA QSSRYH
```

(Bold/underlined in original: 86–141, AQLSE LHCDKLHVDP ENFKLLGNVL VTVLAIHFGK EFTPEVQASW QKMVTAVASA Q)

**86 ... 141**

**AQLSELHCDKLHVDPENFKLLGNVLVTVLAIHFGKEFTPEVQASWQKMVTAVASAQ**

**Subdivided in three peptides of 30 amino acids:**

**Hbg-30-105 (Hbγ1$_{86-115}$) AQLSELHCDKLHVDPENFKLLGNVLVTVLA**

**Hbg-31-106 (Hbγ1$_{99-128}$) DPENFKLLGNVLVTVLAIHFGKEFTPEVQA**

**Hbg-32-107 (Hbγ1$_{112-141}$) TVLAIHFGKEFTPEVQASWQKMVTAVASAQ**

Fig. 7

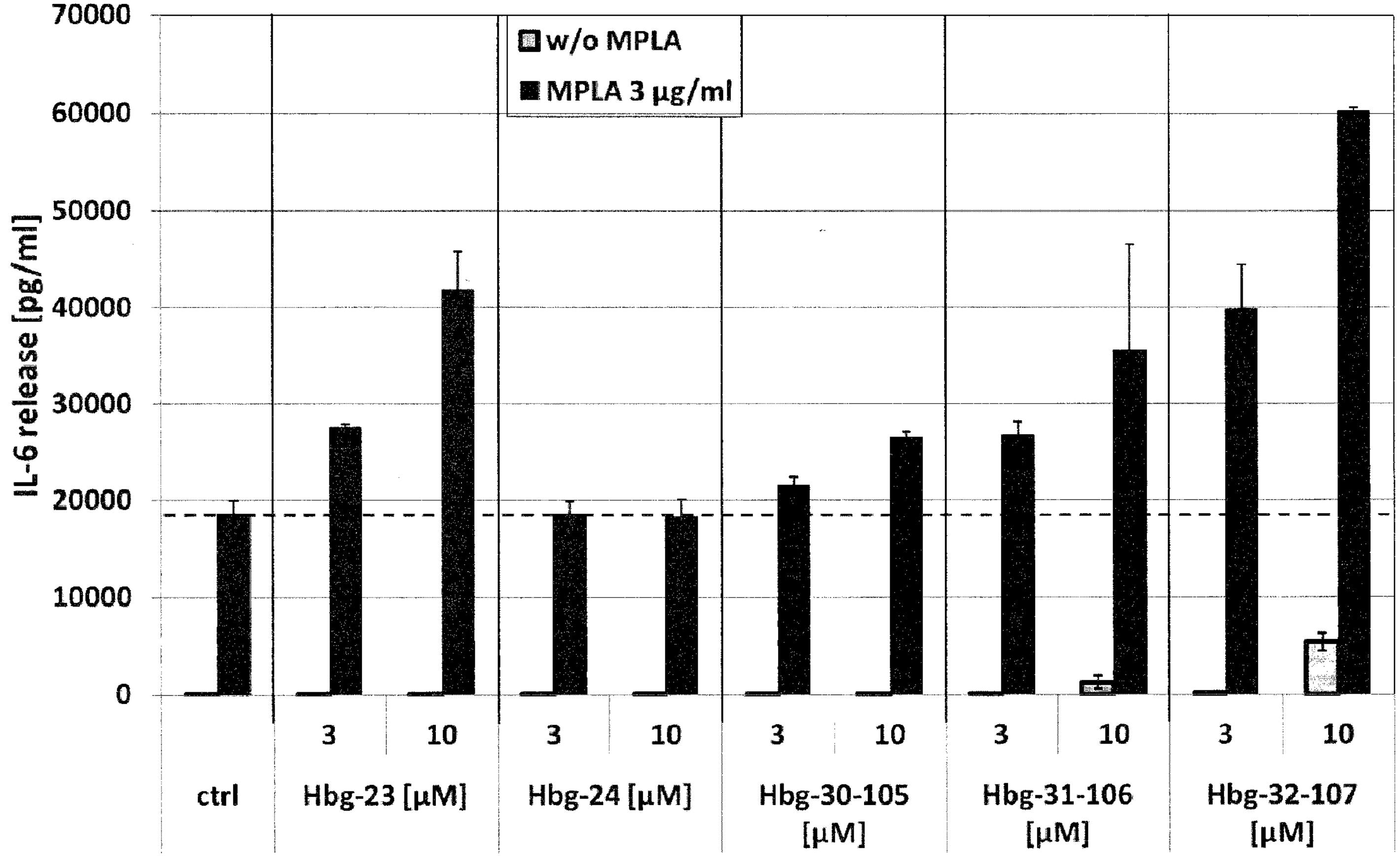


**Hbg-23:** . . . AQLSELHCDKLHVDPENFKLLGNVL**VTVLAIHFGKEFTPE**VQASWQKMVTAVASAQ . . .
**Hbg-24:** . . . AQLSELHCDKLHVDPENFKLLGNVLVTVLA**IHFGKEFTPEVQASW**QKMVTAVASAQ . . .
**Hbg-30-105:** . . . **AQLSELHCDKLHVDPENFKLLGNVLVTVLA**IHFGKEFTPEVQASWQKMVTAVASAQ . . .
**Hbg-31-106:** . . . AQLSELHCDKLHV**DPENFKLLGNVLVTVLAIHFGKEFTPEVQA**SWQKMVTAVASAQ . . .
**Hbg-32-107:** . . . AQLSELHCDKLHVDPENFKLLGNVLV**TVLAIHFGKEFTPEVQASWQKMVTAVASAQ** . . .

Fig. 8

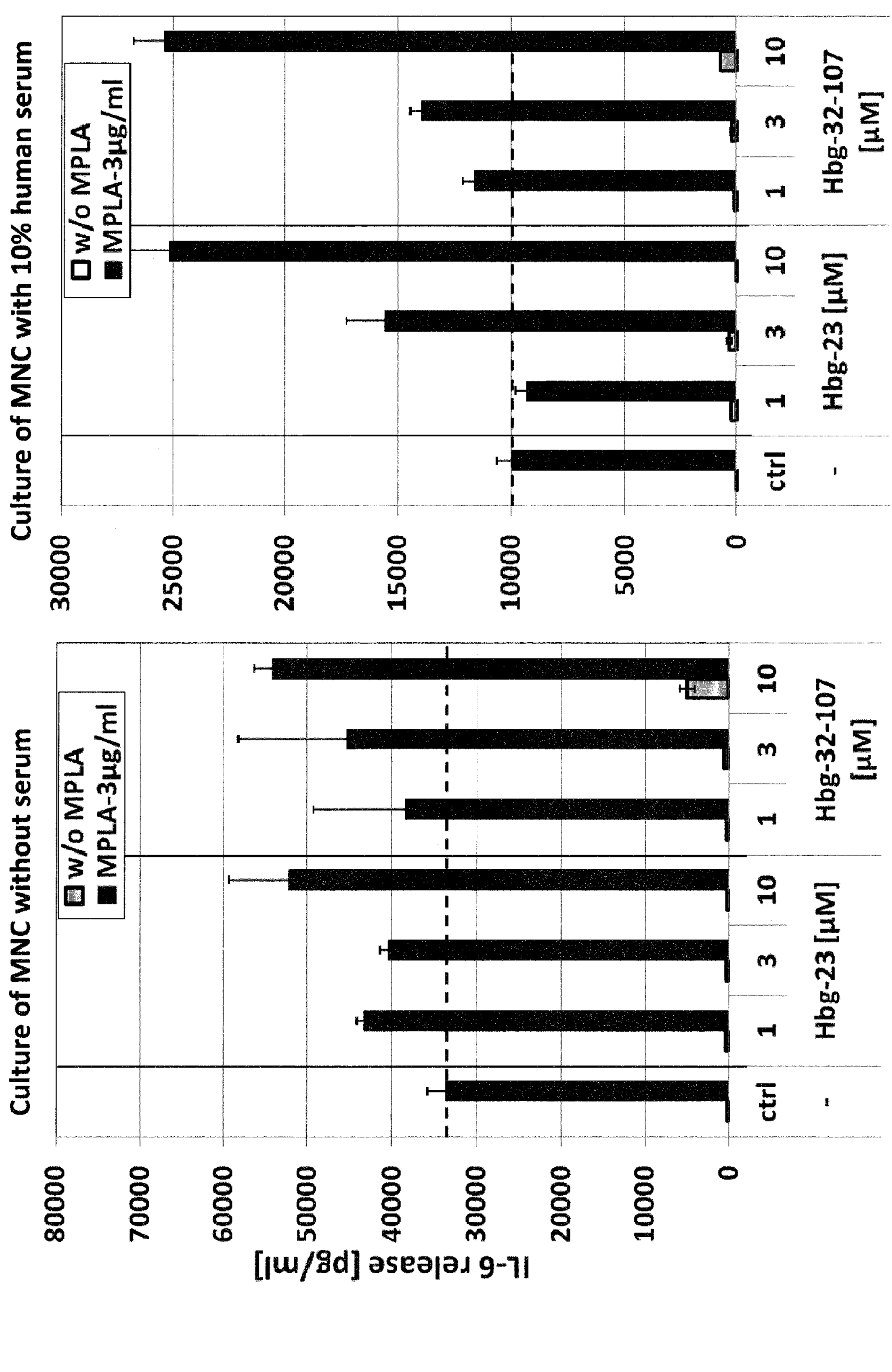
Culture of MNC without serum
w/o MPLA
MPLA-3µg/ml
IL-6 release [pg/ml]
80000
70000
60000
50000
40000
30000
20000
10000
0
ctrl
-
1
3
10
Hbg-23 [µM]
1
3
10
Hbg-32-107 [µM]
Culture of MNC with 10% human serum
w/o MPLA
MPLA-3µg/ml
30000
25000
20000
15000
10000
5000
0
ctrl
-
1
3
10
Hbg-23 [µM]
1
3
10
Hbg-32-107 [µM]

Fig. 9

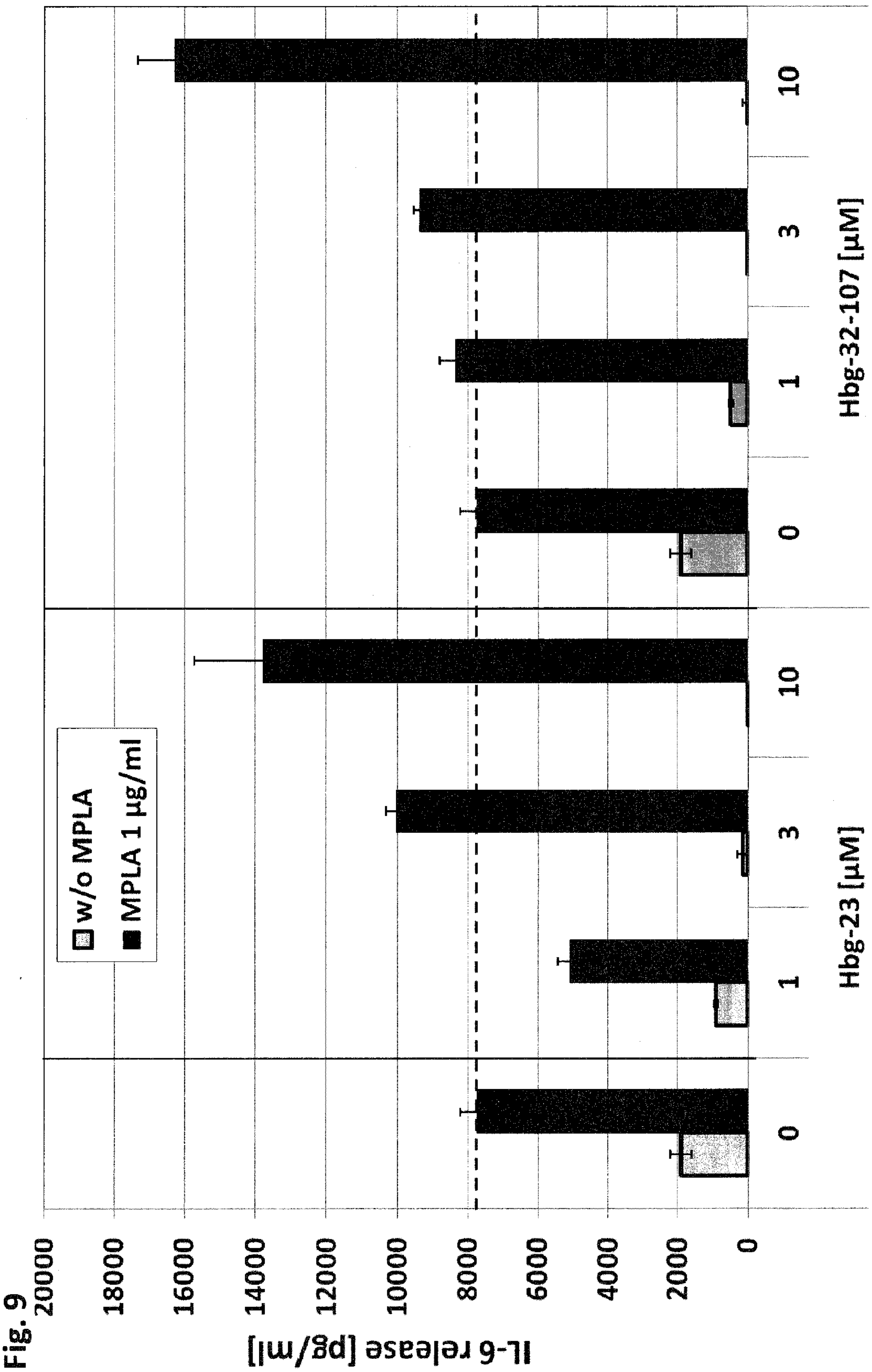
IL-6 release [pg/ml]
20000
18000
16000
14000
12000
10000
8000
6000
4000
2000
0
w/o MPLA
MPLA 1 µg/ml
0
1
3
10
Hbg-23 [µM]
0
1
3
10
Hbg-32-107 [µM]

Fig. 10

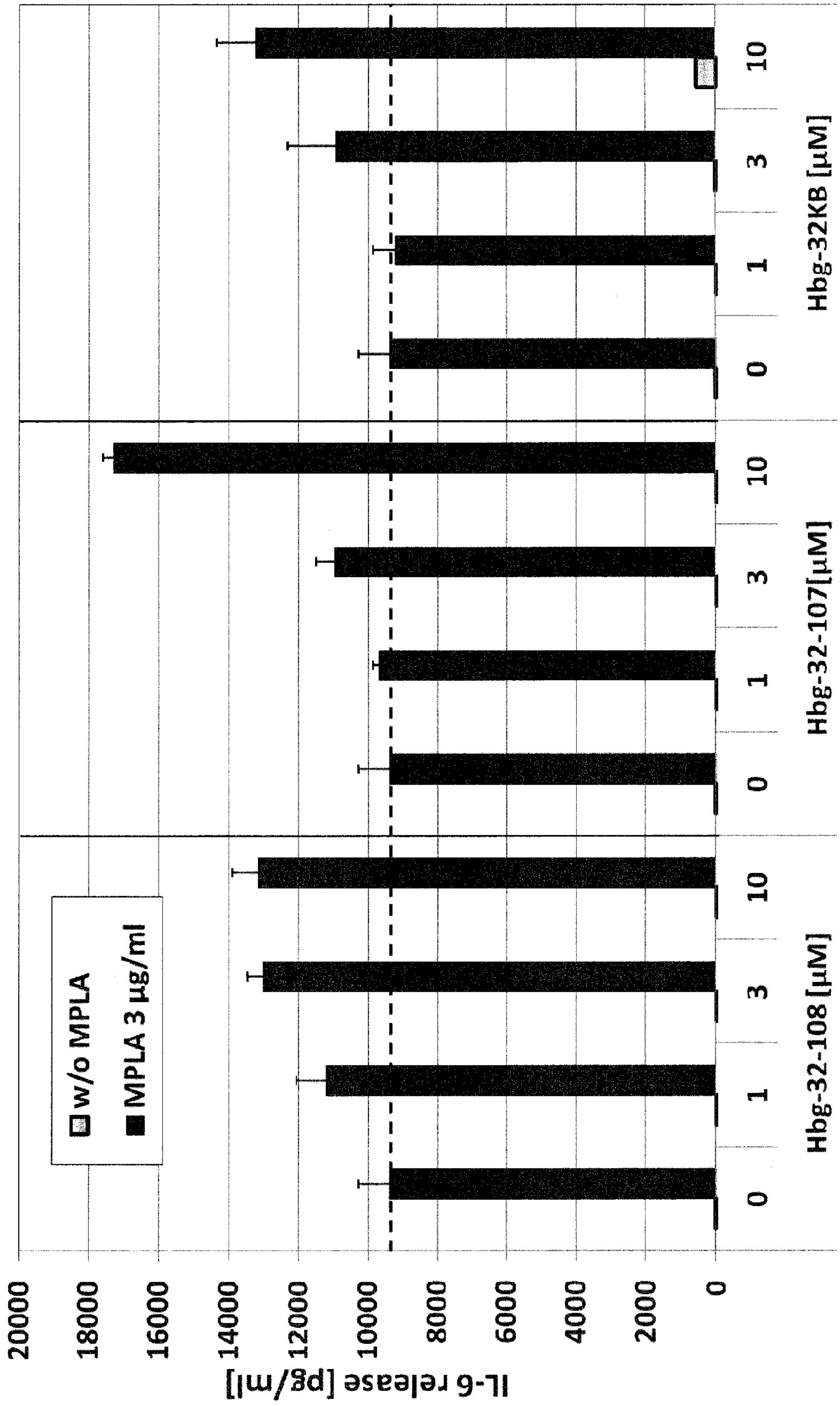

w/o MPLA
MPLA 3 µg/ml
IL-6 release [pg/ml]
20000
18000
16000
14000
12000
10000
8000
6000
4000
2000
0
0
1
3
10
Hbg-32-108 [µM]
Hbg-32-107[µM]
Hbg-32KB [µM]

Fig. 11

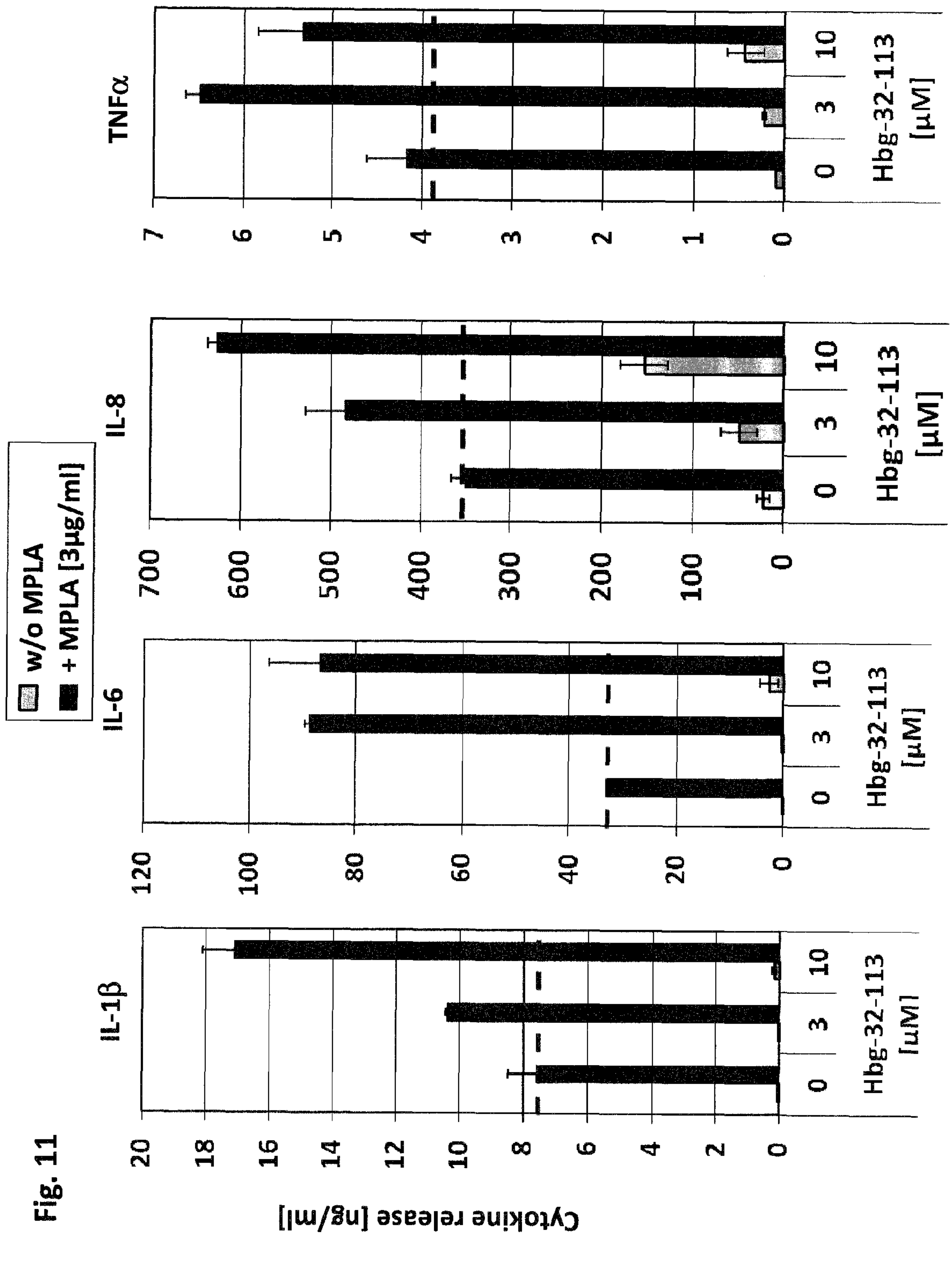
w/o MPLA
+ MPLA [3µg/ml]
IL-1β
IL-6
IL-8
TNFα
Cytokine release [ng/ml]
Hbg-32-113 [µM]
0
3
10

Fig. 12

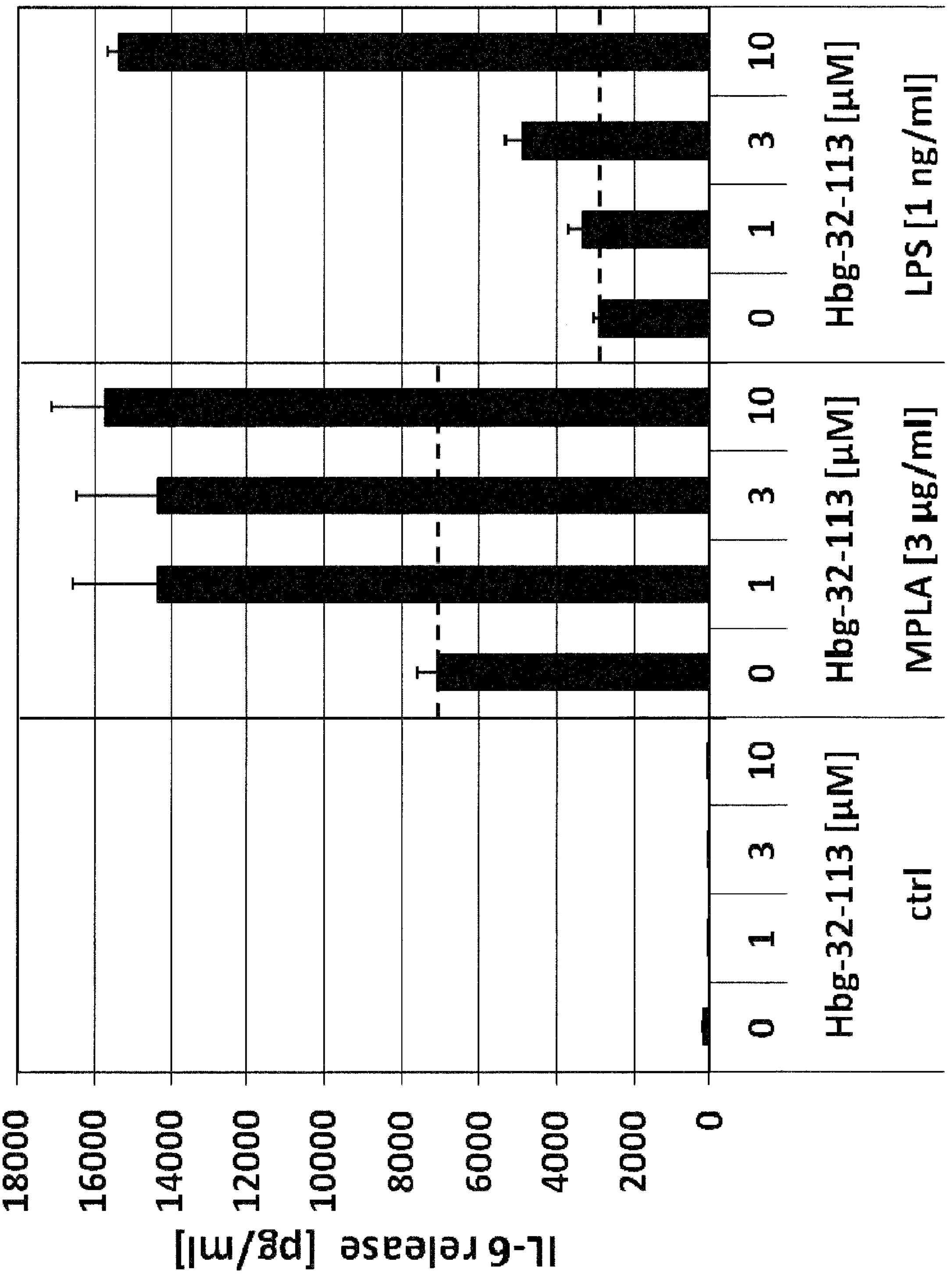
IL-6 release [pg/ml]
18000
16000
14000
12000
10000
8000
6000
4000
2000
0
0
1
3
10
Hbg-32-113 [µM]
ctrl
0
1
3
10
Hbg-32-113 [µM]
MPLA [3 µg/ml]
0
1
3
10
Hbg-32-113 [µM]
LPS [1 ng/ml]

Fig. 13

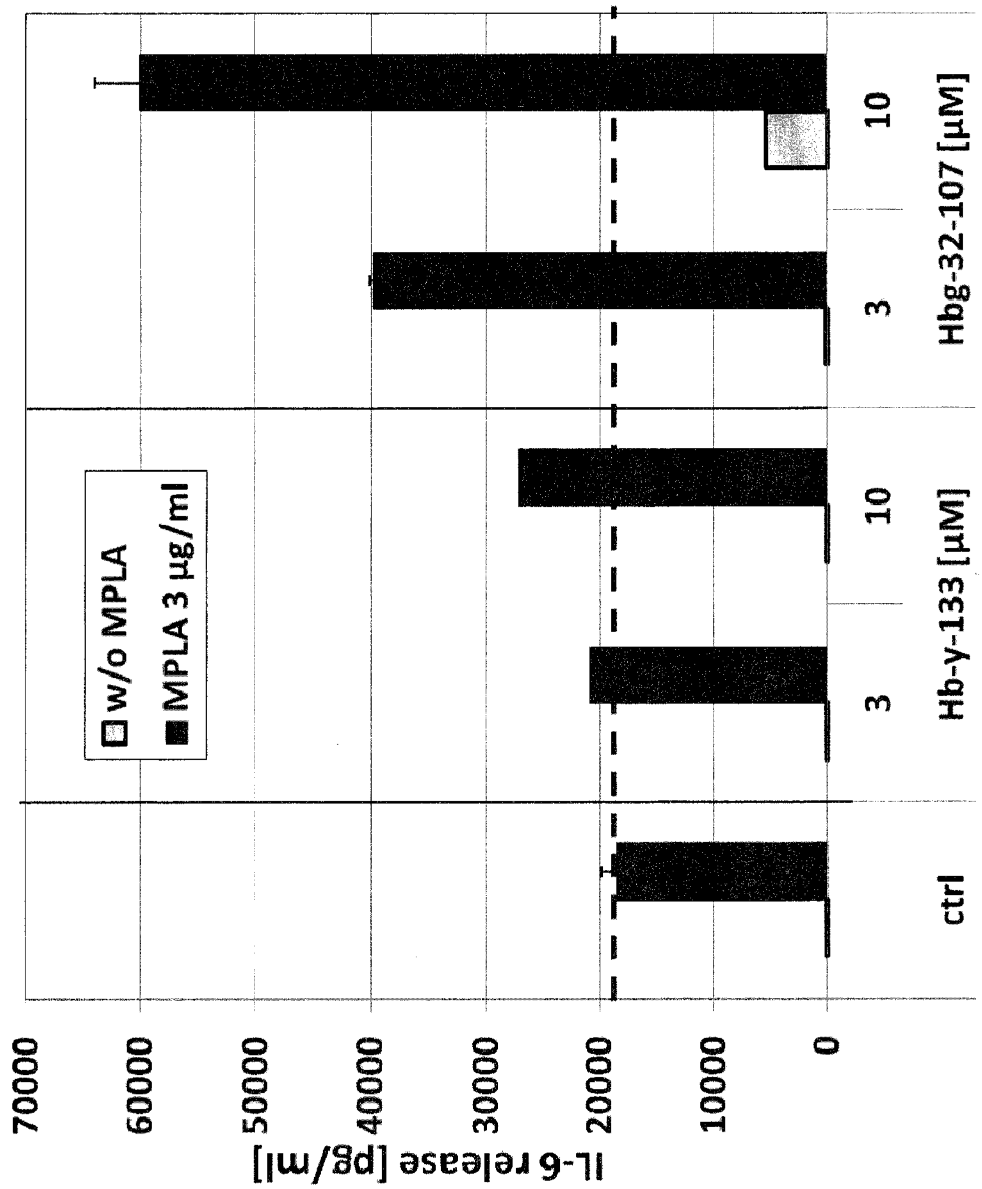
IL-6 release [pg/ml]
70000
60000
50000
40000
30000
20000
10000
0
w/o MPLA
MPLA 3 µg/ml
ctrl
3
10
Hb-γ-133 [µM]
3
10
Hbg-32-107 [µM]

Fig. 14

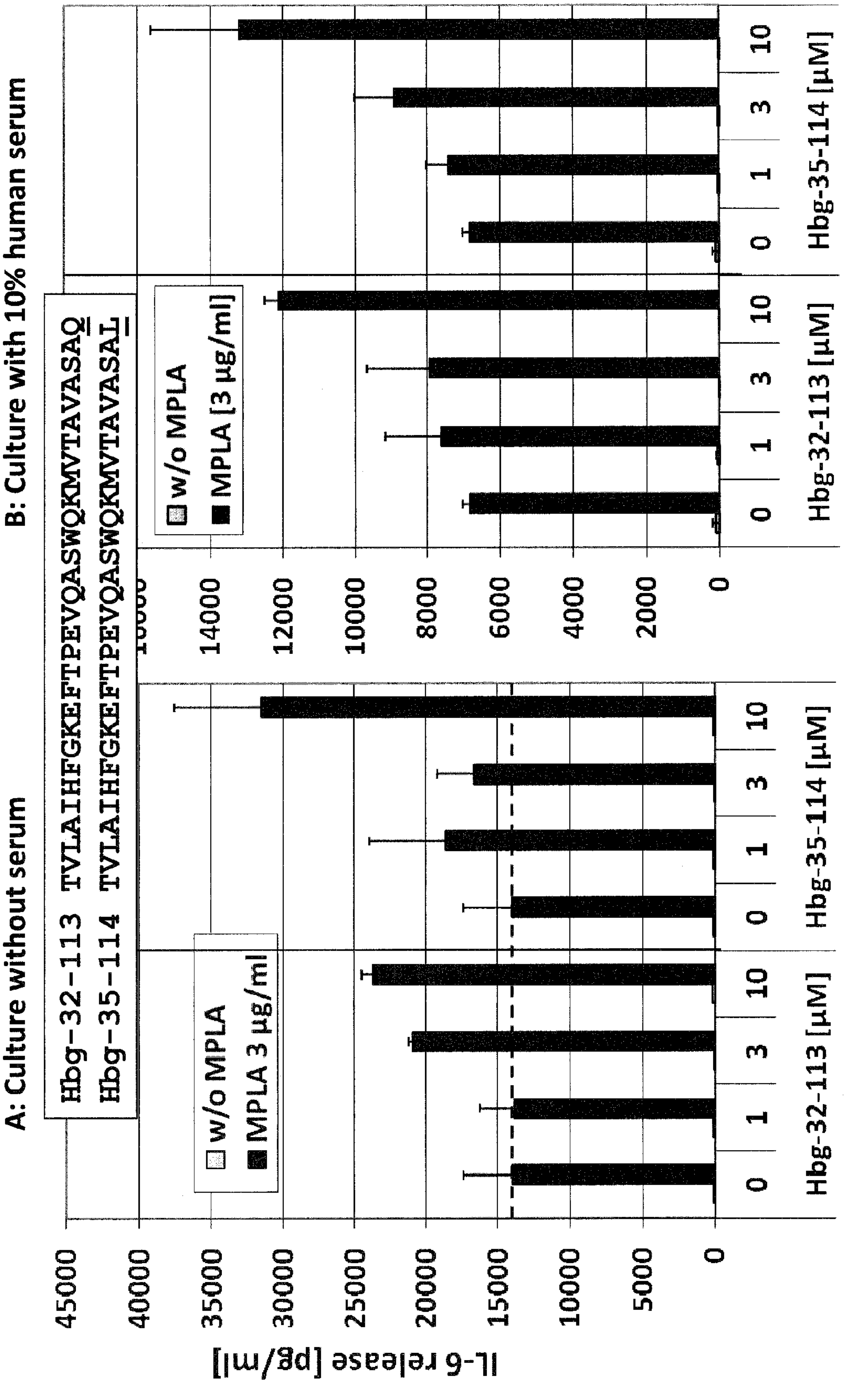
A: Culture without serum
B: Culture with 10% human serum
Hbg-32-113 TVLAIHFGKEFTPEVQASWQKMVTAVASAQ
Hbg-35-114 TVLAIHFGKEFTPEVQASWQKMVTAVASAL
w/o MPLA
MPLA 3 µg/ml
MPLA [3 µg/ml]
IL-6 release [pg/ml]
45000
40000
35000
30000
25000
20000
15000
10000
5000
0
14000
12000
10000
8000
6000
4000
2000
0
0 1 3 10
Hbg-32-113 [µM]
Hbg-35-114 [µM]

Fig. 15

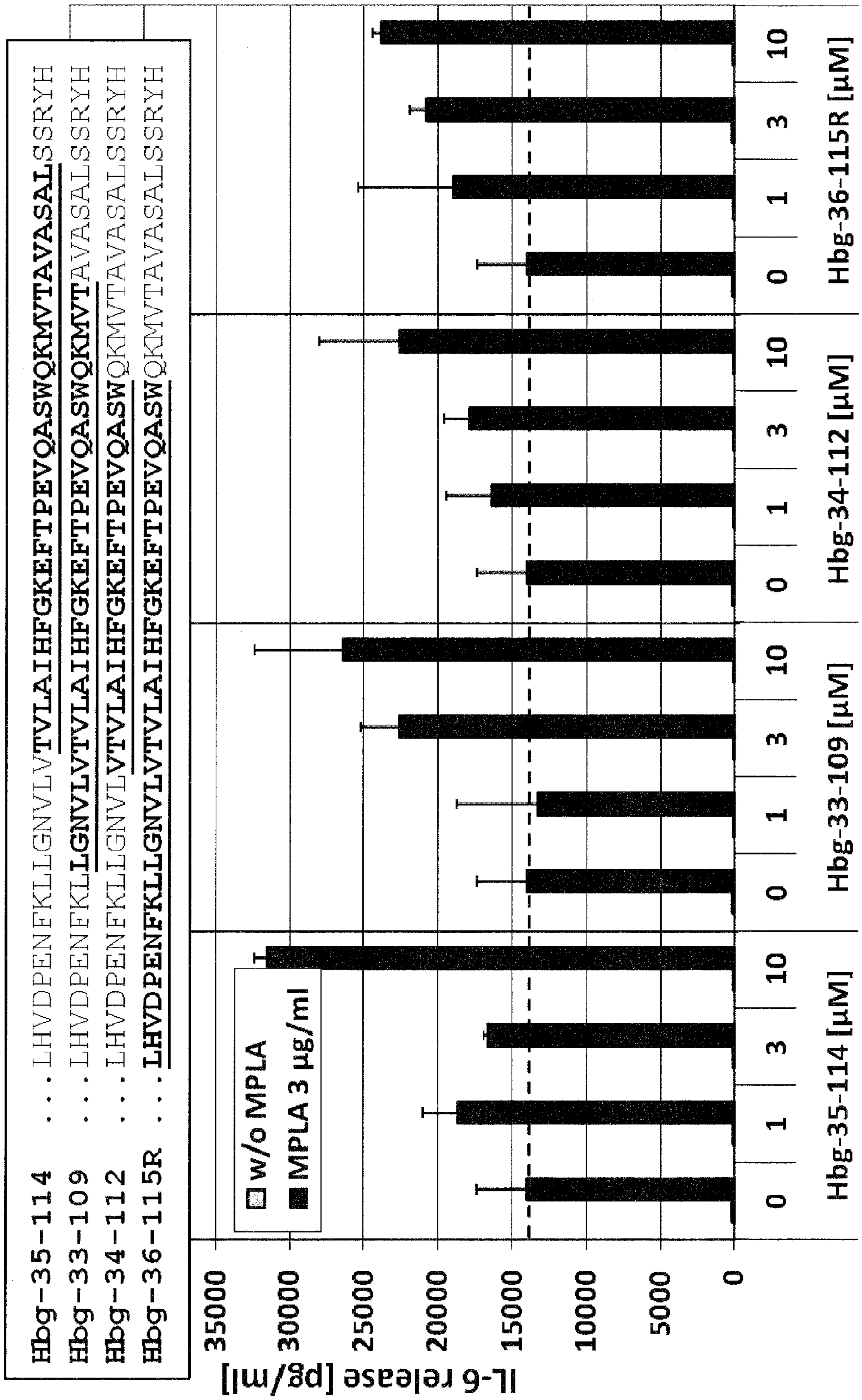

Hbg-35-114 ...LHVDPENFKLLGNVLVTVLAIHFGKEFTPEVQASWQKMVTAVASALSSRYH
Hbg-33-109 ...LHVDPENFKLLGNVLVTVLAIHFGKEFTPEVQASWQKMVTAVASALSSRYH
Hbg-34-112 ...LHVDPENFKLLGNVLVTVLAIHFGKEFTPEVQASWQKMVTAVASALSSRYH
Hbg-36-115R ...LHVDPENFKLLGNVLVTVLAIHFGKEFTPEVQASWQKMVTAVASALSSRYH
w/o MPLA
MPLA 3 µg/ml
IL-6 release [pg/ml]
35000
30000
25000
20000
15000
10000
5000
0
0
1
3
10
Hbg-35-114 [µM]
Hbg-33-109 [µM]
Hbg-34-112 [µM]
Hbg-36-115R [µM]

Fig. 16

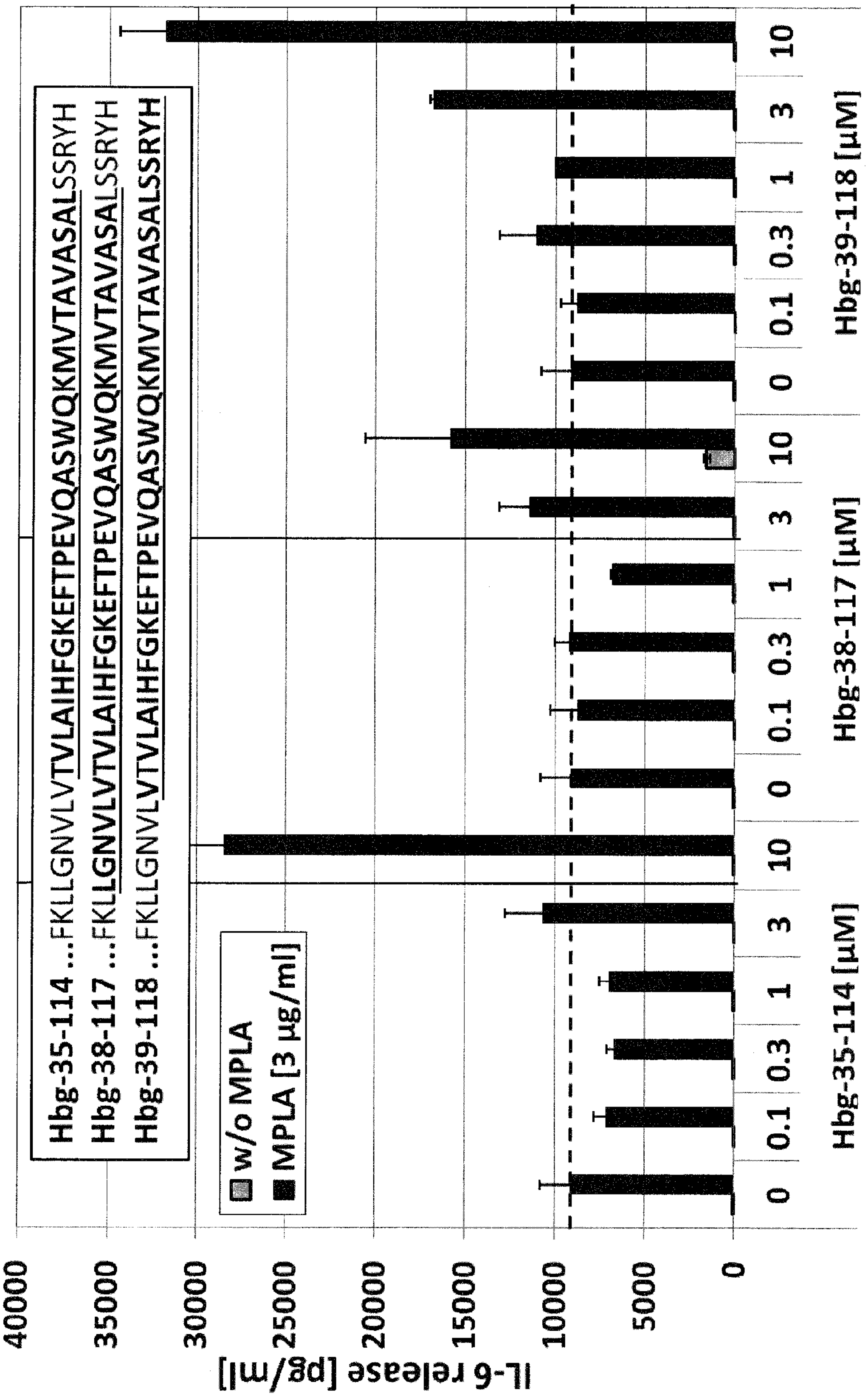
Hbg-35-114 ....FKLLGNVLVTVLAIHFGKEFTPEVQASWQKMVTAVASALSSRYH
Hbg-38-117 ....FKLLGNVLVTVLAIHFGKEFTPEVQASWQKMVTAVASALSSRYH
Hbg-39-118 ....FKLLGNVLVTVLAIHFGKEFTPEVQASWQKMVTAVASALSSRYH
w/o MPLA
MPLA [3 µg/ml]
IL-6 release [pg/ml]
40000
35000
30000
25000
20000
15000
10000
5000
0
0 0.1 0.3 1 3 10
Hbg-35-114 [µM]
0 0.1 0.3 1 3 10
Hbg-38-117 [µM]
0 0.1 0.3 1 3 10
Hbg-39-118 [µM]

Fig. 17

| | | |
|---|---|---|
| Hbg-35 | **TVLAIHFGKEFTPEVQASWQKMVTAVASAL** | |
| Hbg-42 | **A**VLAIHFGKEFTPEVQASWQKMVTAVASAL | $Thr_{112}$/Ala |
| Hbg-43 | T**A**LAIHFGKEFTPEVQASWQKMVTAVASAL | $Val_{113}$/Ala |
| Hbg-44 | TV**A**AIHFGKEFTPEVQASWQKMVTAVASAL | $Leu_{114}$/Ala |
| Hbg-45 | TVLA**A**HFGKEFTPEVQASWQKMVTAVASAL | $Ile_{116}$/Ala |
| Hbg-46 | TVLAI**A**FGKEFTPEVQASWQKMVTAVASAL | $His_{117}$/Ala |
| Hbg-47 | TVLAlH**A**GKEFTPEVQASWQKMVTAVASAL | $Phe_{118}$/Ala |
| Hbg-48 | TVLAIHF**A**KEFTPEVQASWQKMVTAVASAL | $Gly_{119}$/Ala |
| Hbg-49 | TVLAIHFG**A**EFTPEVQASWQKMVTAVASAL | $Lys_{120}$/Ala |
| Hbg-50 | TVLAIHFGK**A**FTPEVQASWQKMVTAVASAL | $Glu_{121}$/Ala |
| Hbg-51 | TVLAIHFGKE**A**TPEVQASWQKMVTAVASAL | $Phe_{122}$/Ala |
| Hbg-52 | TVLAIHFGKEF**A**PEVQASWQKMVTAVASAL | $Thr_{123}$/Ala |
| Hbg-53 | TVLAIHFGKEFT**A**EVQASWQKMVTAVASAL | $Pro_{124}$/Ala |
| Hbg-54 | TVLAIHFGKEFTP**A**VQASWQKMVTAVASAL | $Glu_{125}$/Ala |
| Hbg-55 | TVLAIHFGKEFTPE**A**QASWQKMVTAVASAL | $Val_{126}$/Ala |
| Hbg-56 | TVLAIHFGKEFTPEV**A**ASWQKMVTAVASAL | $Gln_{127}$/Ala |
| Hbg-57 | TVLAIHFGKEFTPEVQA**A**WQKMVTAVASAL | $Ser_{129}$/Ala |
| Hbg-58 | TVLAIHFGKEFTPEVQAS**A**QKMVTAVASAL | $Trp_{130}$/Ala |
| Hbg-59 | TVLAIHFGKEFTPEVQASW**A**KMVTAVASAL | $Gln_{131}$/Ala |
| Hbg-60 | TVLAIHFGKEFTPEVQASWQ**A**MVTAVASAL | $Lys_{132}$/Ala |
| Hbg-61 | TVLAIHFGKEFTPEVQASWQK**A**VTAVASAL | $Met_{133}$/Ala |
| Hbg-62 | TVLAIHFGKEFTPEVQASWQKM**A**TAVASAL | $Val_{134}$/Ala |
| Hbg-63 | TVLAIHFGKEFTPEVQASWQKMV**A**AVASAL | $Thr_{135}$/Ala |
| Hbg-64 | TVLAIHFGKEFTPEVQASWQKMVTA**A**ASAL | $Val_{137}$/Ala |
| Hbg-65 | TVLAIHFGKEFTPEVQASWQKMVTAVA**A**AL | $Ser_{139}$/Ala |
| Hbg-66 | TVLAIHFGKEFTPEVQASWQKMVTAVASA**A** | $Leu_{141}$/Ala |

Fig. 18

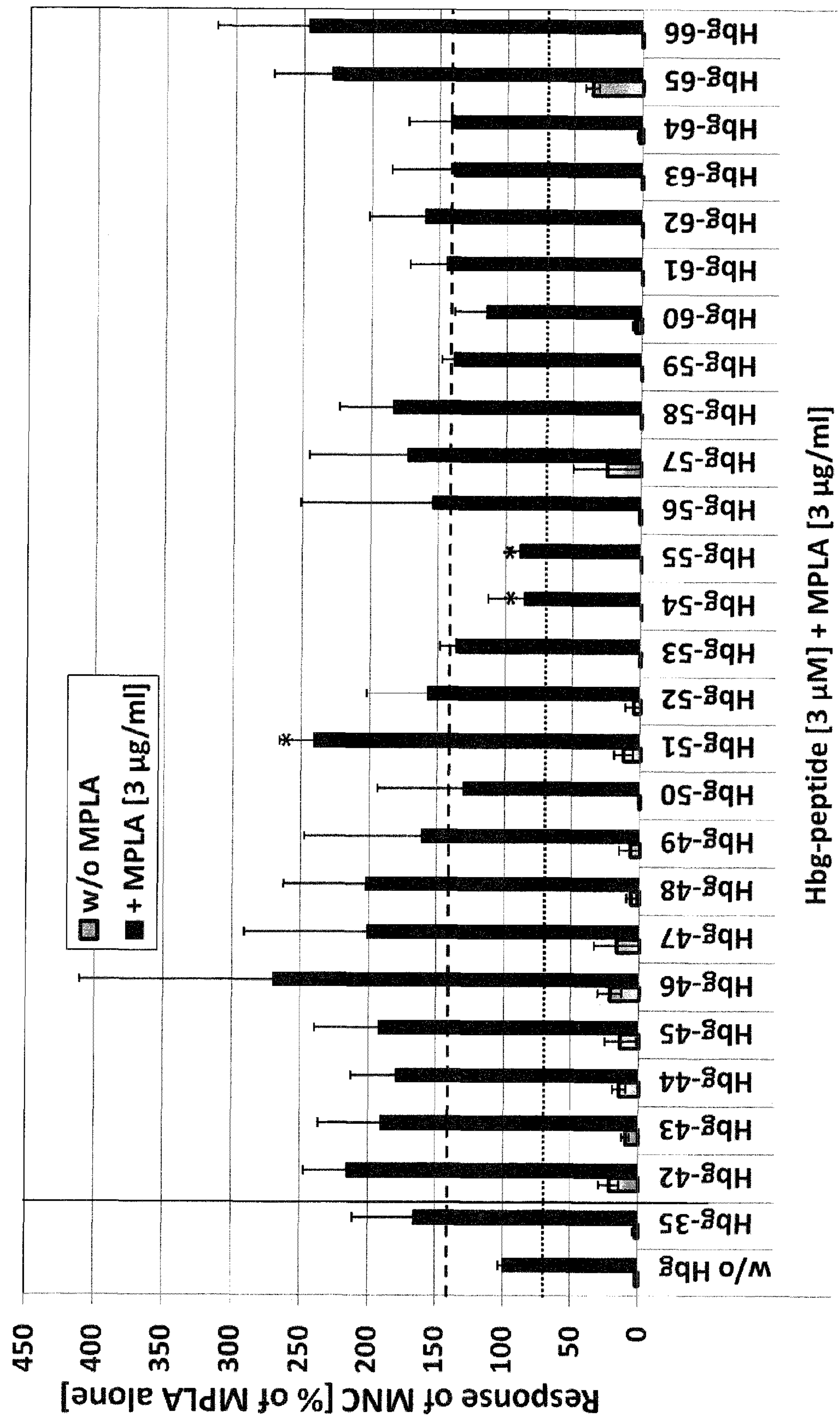
w/o MPLA
+ MPLA [3 µg/ml]
Response of MNC [% of MPLA alone]
450
400
350
300
250
200
150
100
50
0
w/o Hbg
Hbg-35
Hbg-42
Hbg-43
Hbg-44
Hbg-45
Hbg-46
Hbg-47
Hbg-48
Hbg-49
Hbg-50
Hbg-51
Hbg-52
Hbg-53
Hbg-54
Hbg-55
Hbg-56
Hbg-57
Hbg-58
Hbg-59
Hbg-60
Hbg-61
Hbg-62
Hbg-63
Hbg-64
Hbg-65
Hbg-66
Hbg-peptide [3 µM] + MPLA [3 µg/ml]

Fig. 19

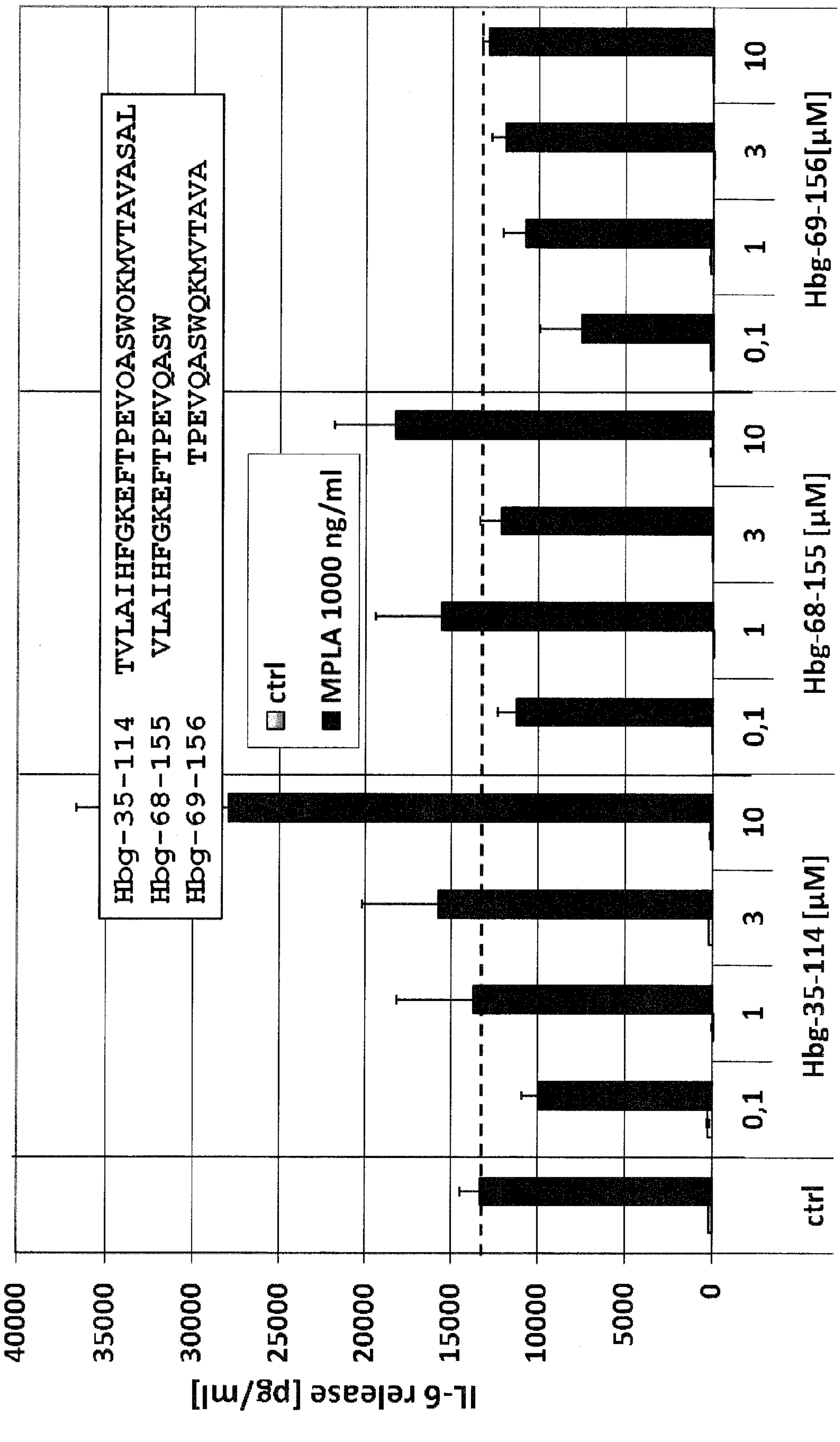
IL-6 release [pg/ml]
40000
35000
30000
25000
20000
15000
10000
5000
0
ctrl
0,1
1
3
10
Hbg-35-114 [µM]
Hbg-68-155 [µM]
Hbg-69-156[µM]
Hbg-35-114 TVLAIHFGKEFTPEVQASWQKMVTAVASAL
Hbg-68-155 VLAIHFGKEFTPEVQASW
Hbg-69-156 TPEVQASWQKMVTAVA
ctrl
MPLA 1000 ng/ml

Fig.20

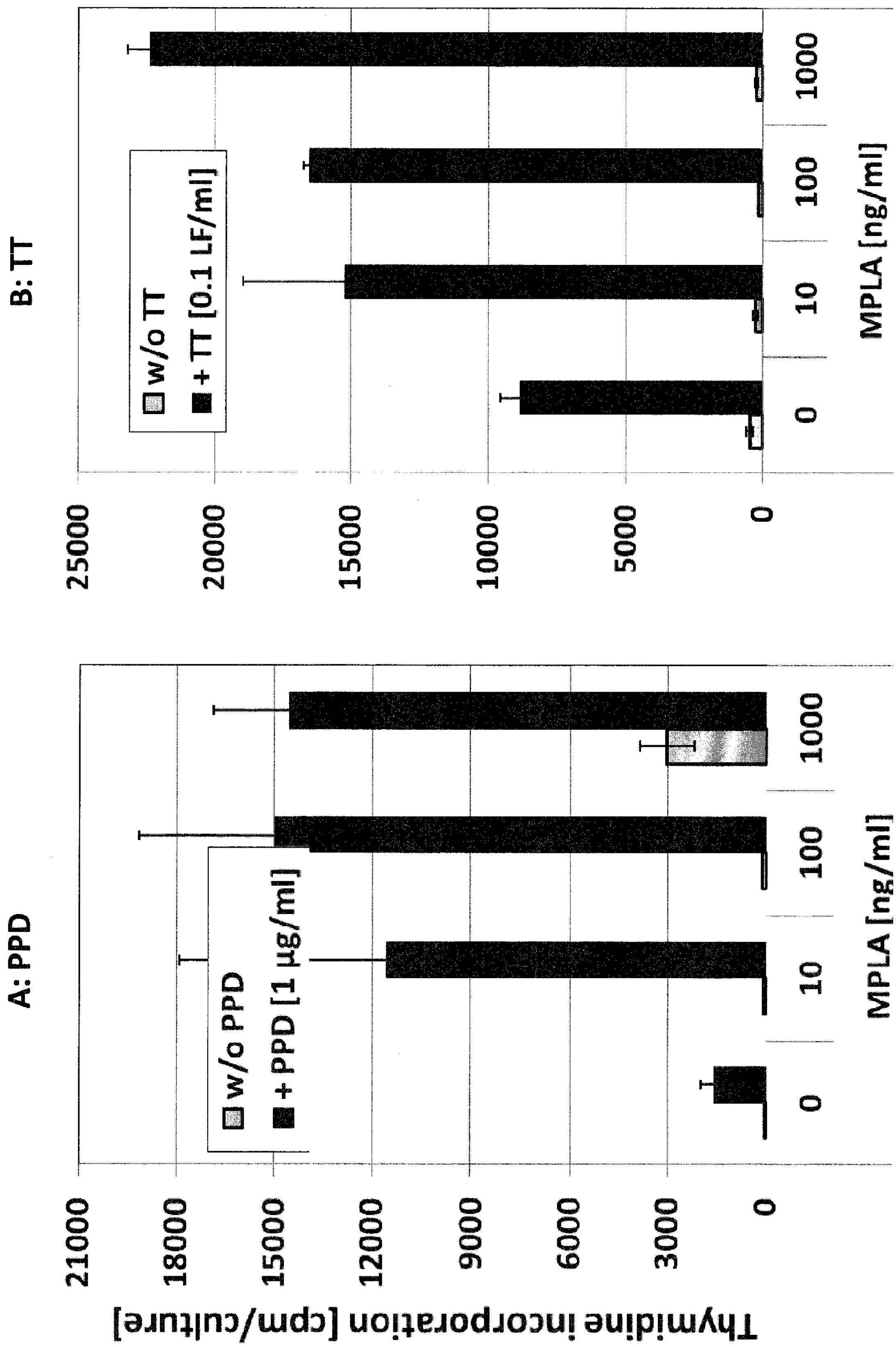
A: PPD
B: TT
w/o PPD
+ PPD [1 µg/ml]
w/o TT
+ TT [0.1 LF/ml]
Thymidine incorporation [cpm/culture]
21000
18000
15000
12000
9000
6000
3000
0
25000
20000
15000
10000
5000
0
0
10
100
1000
MPLA [ng/ml]

Fig. 21

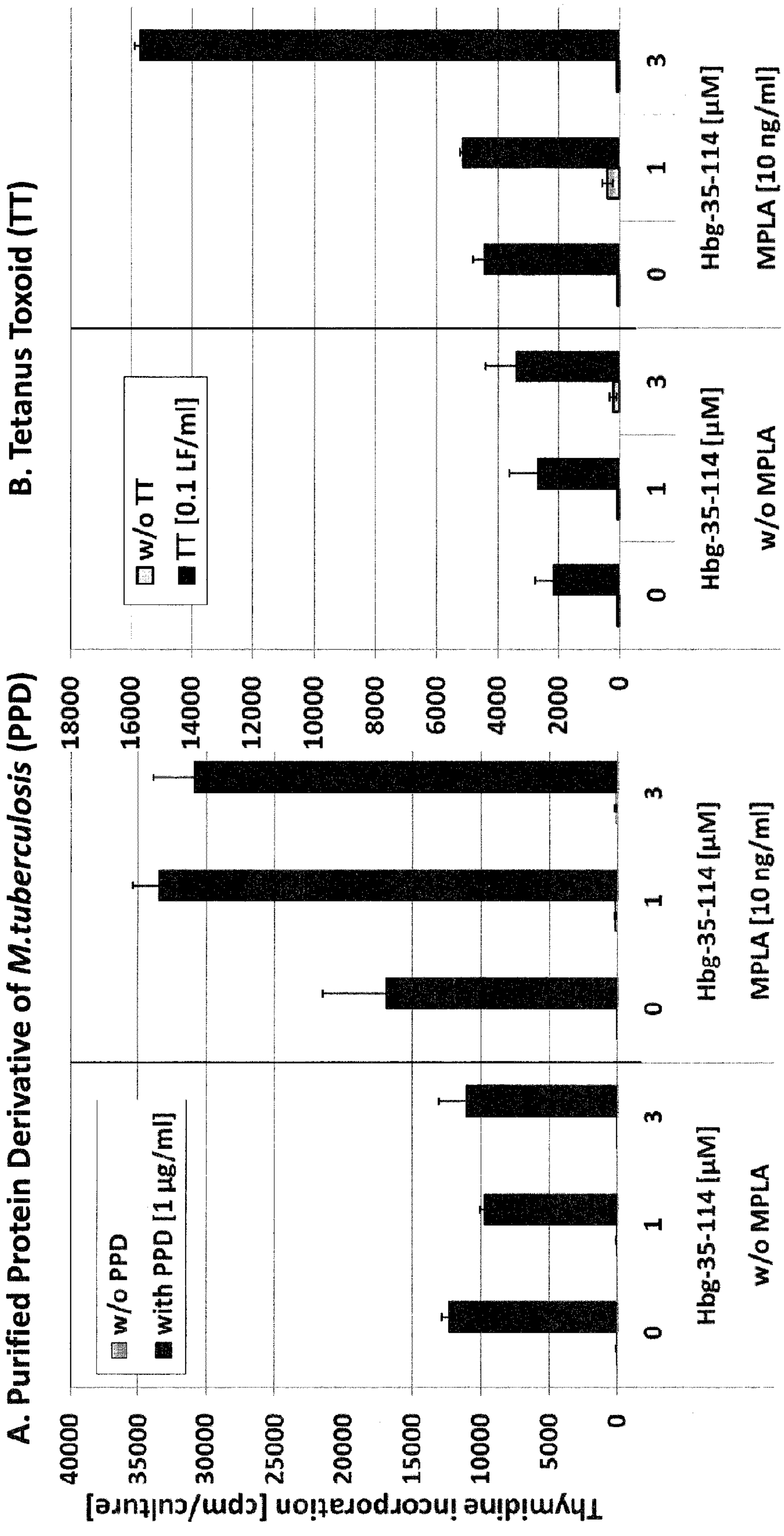
A. Purified Protein Derivative of *M.tuberculosis* (PPD)
Thymidine incorporation [cpm/culture]
40000
35000
30000
25000
20000
15000
10000
5000
0
w/o PPD
with PPD [1 µg/ml]
0
1
3
Hbg-35-114 [µM]
w/o MPLA
MPLA [10 ng/ml]
B. Tetanus Toxoid (TT)
18000
16000
14000
12000
10000
8000
6000
4000
2000
0
w/o TT
TT [0.1 LF/ml]

Fig. 22

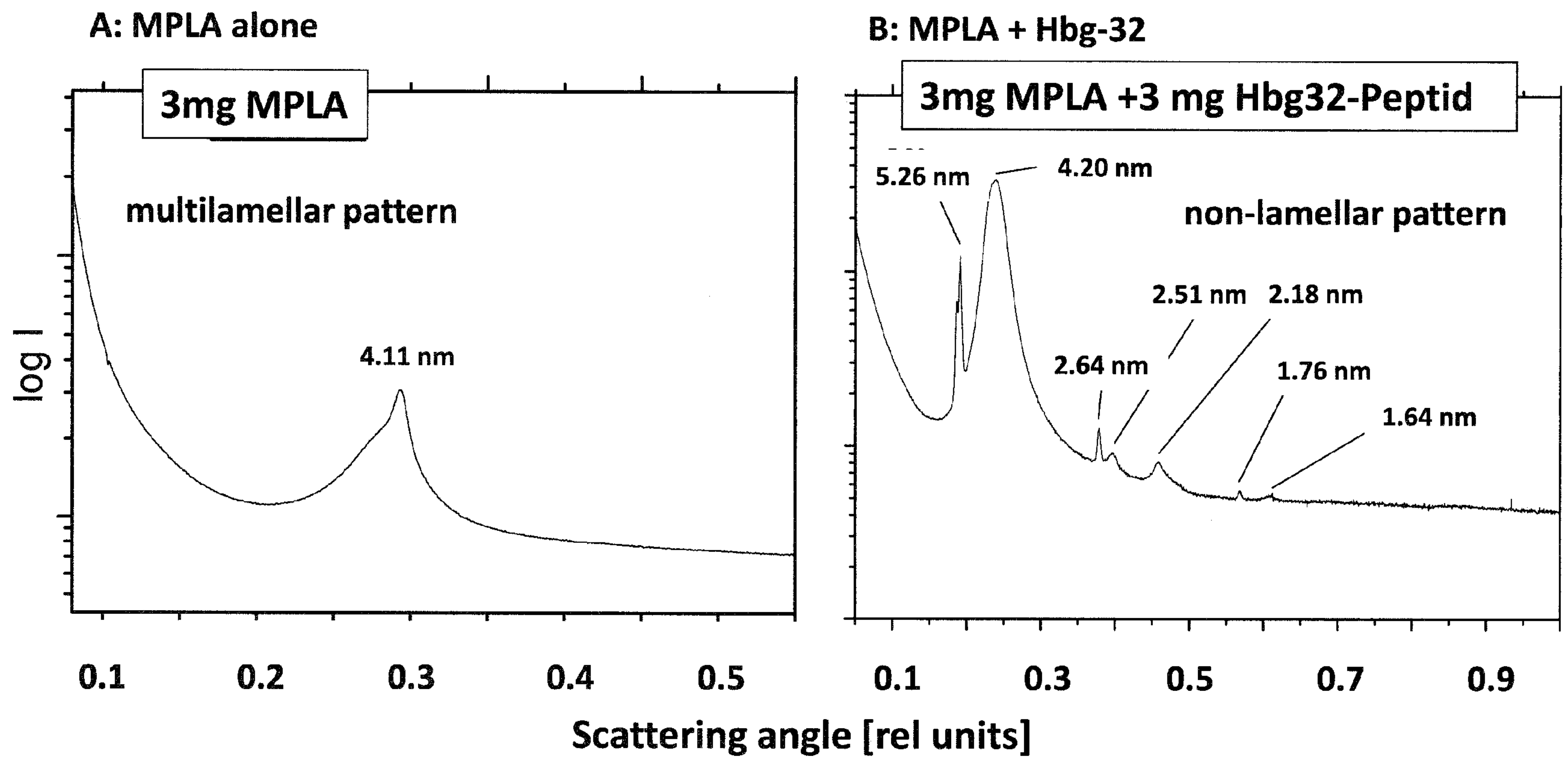


The logarithm of the scattering intensity is plotted versus the scattering vector s (=1/d, d = spacings). In the absence of the peptide, the sharp reflection at 4.11 nm indicates the existence of the multilamellar periodicity. In the presence of the peptide the occurrence of the various additional reflections is characteristic for the existence of a cubic aggregate structure.

Fig. 23

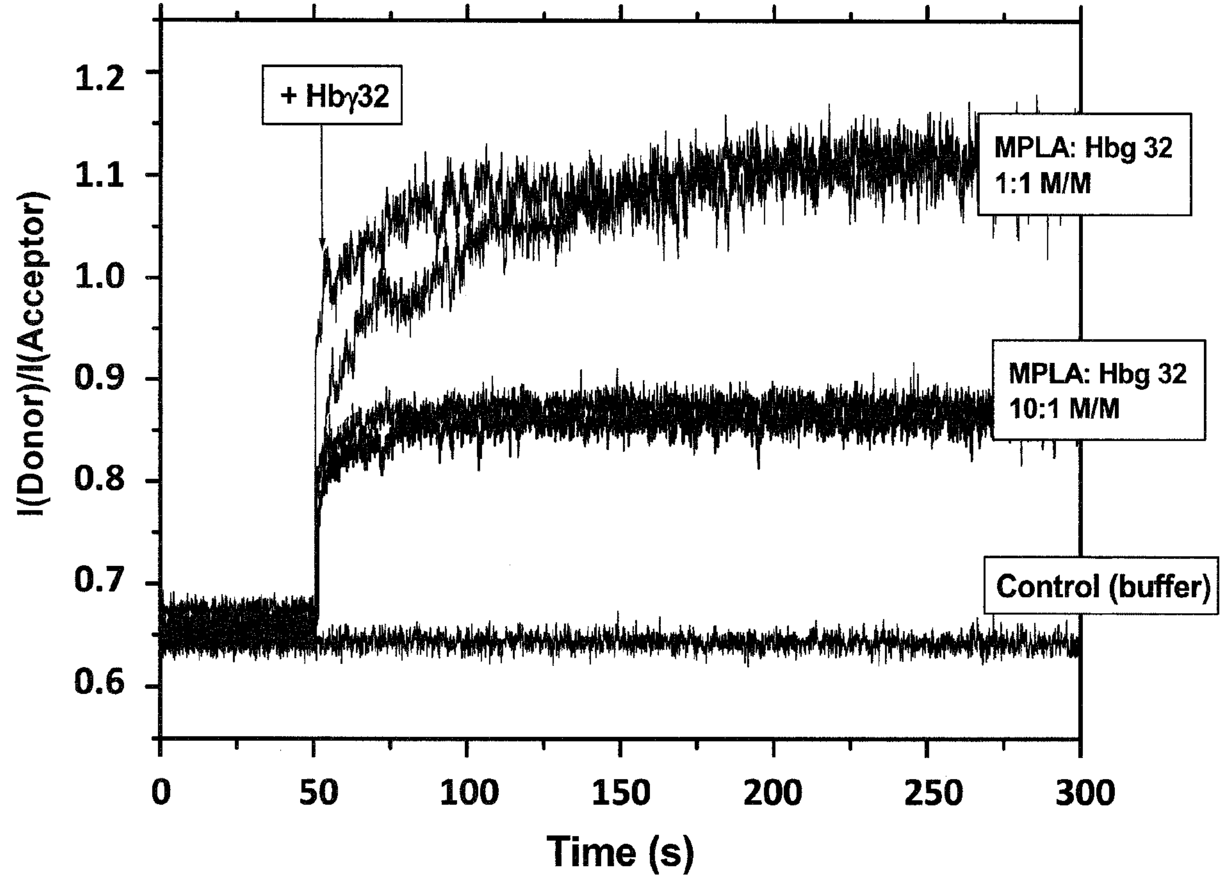


The MPLA aggregates were labeled with the dyes NBD-PE (donor) and RhoPE (acceptor), and the intensity ratio $I_D/I_A$ is a sensitive measure of the incorporation of an external compound such as the Hbg-32 peptide observed here.

Fig. 24

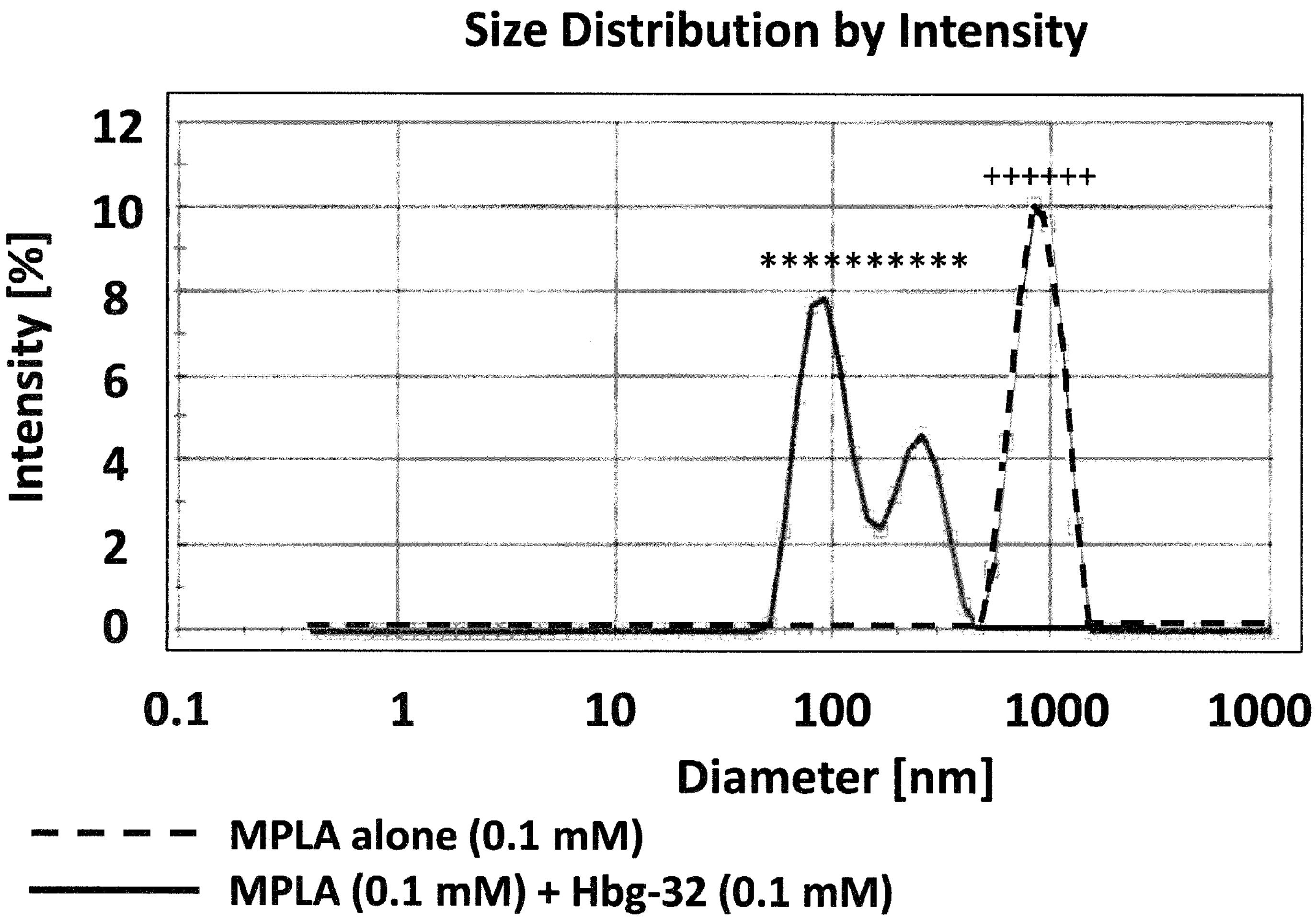


A clear disaggregating effect of MPLA by the peptide is exhibited.

Fig. 25

A: MPLA alone

B: MPLA + Hbg-32

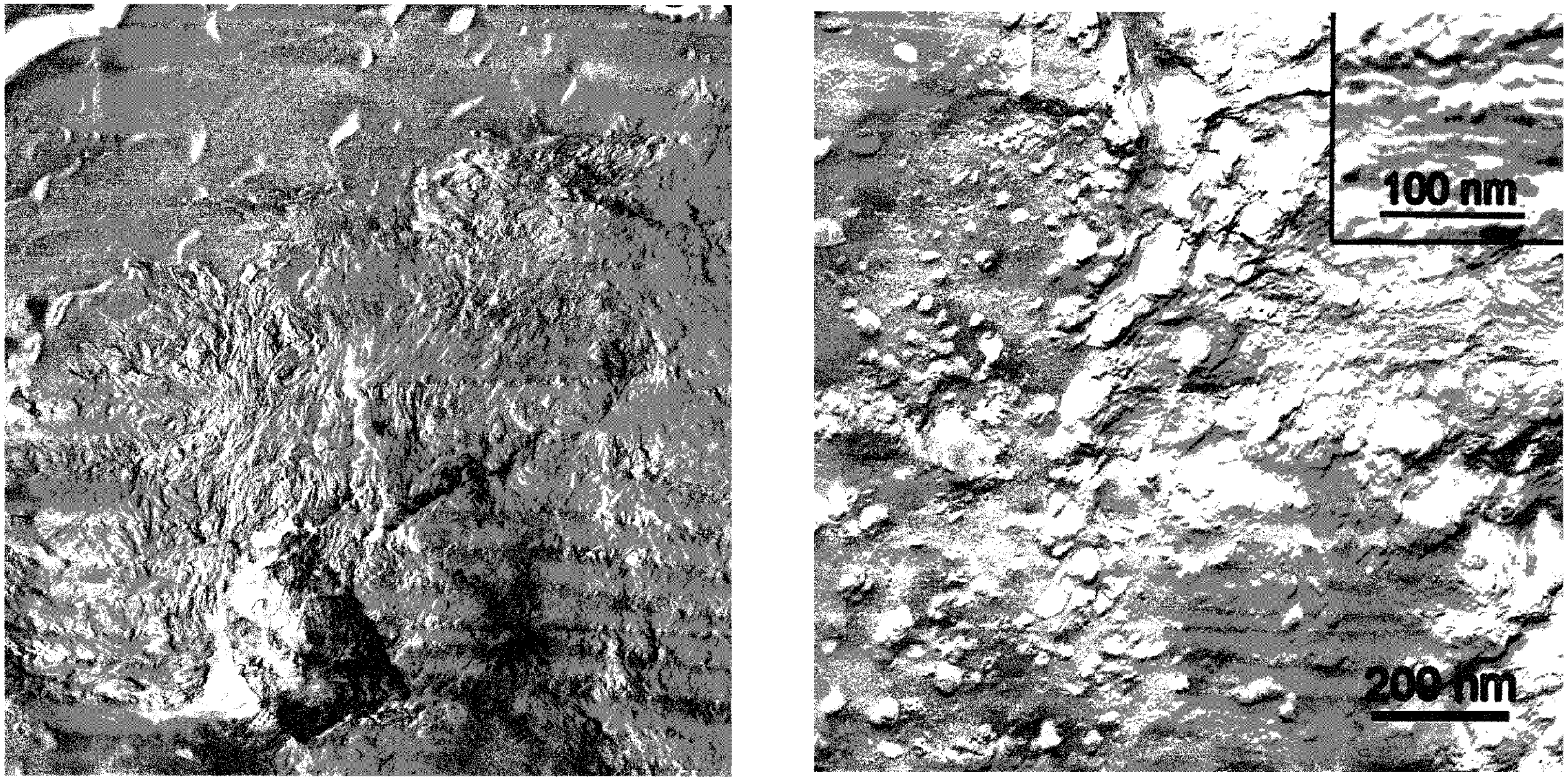


**In the absence of the peptide, the MPLA aggregates are densely packed forming large multibilayered arrangements. In the presence of the peptides, theses aggregates are disspered and become more spherical.**

**Fig. 26**

**A:** MPLA (25 µM) alone on mica.

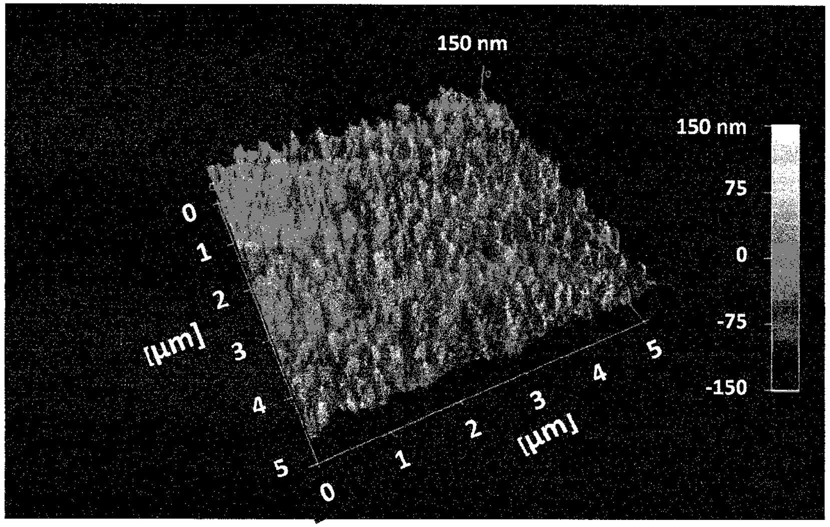


**B:** MPLA (25 µM) + Hbg32 (25 µM) on mica

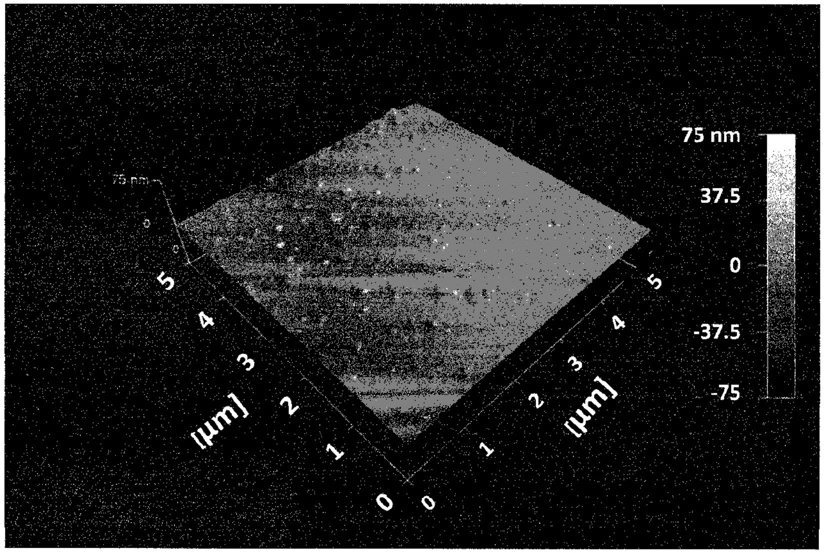

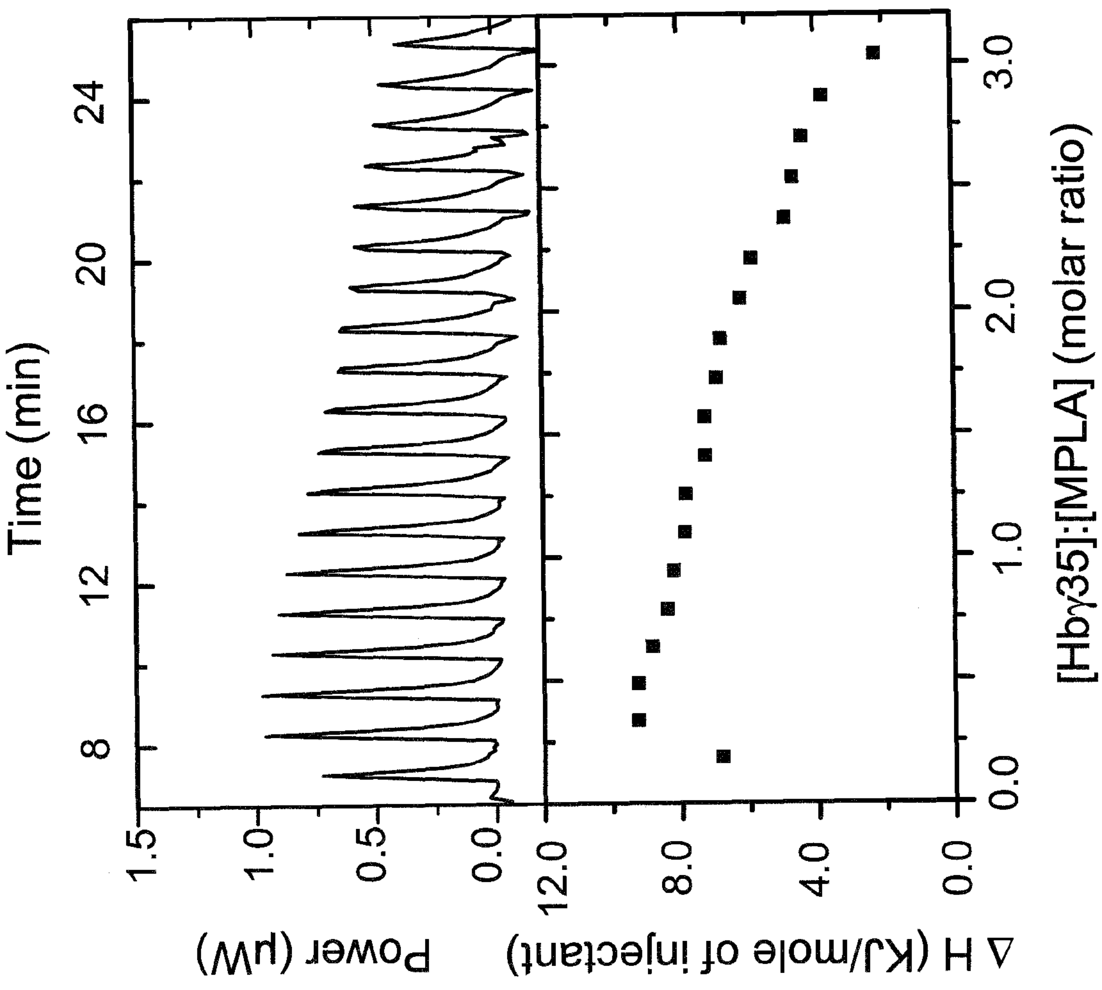

Time (min)
8
12
16
20
24
Power (μW)
1.5
1.0
0.5
0.0
Δ H (KJ/mole of injectant)
12.0
8.0
4.0
0.0
[Hbγ35]:[MPLA] (molar ratio)
0.0
1.0
2.0
3.0


Fig. 27

Fig. 28

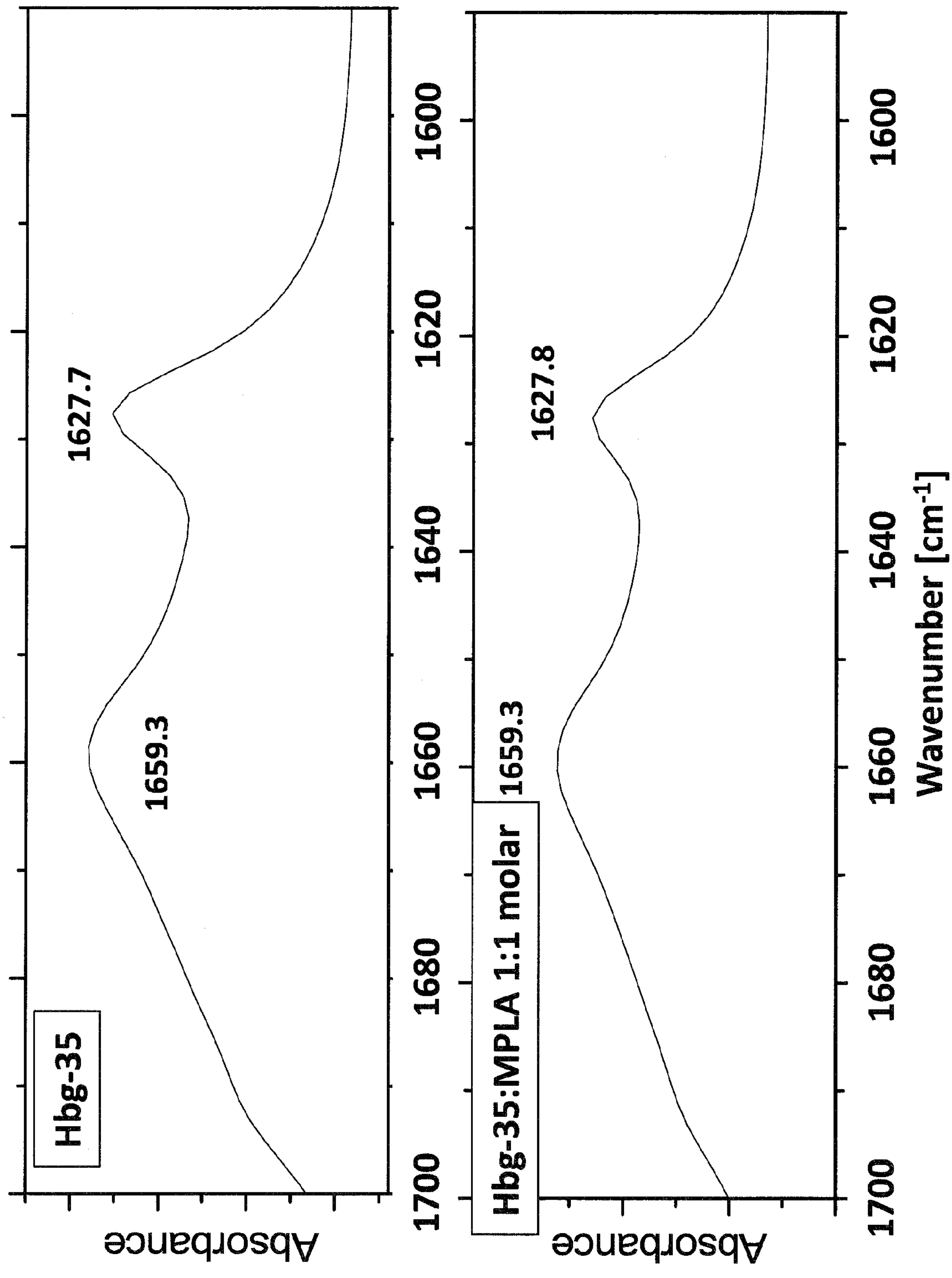
Hbg-35
1627.7
1659.3
Absorbance
Hbg-35:MPLA 1:1 molar
1627.8
1659.3
Absorbance
1700
1680
1660
1640
1620
1600
Wavenumber [cm-1]

Fig. 29

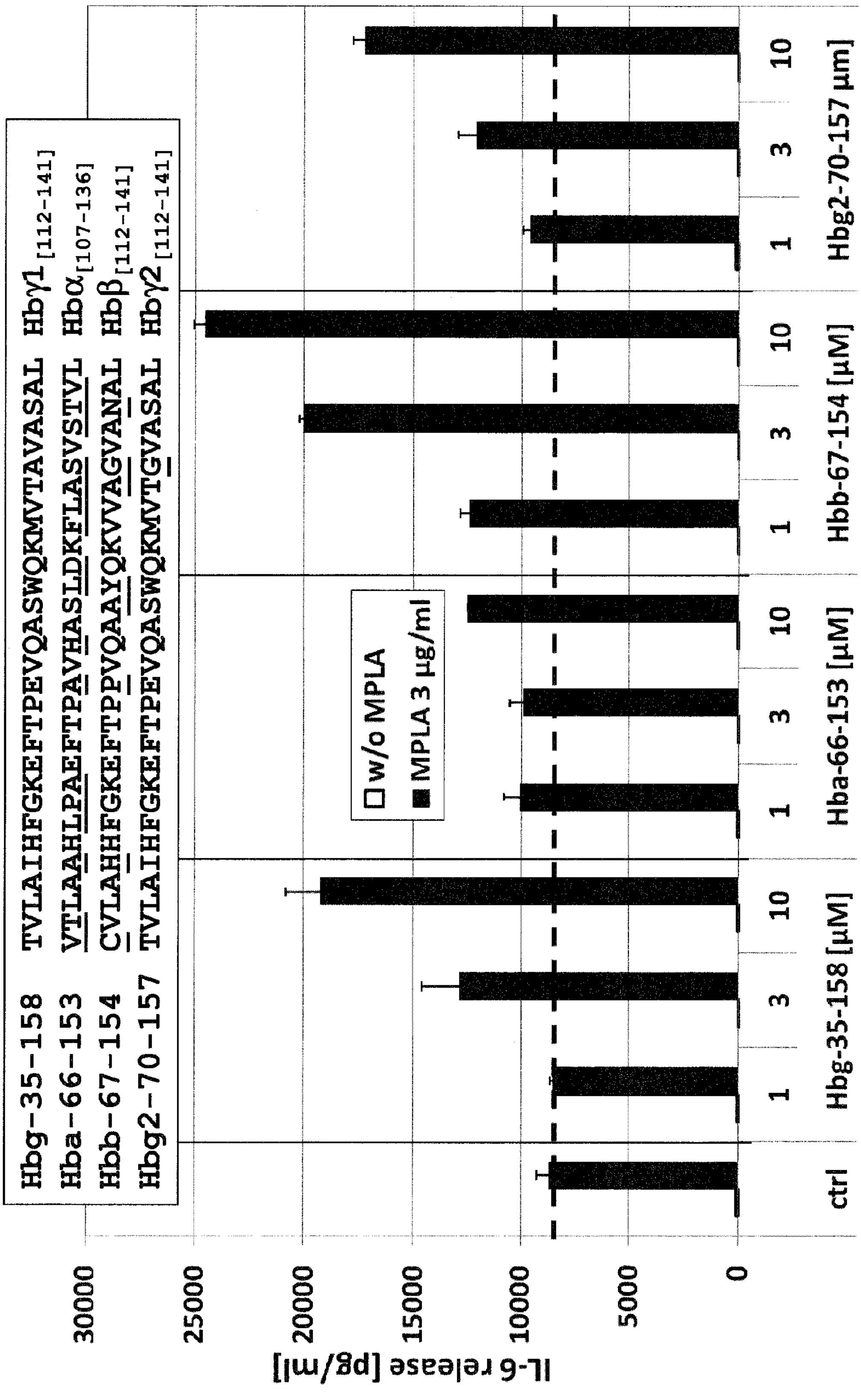
Hbg-35-158 TVLAIHFGKEFTPEVQASWQKMVTAVASAL Hbγ1 [112-141]
Hba-66-153 VTLAAHLPAEFTPAVHASLDKFLASVSTVL Hbα [107-136]
Hbb-67-154 CVLAHHFGKEFTPPVQAAYQKVVAGVANAL Hbβ [112-141]
Hbg2-70-157 TVLAIHFGKEFTPEVQASWQKMVTGVASAL Hbγ2 [112-141]
w/o MPLA
MPLA 3 µg/ml
IL-6 release [pg/ml]
30000
25000
20000
15000
10000
5000
0
ctrl
1
3
10
Hbg-35-158 [µM]
Hba-66-153 [µM]
Hbb-67-154 [µM]
Hbg2-70-157 µm]

Fig.30

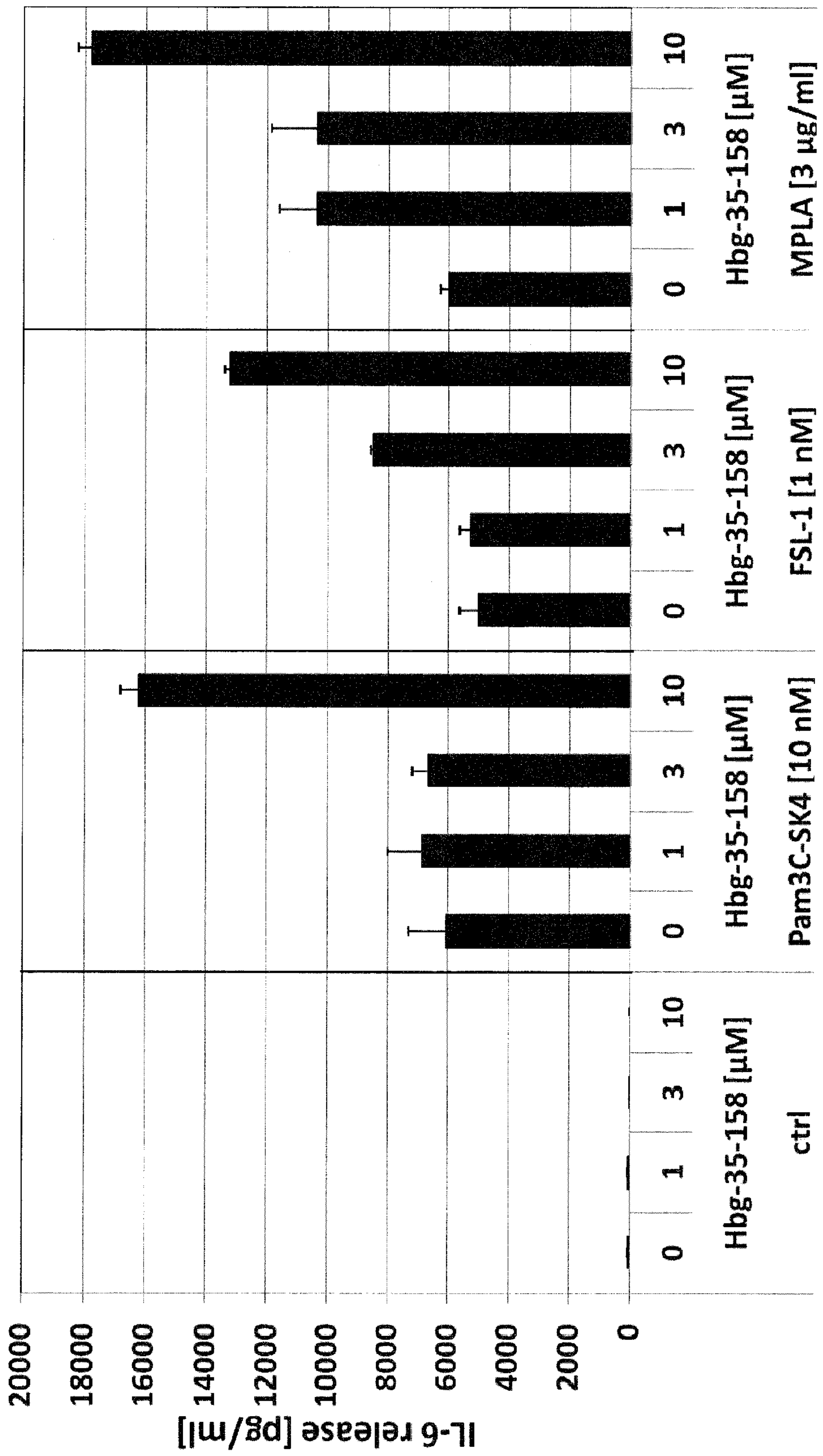
IL-6 release [pg/ml]
20000
18000
16000
14000
12000
10000
8000
6000
4000
2000
0
0 1 3 10
Hbg-35-158 [µM]
ctrl
0 1 3 10
Hbg-35-158 [µM]
Pam3C-SK4 [10 nM]
0 1 3 10
Hbg-35-158 [µM]
FSL-1 [1 nM]
0 1 3 10
Hbg-35-158 [µM]
MPLA [3 µg/ml]

Europäisches Patentamt
European Patent Office
Office européen des brevets

# EUROPEAN SEARCH REPORT

Application Number
EP 13 16 3414

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A,D | GORCZYNSKI R M ET AL: "Role of MIF and glutathione, in association with fetal ovine globin chain (Hbgamma) and LPS, in induction of TNFalpha from cells of young and aged mice, and PBL from healthy human populations", IMMUNOLOGY LETTERS, ELSEVIER BV, NL, vol. 105, no. 2, 15 June 2006 (2006-06-15), pages 140-149, XP024999183, ISSN: 0165-2478, DOI: 10.1016/J.IMLET.2006.02.001 [retrieved on 2006-06-15] * the whole document * ----- | 1-14 | INV. A61K38/42 A61K31/739 C07K14/805 A61P31/04 |
| A,D | WO 2004/073728 A2 (CLINIQUE LA PRAIRIE RES SA [LU]; WESTPHAL OTTO [CH]; WAELLI THIERRY [C) 2 September 2004 (2004-09-02) * the whole document * ----- | 1-14 | |
| A,D | HOWE JOERG ET AL: "Hemoglobin enhances the biological activity of synthetic and natural bacterial (endotoxic) virulence factors: a general principle", MEDICINAL CHEMISTRY, BENTHAM SCIENCE PUBLISHERS LTD, NL, vol. 4, no. 6, 1 November 2008 (2008-11-01), pages 520-525, XP009172062, ISSN: 1573-4064 * the whole document * ----- | 1-14 | TECHNICAL FIELDS SEARCHED (IPC) A61K C07K A61P |
| | -/-- | | |

1

The present search report has been drawn up for all claims

| Place of search | Date of completion of the search | Examiner |
|---|---|---|
| Munich | 27 August 2013 | Vandenbogaerde, Ann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 3414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIEPKE C ET AL: "Human hemoglobin-derived peptides exhibit antimicrobial activity: a class of host defense peptides", JOURNAL OF CHROMATOGRAPHY B: BIOMEDICAL SCIENCES & APPLICATIONS, ELSEVIER, AMSTERDAM, NL, vol. 791, no. 1-2, 5 July 2003 (2003-07-05), pages 345-356, XP004428059, ISSN: 1570-0232, DOI: 10.1016/S1570-0232(03)00245-9 * abstract * ----- | 1-14 | |

TECHNICAL FIELDS SEARCHED (IPC)

The present search report has been drawn up for all claims

1

| Place of search | Date of completion of the search | Examiner |
|---|---|---|
| Munich | 27 August 2013 | Vandenbogaerde, Ann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 3414

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2013

| Patent document cited in search report | Publication date | Patent family member(s) | Publication date |
|---|---|---|---|
| WO 2004073728 A2 | 02-09-2004 | AT 324903 T | 15-06-2006 |
| | | AU 2004212703 A1 | 02-09-2004 |
| | | BR PI0407311 A | 21-02-2006 |
| | | CA 2514485 A1 | 02-09-2004 |
| | | CN 1750840 A | 22-03-2006 |
| | | DE 60304989 T2 | 29-03-2007 |
| | | DK 1449535 T3 | 11-09-2006 |
| | | EP 1449535 A1 | 25-08-2004 |
| | | EP 1601371 A2 | 07-12-2005 |
| | | ES 2263859 T3 | 16-12-2006 |
| | | IL 169760 A | 24-09-2012 |
| | | IS 7940 A | 18-07-2005 |
| | | JP 4619352 B2 | 26-01-2011 |
| | | JP 2006518350 A | 10-08-2006 |
| | | KR 20050111596 A | 25-11-2005 |
| | | MX PA05008797 A | 25-05-2006 |
| | | NZ 541417 A | 29-08-2008 |
| | | PL 211789 B1 | 29-06-2012 |
| | | RU 2366449 C2 | 10-09-2009 |
| | | US 2008075742 A1 | 27-03-2008 |
| | | WO 2004073728 A2 | 02-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004073728 A2
- WO 2004073728 A


### Non-patent literature cited in the description


- **COLEY, W.B.** *Am. J. Med. Sci,* vol. 105, 487-511
- **BUWITT-BECKMANN et al.** *FEBS J.,* 2005
- **AKASHI, S. ; SHIMAZU, R. ; OGATA, H. ; NAGAI, Y. ; TAKEDA, K. ; KIMOTO, M. ; MIYAKE, K.** Cutting edge: cell surface expression and lipopolysaccharide signaling via the toll-like receptor 4-MD-2 complex on mouse peritoneal macrophages. *J.Immunol.,* 2000, vol. 164, 3471-3475
- **AKIRA, S.** Mammalian Toll-like receptors. *Curr.Opin.Immunol.,* 2003, vol. 15, 5-11
- **BAHL, N. ; DU, R. ; WINARSIH, I. ; HO, B. ; TUCKER-KELLOGG, L. ; TIDOR, B. ; DING, J. L.** Delineation of Lipopolysaccharide (LPS)-binding Sites on Hemoglobin: From in silico predictions to biophysical characterization. *J.Biol.Chem.,* 2011, vol. 286, 37793-37803
- **BRANDENBURG, K. ; GARIDEL, P. ; ANDRA, J. ; JURGENS, G. ; MULLER, M. ; BLUME, A. ; KOCH, M. H. ; LEVIN, J.** Cross-linked hemoglobin converts endotoxically inactive pentaacyl endotoxins into a physiologically active conformation. *J.Biol.Chem.,* 2003, vol. 278, 47660-47669
- **BUWITT-BECKMANN, U. ; HEINE, H. ; WIESMÜLLER, K.-H. ; JUNG, G. ; BROCK, R. ; ULMER, A.J.** Lipopeptide structure determines TLR2 dependent cell activation level. *FEBS J.,* 2005, vol. 272, 6354-6364
- **DA SILVA, C. J. ; SOLDAU, K. ; CHRISTEN, U. ; TOBIAS, P. S. ; ULEVITCH, R. J.** Lipopolysaccharide is in close proximity to each of the proteins in its membrane receptor complex. transfer from CD14 to TLR4 and MD-2. *J.Biol.Chem.,* 2001, vol. 276, 21129-21135
- **FOX, C. B. ; FRIEDE, M. ; REED, S. G. ; IRETON, G. C.** Synthetic and natural TLR4 agonists as safe and effective vaccine adjuvants. *Subcell.Biochem.,* 2010, vol. 53, 303-321
- **FRIER, J. A. ; PERUTZ, M. F.** Structure of human foetal deoxyhaemoglobin. *J.Mol.Biol.,* 1977, vol. 112, 97-112
- **GALANOS, C. ; LUDERITZ, O. ; RIETSCHEL, E. T. ; WESTPHAL, O. ; BRADE, H. ; BRADE, L. ; FREUDENBERG, M. ; SCHADE, U. ; IMOTO, M. ; YOSHIMURA, H.** Synthetic and natural Escherichia coli free lipid A express identical endotoxic activities. *Eur.J.Biochem.,* 1985, vol. 148, 1-5
- Tumor necrosis factor and host response to endotoxin. **GALANOS, C. ; FREUDENBERG, M. A. ; KATSCHINSKI T. ; SALOMAO, R. ; MOSSMANN, H. ; KUMAZAWA, Y.** Bacterial endotoxic lipopolysaccharides. II. Immunopharmacology and pathophysiology. CRC Press, Boca Raton, 1992, 75-104
- **GORCZYNSKI, R. M. ; ALEXANDER, C. ; BESSLER, W. ; BRANDENBURG, K. ; FOURNIER, K. ; HOFFMANN, P. ; MACH, J. P. ; MUELLER, S. ; RIETSCHEL, E. T. ; TERZIOGLU, E.** Role of MIF and glutathione, in association with fetal ovine globin chain (Hbgamma) and LPS, in induction of TNFalpha from cells of young and aged mice, and PBL from healthy human populations. *Immunol.Lett.,* 2006, vol. 105, 140-149
- **HAZIOT, A. ; CHEN, S. ; FERRERO, E. ; LOW, M. G. ; SILBER, R. ; GOYERT, S. M.** The monocyte differentiation antigen, CD14, is anchored to the cell membrane by a phosphatidylinositol linkage. *J.Immunol.,* 1988, vol. 141, 547-552
- **HOWE, J. ; HAMMER, M. ; ALEXANDER, C. ; ROSSLE, M. ; FOURNIER, K. ; MACH, J. P., WAELLI, T. ; GORCZYNSKI, R. M. ; ULMER, A. J. ; ZAHRINGER, U. ; RIETSCHEL, E. T.** Biophysical characterization of the interaction of endotoxins with hemoglobins. *Med.Chem.,* 2007, vol. 3, 13-20
- **HOWE, J. ; RICHTER, W. ; HAWKINS, L. ; ROSSLE, M. ; ALEXANDER, C. ; FOURNIER, K. ; MACH, J. P. ; WAELLI, T. ; GORCZYNSKI, R. M. ; ULMER, A. J.** Hemoglobin enhances the biological activity of synthetic and natural bacterial (endotoxic) virulence factors: a general principle. *Med.Chem.,* 2008, vol. 4, 520-525

- **HOWE, J. ; GARIDEL, P. ; ROESSLE, M. ; RICHTER, W. ; ALEXANDER, C. ; FOURNIER, K. ; MACH, J. P. ; WAELLI, T. ; GORCZYNSKI, R. M. ; ULMER, A. J.** Structural investigations into the interaction of hemoglobin and part structures with bacterial endotoxins. *Innate.Immun.,* 2008, vol. 14, 39-49
- **IMOTO, M. ; YOSHIMURA, H. ; SHIMAMOTO, S. ; SAKAGUCHI, N. ; KUSUMOTO, S. ; SHIBA, T.** Total synthesis of Escherichia coli lipid A, the endotoxically active principle of cellsurface lipopolysaccharide. *Bull. Chem. Soc.Jpn.,* 1987, vol. 60, 2205-2214
- **KACONIS, Y. ; KOWALSKI, I. ; HOWE, J. ; BRAUSER, A. ; RICHTER, W. ; RAZQUIN-OLAZARAN, I. ; INIGO-PESTANA, M. ; GARIDEL, P. ; ROSSLE, M. ; MARTINEZ DE, T. G.** Biophysical mechanisms of endotoxin neutralization by cationic amphiphilic peptides. *Biophys.J.,* 2011, vol. 100, 2652-2661
- **KARLSSON, S. ; NIENHUIS, A. W.** Developmental regulation of human globin genes. *Annu.Rev.Biochem.,* 1985, vol. 54, 1071-1108
- **LOPPNOW, H.** LPS, reclL1 and smooth muscle cell-IL1 activate vascular cells by specific mechanisms. *Prog.Clin.Biol.Res.,* 1994, vol. 388, 309-321
- **MAK, P. ; SIWEK, M. ; POHL, J. ; DUBIN, A.** Menstrual hemocidin HbB115-146 is an acidophilic antibacterial peptide potentiating the activity of human defensins, cathelicidin and lysozyme. *Am.J.Reprod.Immunol.,* 2007, vol. 57, 81-91
- **MATTERN, T. ; THANHAUSER, A. ; REILING, N. ; TOELLNER, K. M. ; DUCHROW, M. ; KUSUMOTO, S. ; RIETSCHEL, E. T. ; ERNST, M. ; BRADE, H. ; FLAD, H. D.** Endotoxin and lipid A stimulate proliferation of human T cells in the presence of autologous monocytes. *J.Immunol.,* 1994, vol. 153, 2996-3004
- **PARISH, C. A. ; JIANG, H. ; TOKIWA, Y. ; BEROVA, N. ; NAKANISHI, K. ; MCCABE, D. ; ZUCKERMAN, W. ; XIA, M. M. ; GABAY, J. E.** Broad-spectrum antimicrobial activity of hemoglobin. *Bioorg.Med.Chem.,* 2001, vol. 9, 377-382
- **PERUTZ, M. F.** Regulation of oxygen affinity of hemoglobin: influence of structure of the globin on the heme iron. *Annu.Rev.Biochem.,* 1979, vol. 48, 327-386
- **POLTORAK, A. ; HE, X. ; SMIRNOVA, I. ; LIU, M. Y. ; VAN, H. C. ; DU, X. ; BIRDWELL, D. ; ALEJOS, E. ; SILVA, M. ; GALANOS, C.** Defective LPS signaling in C3H/HeJ and C57BL/10ScCr mice: mutations in Tlr4 gene. *Science,* 1998, vol. 282, 2085-2088
- **PUGIN, J. ; SCHÜRER-MALY, C.-C. ; LETURCQ, D. ; MORIARTY, A. ; ULEVITCH, R. J. ; TOBIAS, P. S.** Lipopolysaccharide activation of human endothelial and epithelial cells is mediated by lipopolysaccharide-binding protein and soluble CD14. *Proc.Natl.Acad.Sci. USA,* 1993, vol. 90, 2744-2748
- **RIETSCHEL, E. T. ; BRADE, L. ; BRANDENBURG, K. ; FLAD, H. D. ; DE JONG-LEUVENINCK, J. ; KAWAHARA, K. ; LINDNER, B. ; LOPPNOW, H. ; LUDERITZ, T. ; SCHADE, U.** Chemical structure and biologic activity of bacterial and synthetic lipid A. *Rev.Infect.Dis.,* 1987, vol. 9 (5), S527-S536
- **RIETSCHEL, E. T. ; KIRIKAE, T. ; SCHADE, F. U. ; MAMAT, U. ; SCHMIDT, G. ; LOPPNOW, H. ; ULMER, A. J. ; ZAHRINGER, U. ; SEYDEL, U. ; DI, P. F.** Bacterial endotoxin: molecular relationships of structure to activity and function. *FASEB J.,* 1994, vol. 8, 217-225
- **SALUNKE, DB. ; SHUKLA, N.M. ; YOO, E. ; CRALL, B.M. ; BALAKRISHNA, R. ; MALLADI, S.S. ; DAVID, S.A.** Structure-activity relationships in human Toll-like receptor 2-specific monoacyl lipopeptides. *J Med Chem.,* 2012, vol. 55, 3353-3363
- **SEYDEL, U. ; OIKAWA, M. ; FUKASE, K. ; KUSUMOTO, S. ; BRANDENBURG, K.** Intrinsic conformation of lipid A is responsible for agonistic and antagonistic activity. *Eur.J.Biochem.,* 2000, vol. 267, 3032-3039
- **SHIMAZU, R. ; AKASHI, S. ; OGATA, H. ; NAGAI, Y. ; FUKUDOME, K. ; MIYAKE, K. ; KIMOTO, M.** MD-2, a molecule that confers lipopolysaccharide responsiveness on Toll-like receptor. *J.Exp.Med.,* 1999, vol. 189, 1777-1782
- **SUPAJATURA, V. ; USHIO, H. ; NAKAO, A. ; OKUMURA, K. ; RA, C. ; OGAWA, H.** Protective roles of mast cells against enterobacterial infection are mediated by Toll-like receptor. *J.Immunol.,* 2001, vol. 167, 2250-2256

SEQUENCE LISTING

<110> Clinique La Prairie

<120> Novel hemoglobin-derived peptide based pharmaceutical compositions

<130> S1160 EP

<160> 107

<170> PatentIn version 3.5

<210> 1
<211> 19
<212> PRT
<213> artificial sequence

<220>
<223> core sequence of Hbg-32 to Hbg-36, Hbg-39 and Hbg2-70

<400> 1

Thr Val Leu Ala Ile His Phe Gly Lys Glu Phe Thr Pro Glu Val Gln
1               5                   10                  15

Ala Ser Trp

<210> 2
<211> 19
<212> PRT
<213> artificial sequence

<220>
<223> core sequence of Hbb-67

<400> 2

Cys Val Leu Ala His His Phe Gly Lys Glu Phe Thr Pro Pro Val Gln
1               5                   10                  15

Ala Ala Tyr

<210> 3
<211> 30
<212> PRT
<213> artificial sequence

<220>
<223> hemoglobin-derived peptide

<400> 3

Thr Val Leu Ala Ile His Phe Gly Lys Glu Phe Thr Pro Glu Ala Gln
1               5                   10                  15

Ala Ser Trp Gln Lys Met Val Thr Ala Val Ala Ser Ala Leu
            20                  25                  30

<210> 4
<211> 6
<212> PRT
<213> artificial sequence

<220>
<223> partial sequence of Hbg-33

<400> 4

Leu Gly Asn Val Leu Val
1 5

<210> 5
<211> 16
<212> PRT
<213> artificial sequence

<220>
<223> partial sequence of Hbg-36

<400> 5

Leu His Val Asp Pro Glu Asn Phe Lys Leu Leu Gly Asn Val Leu Val
1 5 10 15

<210> 6
<211> 5
<212> PRT
<213> artificial sequence

<220>
<223> partial sequence of Hbg-33

<400> 6

Gln Lys Met Val Thr
1 5

<210> 7
<211> 5
<212> PRT
<213> artificial sequence

<220>
<223> partial sequence of Hbb-67

<400> 7

Gln Lys Val Val Ala
1 5

<210> 8
<211> 11
<212> PRT
<213> artificial sequence

<220>
<223> partial sequence of Hbg-35

<400> 8

Gln Lys Met Val Thr Ala Val Ala Ser Ala Leu
1 5 10

<210> 9
<211> 11
<212> PRT
<213> artificial sequence

<220>
<223> partial sequence of Hbg-32

<400> 9

Gln Lys Met Val Thr Ala Val Ala Ser Ala Gln
1 5 10

<210> 10
<211> 11
<212> PRT
<213> artificial sequence

<220>
<223> partial sequence of Hbg2-70

<400> 10

Gln Lys Met Val Thr Gly Val Ala Ser Ala Leu
1 5 10

<210> 11
<211> 11
<212> PRT
<213> artificial sequence

<220>
<223> partial sequence of Hbb-67

<400> 11

Gln Lys Val Val Ala Gly Val Ala Asn Ala Leu
1 5 10

<210> 12
<211> 16
<212> PRT
<213> artificial sequence

<220>
<223> partial sequence of Hbg-39

<400> 12

Gln Lys Met Val Thr Ala Val Ala Ser Ala Leu Ser Ser Arg Tyr His
1 5 10 15

<210> 13
<211> 30

<212> PRT
<213> artificial sequence

<220>
<223> Hbg-35

<400> 13

Thr Val Leu Ala Ile His Phe Gly Lys Glu Phe Thr Pro Glu Val Gln
1 5 10 15

Ala Ser Trp Gln Lys Met Val Thr Ala Val Ala Ser Ala Leu
20 25 30

<210> 14
<211> 20
<212> PRT
<213> artificial sequence

<220>
<223> Hbg-34

<400> 14

Val Thr Val Leu Ala Ile His Phe Gly Lys Glu Phe Thr Pro Glu Val
1 5 10 15

Gln Ala Ser Trp
20

<210> 15
<211> 36
<212> PRT
<213> artificial sequence

<220>
<223> Hbg-39

<400> 15

Val Thr Val Leu Ala Ile His Phe Gly Lys Glu Phe Thr Pro Glu Val
1 5 10 15

Gln Ala Ser Trp Gln Lys Met Val Thr Ala Val Ala Ser Ala Leu Ser
20 25 30

Ser Arg Tyr His
35

<210> 16
<211> 30
<212> PRT
<213> artificial sequence

<220>
<223> Hbg-32

<400> 16

```
Thr Val Leu Ala Ile His Phe Gly Lys Glu Phe Thr Pro Glu Val Gln
1               5                   10                  15

Ala Ser Trp Gln Lys Met Val Thr Ala Val Ala Ser Ala Gln
            20                  25                  30


<210>  17
<211>  30
<212>  PRT
<213>  artificial sequence

<220>
<223>  Hbg-33

<400>  17

Leu Gly Asn Val Leu Val Thr Val Leu Ala Ile His Phe Gly Lys Glu
1               5                   10                  15

Phe Thr Pro Glu Val Gln Ala Ser Trp Gln Lys Met Val Thr
            20                  25                  30


<210>  18
<211>  35
<212>  PRT
<213>  artificial sequence

<220>
<223>  Hbg-36

<400>  18

Leu His Val Asp Pro Glu Asn Phe Lys Leu Leu Gly Asn Val Leu Val
1               5                   10                  15

Thr Val Leu Ala Ile His Phe Gly Lys Glu Phe Thr Pro Glu Val Gln
            20                  25                  30

Ala Ser Trp
        35


<210>  19
<211>  30
<212>  PRT
<213>  artificial sequence

<220>
<223>  Hbg2-70

<400>  19

Thr Val Leu Ala Ile His Phe Gly Lys Glu Phe Thr Pro Glu Val Gln
1               5                   10                  15

Ala Ser Trp Gln Lys Met Val Thr Gly Val Ala Ser Ala Leu
            20                  25                  30
```

<210> 20
<211> 30
<212> PRT
<213> artificial sequence

<220>
<223> Hbb-67

<400> 20

```
Cys Val Leu Ala His His Phe Gly Lys Glu Phe Thr Pro Pro Val Gln
1               5                   10                  15

Ala Ala Tyr Gln Lys Val Val Ala Gly Val Ala Asn Ala Leu
            20                  25                  30
```

<210> 21
<211> 30
<212> PRT
<213> artificial sequence

<220>
<223> Hba-66

<400> 21

```
Val Thr Leu Ala Ala His Leu Pro Ala Glu Phe Thr Pro Ala Val His
1               5                   10                  15

Ala Ser Leu Asp Lys Phe Leu Ala Ser Val Ser Thr Val Leu
            20                  25                  30
```

<210> 22
<211> 147
<212> PRT
<213> homo sapiens

<400> 22

```
Met Gly His Phe Thr Glu Glu Asp Lys Ala Thr Ile Thr Ser Leu Trp
1               5                   10                  15

Gly Lys Val Asn Val Glu Asp Ala Gly Gly Glu Thr Leu Gly Arg Leu
            20                  25                  30

Leu Val Val Tyr Pro Trp Thr Gln Arg Phe Phe Asp Ser Phe Gly Asn
        35                  40                  45

Leu Ser Ser Ala Ser Ala Ile Met Gly Asn Pro Lys Val Lys Ala His
    50                  55                  60

Gly Lys Lys Val Leu Thr Ser Leu Gly Asp Ala Ile Lys His Leu Asp
65                  70                  75                  80
```

```
Asp Leu Lys Gly Thr Phe Ala Gln Leu Ser Glu Leu His Cys Asp Lys
                85                  90                  95

Leu His Val Asp Pro Glu Asn Phe Lys Leu Leu Gly Asn Val Leu Val
            100                 105                 110

Thr Val Leu Ala Ile His Phe Gly Lys Glu Phe Thr Pro Glu Val Gln
        115                 120                 125

Ala Ser Trp Gln Lys Met Val Thr Ala Val Ala Ser Ala Leu Ser Ser
    130                 135                 140

Arg Tyr His
145


<210>  23
<211>  21
<212>  PRT
<213>  artificial sequence

<220>
<223>  Hbg-1-K5

<400>  23

Lys Lys Lys Lys Lys Met Gly His Phe Thr Glu Glu Asp Lys Ala Thr
1               5                   10                  15

Ile Thr Ser Leu Trp
            20


<210>  24
<211>  20
<212>  PRT
<213>  artificial sequence

<220>
<223>  Hbg-2-K5

<400>  24

Lys Lys Lys Lys Lys Glu Asp Lys Ala Thr Ile Thr Ser Leu Trp Gly
1               5                   10                  15

Lys Val Asn Val
            20


<210>  25
<211>  20
<212>  PRT
<213>  artificial sequence

<220>
<223>  Hbg-3-K5

<400>  25
```

```
Lys Lys Lys Lys Lys Ile Thr Ser Leu Trp Gly Lys Val Asn Val Glu
1               5                   10                  15


Asp Ala Gly Gly
            20


<210>  26
<211>  20
<212>  PRT
<213>  artificial sequence

<220>
<223>  Hbg-4-K5

<400>  26

Lys Lys Lys Lys Lys Gly Lys Val Asn Val Glu Asp Ala Gly Gly Glu
1               5                   10                  15


Thr Leu Gly Arg
            20


<210>  27
<211>  20
<212>  PRT
<213>  artificial sequence

<220>
<223>  Hbg-5-K5

<400>  27

Lys Lys Lys Lys Lys Glu Asp Ala Gly Gly Glu Thr Leu Gly Arg Leu
1               5                   10                  15


Leu Val Val Tyr
            20


<210>  28
<211>  20
<212>  PRT
<213>  artificial sequence

<220>
<223>  Hbg-6-K5

<400>  28

Lys Lys Lys Lys Lys Glu Thr Leu Gly Arg Leu Leu Val Val Tyr Pro
1               5                   10                  15


Trp Thr Gln Arg
            20


<210>  29
<211>  20
```

<212> PRT
<213> artificial sequence

<220>
<223> Hbg-7-K5

<400> 29

Lys Lys Lys Lys Lys Leu Leu Val Val Tyr Pro Trp Thr Gln Arg Phe
1 5 10 15

Phe Asp Ser Phe
20

<210> 30
<211> 20
<212> PRT
<213> artificial sequence

<220>
<223> Hbg-8-K5

<400> 30

Lys Lys Lys Lys Lys Pro Trp Thr Gln Arg Phe Phe Asp Ser Phe Gly
1 5 10 15

Asn Leu Ser Ser
20

<210> 31
<211> 20
<212> PRT
<213> artificial sequence

<220>
<223> Hbg-9-K5

<400> 31

Lys Lys Lys Lys Lys Phe Phe Asp Ser Phe Gly Asn Leu Ser Ser Ala
1 5 10 15

Ser Ala Ile Met
20

<210> 32
<211> 20
<212> PRT
<213> artificial sequence

<220>
<223> Hbg-10-K5

<400> 32

Lys Lys Lys Lys Lys Gly Asn Leu Ser Ser Ala Ser Ala Ile Met Gly
1 5 10 15

Asn Pro Lys Val
20

<210> 33
<211> 20
<212> PRT
<213> artificial sequence

<220>
<223> Hbg-11-K5

<400> 33

Lys Lys Lys Lys Lys Ala Ser Ala Ile Met Gly Asn Pro Lys Val Lys
1 5 10 15

Ala His Gly Lys
20

<210> 34
<211> 20
<212> PRT
<213> artificial sequence

<220>
<223> Hbg-12-K5

<400> 34

Lys Lys Lys Lys Lys Gly Asn Pro Lys Val Lys Ala His Gly Lys Lys
1 5 10 15

Val Leu Thr Ser
20

<210> 35
<211> 20
<212> PRT
<213> artificial sequence

<220>
<223> Hbg-13-K5

<400> 35

Lys Lys Lys Lys Lys Lys Ala His Gly Lys Lys Val Leu Thr Ser Leu
1 5 10 15

Gly Asp Ala Ile
20

<210> 36
<211> 20
<212> PRT
<213> artificial sequence

<220>

<223> Hbg-14-K5

<400> 36

Lys Lys Lys Lys Lys Lys Val Leu Thr Ser Leu Gly Asp Ala Ile Lys
1               5                   10                  15

His Leu Asp Asp
            20

<210> 37
<211> 20
<212> PRT
<213> artificial sequence

<220>
<223> Hbg-15-K5

<400> 37

Lys Lys Lys Lys Lys Leu Gly Asp Ala Ile Lys His Leu Asp Asp Leu
1               5                   10                  15

Lys Gly Thr Phe
            20

<210> 38
<211> 20
<212> PRT
<213> artificial sequence

<220>
<223> Hbg-16-K5

<400> 38

Lys Lys Lys Lys Lys Lys His Leu Asp Asp Leu Lys Gly Thr Phe Ala
1               5                   10                  15

Gln Leu Ser Glu
            20

<210> 39
<211> 20
<212> PRT
<213> artificial sequence

<220>
<223> Hbg-17-K5

<400> 39

Lys Lys Lys Lys Lys Leu Lys Gly Thr Phe Ala Gln Leu Ser Glu Leu
1               5                   10                  15

His Cys Asp Lys
            20

<210> 40
<211> 20
<212> PRT
<213> artificial sequence

<220>
<223> Hbg-18-K5

<400> 40

Lys Lys Lys Lys Lys Ala Gln Leu Ser Glu Leu His Cys Asp Lys Leu
1               5                   10                  15

His Val Asp Pro
            20

<210> 41
<211> 20
<212> PRT
<213> artificial sequence

<220>
<223> Hbg-19-K5

<400> 41

Lys Lys Lys Lys Lys Leu His Cys Asp Lys Leu His Val Asp Pro Glu
1               5                   10                  15

Asn Phe Lys Leu
            20

<210> 42
<211> 20
<212> PRT
<213> artificial sequence

<220>
<223> Hbg-20-K5

<400> 42

Lys Lys Lys Lys Lys Leu His Val Asp Pro Glu Asn Phe Lys Leu Leu
1               5                   10                  15

Gly Asn Val Leu
            20

<210> 43
<211> 20
<212> PRT
<213> artificial sequence

<220>
<223> Hbg-21-K5

<400> 43

Lys Lys Lys Lys Lys Glu Asn Phe Lys Leu Leu Gly Asn Val Leu Val
1               5                   10                  15

Thr Val Leu Ala
            20

<210> 44
<211> 20
<212> PRT
<213> artificial sequence

<220>
<223> Hbg-22-K5

<400> 44

Lys Lys Lys Lys Lys Leu Gly Asn Val Leu Val Thr Val Leu Ala Ile
1               5                   10                  15

His Phe Gly Lys
            20

<210> 45
<211> 20
<212> PRT
<213> artificial sequence

<220>
<223> Hbg-23-K5

<400> 45

Lys Lys Lys Lys Lys Val Thr Val Leu Ala Ile His Phe Gly Lys Glu
1               5                   10                  15

Phe Thr Pro Glu
            20

<210> 46
<211> 20
<212> PRT
<213> artificial sequence

<220>
<223> Hbg-24-K5

<400> 46

Lys Lys Lys Lys Lys Ile His Phe Gly Lys Glu Phe Thr Pro Glu Val
1               5                   10                  15

Gln Ala Ser Trp
            20

<210> 47
<211> 20
<212> PRT

<213> artificial sequence

<220>
<223> Hbg-25-K5

<400> 47

Lys Lys Lys Lys Lys Glu Phe Thr Pro Glu Val Gln Ala Ser Trp Gln
1               5                   10                  15

Lys Met Val Thr
            20

<210> 48
<211> 20
<212> PRT
<213> artificial sequence

<220>
<223> Hbg-26-K5

<400> 48

Lys Lys Lys Lys Lys Val Gln Ala Ser Trp Gln Lys Met Val Thr Ala
1               5                   10                  15

Val Ala Ser Ala
            20

<210> 49
<211> 21
<212> PRT
<213> artificial sequence

<220>
<223> Hbg-27-K5

<400> 49

Lys Lys Lys Lys Lys Gln Lys Met Val Thr Ala Val Ala Ser Ala Leu
1               5                   10                  15

Ser Ser Arg Tyr His
            20

<210> 50
<211> 16
<212> PRT
<213> artificial sequence

<220>
<223> Hbg-1

<400> 50

Met Gly His Phe Thr Glu Glu Asp Lys Ala Thr Ile Thr Ser Leu Trp
1               5                   10                  15

<210> 51
<211> 15
<212> PRT
<213> artificial sequence

<220>
<223> Hbg-2

<400> 51

Glu Asp Lys Ala Thr Ile Thr Ser Leu Trp Gly Lys Val Asn Val
1               5                   10                  15

<210> 52
<211> 15
<212> PRT
<213> artificial sequence

<220>
<223> Hbg-3

<400> 52

Ile Thr Ser Leu Trp Gly Lys Val Asn Val Glu Asp Ala Gly Gly
1               5                   10                  15

<210> 53
<211> 15
<212> PRT
<213> artificial sequence

<220>
<223> Hbg-4

<400> 53

Gly Lys Val Asn Val Glu Asp Ala Gly Gly Glu Thr Leu Gly Arg
1               5                   10                  15

<210> 54
<211> 15
<212> PRT
<213> artificial sequence

<220>
<223> Hbg-5

<400> 54

Glu Asp Ala Gly Gly Glu Thr Leu Gly Arg Leu Leu Val Val Tyr
1               5                   10                  15

<210> 55
<211> 15
<212> PRT
<213> artificial sequence

<220>
<223> Hbg-6

<400> 55

```
Glu Thr Leu Gly Arg Leu Leu Val Val Tyr Pro Trp Thr Gln Arg
1               5                   10                  15


<210>  56
<211>  15
<212>  PRT
<213>  artificial sequence

<220>
<223>  Hbg-7

<400>  56

Leu Leu Val Val Tyr Pro Trp Thr Gln Arg Phe Phe Asp Ser Phe
1               5                   10                  15


<210>  57
<211>  15
<212>  PRT
<213>  artificial sequence

<220>
<223>  Hbg-8

<400>  57

Pro Trp Thr Gln Arg Phe Phe Asp Ser Phe Gly Asn Leu Ser Ser
1               5                   10                  15


<210>  58
<211>  15
<212>  PRT
<213>  artificial sequence

<220>
<223>  Hbg-9

<400>  58

Phe Phe Asp Ser Phe Gly Asn Leu Ser Ser Ala Ser Ala Ile Met
1               5                   10                  15


<210>  59
<211>  15
<212>  PRT
<213>  artificial sequence

<220>
<223>  Hbg-10

<400>  59

Gly Asn Leu Ser Ser Ala Ser Ala Ile Met Gly Asn Pro Lys Val
1               5                   10                  15


<210>  60
<211>  15
<212>  PRT
<213>  artificial sequence
```

<220>
<223> Hbg-11

<400> 60

Ala Ser Ala Ile Met Gly Asn Pro Lys Val Lys Ala His Gly Lys
1               5                   10                  15

<210> 61
<211> 15
<212> PRT
<213> artificial sequence

<220>
<223> Hbg-12

<400> 61

Gly Asn Pro Lys Val Lys Ala His Gly Lys Lys Val Leu Thr Ser
1               5                   10                  15

<210> 62
<211> 15
<212> PRT
<213> artificial sequence

<220>
<223> Hbg-13

<400> 62

Lys Ala His Gly Lys Lys Val Leu Thr Ser Leu Gly Asp Ala Ile
1               5                   10                  15

<210> 63
<211> 15
<212> PRT
<213> artificial sequence

<220>
<223> Hbg-14

<400> 63

Lys Val Leu Thr Ser Leu Gly Asp Ala Ile Lys His Leu Asp Asp
1               5                   10                  15

<210> 64
<211> 15
<212> PRT
<213> artificial sequence

<220>
<223> Hbg-15

<400> 64

Leu Gly Asp Ala Ile Lys His Leu Asp Asp Leu Lys Gly Thr Phe
1               5                   10                  15

<210> 65
<211> 15
<212> PRT
<213> artificial sequence

<220>
<223> Hbg-16

<400> 65

Lys His Leu Asp Asp Leu Lys Gly Thr Phe Ala Gln Leu Ser Glu
1 5 10 15

<210> 66
<211> 15
<212> PRT
<213> artificial sequence

<220>
<223> Hbg-17

<400> 66

Leu Lys Gly Thr Phe Ala Gln Leu Ser Glu Leu His Cys Asp Lys
1 5 10 15

<210> 67
<211> 15
<212> PRT
<213> artificial sequence

<220>
<223> Hbg-18

<400> 67

Ala Gln Leu Ser Glu Leu His Cys Asp Lys Leu His Val Asp Pro
1 5 10 15

<210> 68
<211> 15
<212> PRT
<213> artificial sequence

<220>
<223> Hbg-19

<400> 68

Leu His Cys Asp Lys Leu His Val Asp Pro Glu Asn Phe Lys Leu
1 5 10 15

<210> 69
<211> 15
<212> PRT
<213> artificial sequence

<220>
<223> Hbg-20

<400> 69

Leu His Val Asp Pro Glu Asn Phe Lys Leu Leu Gly Asn Val Leu
1 5 10 15

<210> 70
<211> 15
<212> PRT
<213> artificial sequence

<220>
<223> Hbg-21

<400> 70

Glu Asn Phe Lys Leu Leu Gly Asn Val Leu Val Thr Val Leu Ala
1 5 10 15

<210> 71
<211> 15
<212> PRT
<213> artificial sequence

<220>
<223> Hbg-22

<400> 71

Leu Gly Asn Val Leu Val Thr Val Leu Ala Ile His Phe Gly Lys
1 5 10 15

<210> 72
<211> 15
<212> PRT
<213> artificial sequence

<220>
<223> Hbg-23

<400> 72

Val Thr Val Leu Ala Ile His Phe Gly Lys Glu Phe Thr Pro Glu
1 5 10 15

<210> 73
<211> 15
<212> PRT
<213> artificial sequence

<220>
<223> Hbg-24

<400> 73

Ile His Phe Gly Lys Glu Phe Thr Pro Glu Val Gln Ala Ser Trp
1 5 10 15

<210> 74
<211> 15
<212> PRT

<213> artificial sequence

<220>
<223> Hbg-25

<400> 74

Glu Phe Thr Pro Glu Val Gln Ala Ser Trp Gln Lys Met Val Thr
1 5 10 15

<210> 75
<211> 15
<212> PRT
<213> artificial sequence

<220>
<223> Hbg-26

<400> 75

Val Gln Ala Ser Trp Gln Lys Met Val Thr Ala Val Ala Ser Ala
1 5 10 15

<210> 76
<211> 16
<212> PRT
<213> artificial sequence

<220>
<223> Hbg-27

<400> 76

Gln Lys Met Val Thr Ala Val Ala Ser Ala Leu Ser Ser Arg Tyr His
1 5 10 15

<210> 77
<211> 30
<212> PRT
<213> artificial sequence

<220>
<223> Hbg-30

<400> 77

Ala Gln Leu Ser Glu Leu His Cys Asp Lys Leu His Val Asp Pro Glu
1 5 10 15

Asn Phe Lys Leu Leu Gly Asn Val Leu Val Thr Val Leu Ala
20 25 30

<210> 78
<211> 30
<212> PRT
<213> artificial sequence

<220>
<223> Hbg-31

<400> 78

Asp Pro Glu Asn Phe Lys Leu Leu Gly Asn Val Leu Val Thr Val Leu
1 5 10 15

Ala Ile His Phe Gly Lys Glu Phe Thr Pro Glu Val Gln Ala
20 25 30

<210> 79
<211> 30
<212> PRT
<213> artificial sequence

<220>
<223> Hbg-32

<400> 79

Thr Val Leu Ala Ile His Phe Gly Lys Glu Phe Thr Pro Glu Val Gln
1 5 10 15

Ala Ser Trp Gln Lys Met Val Thr Ala Val Ala Ser Ala Gln
20 25 30

<210> 80
<211> 44
<212> PRT
<213> artificial sequence

<220>
<223> Hbg-38

<400> 80

Phe Lys Leu Leu Gly Asn Val Leu Val Thr Val Leu Ala Ile His Phe
1 5 10 15

Gly Lys Glu Phe Thr Pro Glu Val Gln Ala Ser Trp Gln Lys Met Val
20 25 30

Thr Ala Val Ala Ser Ala Leu Ser Ser Arg Tyr His
35 40

<210> 81
<211> 30
<212> PRT
<213> artificial sequence

<220>
<223> Hbg-42

<400> 81

Ala Val Leu Ala Ile His Phe Gly Lys Glu Phe Thr Pro Glu Val Gln
1 5 10 15

Ala Ser Trp Gln Lys Met Val Thr Ala Val Ala Ser Ala Leu

20 25 30

<210> 82
<211> 30
<212> PRT
<213> artificial sequence

<220>
<223> Hbg-43

<400> 82

Thr Ala Leu Ala Ile His Phe Gly Lys Glu Phe Thr Pro Glu Val Gln
1 5 10 15

Ala Ser Trp Gln Lys Met Val Thr Ala Val Ala Ser Ala Leu
20 25 30

<210> 83
<211> 30
<212> PRT
<213> artificial sequence

<220>
<223> Hbg-44

<400> 83

Thr Val Ala Ala Ile His Phe Gly Lys Glu Phe Thr Pro Glu Val Gln
1 5 10 15

Ala Ser Trp Gln Lys Met Val Thr Ala Val Ala Ser Ala Leu
20 25 30

<210> 84
<211> 30
<212> PRT
<213> artificial sequence

<220>
<223> Hbg-45

<400> 84

Thr Val Leu Ala Ala His Phe Gly Lys Glu Phe Thr Pro Glu Val Gln
1 5 10 15

Ala Ser Trp Gln Lys Met Val Thr Ala Val Ala Ser Ala Leu
20 25 30

<210> 85
<211> 30
<212> PRT
<213> artificial sequence

<220>
<223> Hbg-46

<400> 85

Thr Val Leu Ala Ile Ala Phe Gly Lys Glu Phe Thr Pro Glu Val Gln
1               5                   10                  15

Ala Ser Trp Gln Lys Met Val Thr Ala Val Ala Ser Ala Leu
            20                  25                  30

<210> 86
<211> 30
<212> PRT
<213> artificial sequence

<220>
<223> Hbg-47

<400> 86

Thr Val Leu Ala Ile His Ala Gly Lys Glu Phe Thr Pro Glu Val Gln
1               5                   10                  15

Ala Ser Trp Gln Lys Met Val Thr Ala Val Ala Ser Ala Leu
            20                  25                  30

<210> 87
<211> 30
<212> PRT
<213> artificial sequence

<220>
<223> Hbg-48

<400> 87

Thr Val Leu Ala Ile His Phe Ala Lys Glu Phe Thr Pro Glu Val Gln
1               5                   10                  15

Ala Ser Trp Gln Lys Met Val Thr Ala Val Ala Ser Ala Leu
            20                  25                  30

<210> 88
<211> 30
<212> PRT
<213> artificial sequence

<220>
<223> Hbg-49

<400> 88

Thr Val Leu Ala Ile His Phe Gly Ala Glu Phe Thr Pro Glu Val Gln
1               5                   10                  15

Ala Ser Trp Gln Lys Met Val Thr Ala Val Ala Ser Ala Leu
            20                  25                  30

<210> 89

<211> 30
<212> PRT
<213> artificial sequence

<220>
<223> Hbg-50

<400> 89

Thr Val Leu Ala Ile His Phe Gly Lys Ala Phe Thr Pro Glu Val Gln
1 5 10 15

Ala Ser Trp Gln Lys Met Val Thr Ala Val Ala Ser Ala Leu
20 25 30

<210> 90
<211> 30
<212> PRT
<213> artificial sequence

<220>
<223> Hbg-51

<400> 90

Thr Val Leu Ala Ile His Phe Gly Lys Glu Ala Thr Pro Glu Val Gln
1 5 10 15

Ala Ser Trp Gln Lys Met Val Thr Ala Val Ala Ser Ala Leu
20 25 30

<210> 91
<211> 30
<212> PRT
<213> artificial sequence

<220>
<223> Hbg-52

<400> 91

Thr Val Leu Ala Ile His Phe Gly Lys Glu Phe Ala Pro Glu Val Gln
1 5 10 15

Ala Ser Trp Gln Lys Met Val Thr Ala Val Ala Ser Ala Leu
20 25 30

<210> 92
<211> 30
<212> PRT
<213> artificial sequence

<220>
<223> Hbg-53

<400> 92

Thr Val Leu Ala Ile His Phe Gly Lys Glu Phe Thr Ala Glu Val Gln
1 5 10 15

```
Ala Ser Trp Gln Lys Met Val Thr Ala Val Ala Ser Ala Leu
            20                  25                  30


<210>  93
<211>  30
<212>  PRT
<213>  artificial sequence

<220>
<223>  Hbg-54

<400>  93

Thr Val Leu Ala Ile His Phe Gly Lys Glu Phe Thr Pro Ala Val Gln
1               5                   10                  15


Ala Ser Trp Gln Lys Met Val Thr Ala Val Ala Ser Ala Leu
            20                  25                  30


<210>  94
<211>  30
<212>  PRT
<213>  artificial sequence

<220>
<223>  Hbg-55

<400>  94

Thr Val Leu Ala Ile His Phe Gly Lys Glu Phe Thr Pro Glu Ala Gln
1               5                   10                  15


Ala Ser Trp Gln Lys Met Val Thr Ala Val Ala Ser Ala Leu
            20                  25                  30


<210>  95
<211>  30
<212>  PRT
<213>  artificial sequence

<220>
<223>  Hbg-56

<400>  95

Thr Val Leu Ala Ile His Phe Gly Lys Glu Phe Thr Pro Glu Val Ala
1               5                   10                  15


Ala Ser Trp Gln Lys Met Val Thr Ala Val Ala Ser Ala Leu
            20                  25                  30


<210>  96
<211>  30
<212>  PRT
<213>  artificial sequence
```

<220>
<223> Hbg-57

<400> 96

Thr Val Leu Ala Ile His Phe Gly Lys Glu Phe Thr Pro Glu Val Gln
1 5 10 15

Ala Ala Trp Gln Lys Met Val Thr Ala Val Ala Ser Ala Leu
20 25 30

<210> 97
<211> 30
<212> PRT
<213> artificial sequence

<220>
<223> Hbg-58

<400> 97

Thr Val Leu Ala Ile His Phe Gly Lys Glu Phe Thr Pro Glu Val Gln
1 5 10 15

Ala Ser Ala Gln Lys Met Val Thr Ala Val Ala Ser Ala Leu
20 25 30

<210> 98
<211> 30
<212> PRT
<213> artificial sequence

<220>
<223> Hbg-59

<400> 98

Thr Val Leu Ala Ile His Phe Gly Lys Glu Phe Thr Pro Glu Val Gln
1 5 10 15

Ala Ser Trp Ala Lys Met Val Thr Ala Val Ala Ser Ala Leu
20 25 30

<210> 99
<211> 30
<212> PRT
<213> artificial sequence

<220>
<223> Hbg-60

<400> 99

Thr Val Leu Ala Ile His Phe Gly Lys Glu Phe Thr Pro Glu Val Gln
1 5 10 15

Ala Ser Trp Gln Ala Met Val Thr Ala Val Ala Ser Ala Leu
20 25 30

<210> 100
<211> 30
<212> PRT
<213> artificial sequence

<220>
<223> Hbg-61

<400> 100

Thr Val Leu Ala Ile His Phe Gly Lys Glu Phe Thr Pro Glu Val Gln
1 5 10 15

Ala Ser Trp Gln Lys Ala Val Thr Ala Val Ala Ser Ala Leu
20 25 30

<210> 101
<211> 30
<212> PRT
<213> artificial sequence

<220>
<223> Hbg-62

<400> 101

Thr Val Leu Ala Ile His Phe Gly Lys Glu Phe Thr Pro Glu Val Gln
1 5 10 15

Ala Ser Trp Gln Lys Met Ala Thr Ala Val Ala Ser Ala Leu
20 25 30

<210> 102
<211> 30
<212> PRT
<213> artificial sequence

<220>
<223> Hbg-63

<400> 102

Thr Val Leu Ala Ile His Phe Gly Lys Glu Phe Thr Pro Glu Val Gln
1 5 10 15

Ala Ser Trp Gln Lys Met Val Ala Ala Val Ala Ser Ala Leu
20 25 30

<210> 103
<211> 30
<212> PRT
<213> artificial sequence

<220>
<223> Hbg-64

<400> 103

Thr Val Leu Ala Ile His Phe Gly Lys Glu Phe Thr Pro Glu Val Gln
1               5                   10                  15

Ala Ser Trp Gln Lys Met Val Thr Ala Ala Ala Ser Ala Leu
            20                  25                  30

<210>  104
<211>  30
<212>  PRT
<213>  artificial sequence

<220>
<223>  Hbg-65

<400>  104

Thr Val Leu Ala Ile His Phe Gly Lys Glu Phe Thr Pro Glu Val Gln
1               5                   10                  15

Ala Ser Trp Gln Lys Met Val Thr Ala Val Ala Ala Ala Leu
            20                  25                  30

<210>  105
<211>  30
<212>  PRT
<213>  artificial sequence

<220>
<223>  Hbg-66

<400>  105

Thr Val Leu Ala Ile His Phe Gly Lys Glu Phe Thr Pro Glu Val Gln
1               5                   10                  15

Ala Ser Trp Gln Lys Met Val Thr Ala Val Ala Ser Ala Ala
            20                  25                  30

<210>  106
<211>  18
<212>  PRT
<213>  artificial sequence

<220>
<223>  Hbg-68

<400>  106

Val Leu Ala Ile His Phe Gly Lys Glu Phe Thr Pro Glu Val Gln Ala
1               5                   10                  15

Ser Trp

<210>  107
<211>  16

```
<212>  PRT
<213>  artificial sequence

<220>
<223>  Hbg-69

<400>  107

Thr Pro Glu Val Gln Ala Ser Trp Gln Lys Met Val Thr Ala Val Ala
1               5                   10                  15
```